(19) Europäisches Patentamt
European Patent Office
Office européen des brevets

(11) EP 1 812 080 B1

(12) **EUROPEAN PATENT SPECIFICATION**

(45) Date of publication and mention of the grant of the patent:
**09.07.2014 Bulletin 2014/28**

(21) Application number: **05818064.7**

(22) Date of filing: **01.11.2005**

(51) Int Cl.:
***G01N 33/66*** *(2006.01)*

(86) International application number:
**PCT/US2005/039551**

(87) International publication number:
**WO 2006/052566 (18.05.2006 Gazette 2006/20)**

# (54) GPR41 AND MODULATORS THEREOF FOR THE TREATMENT OF INSULIN-RELATED DISORDERS

GPR41 UND SEINE MODULATOREN ZUR BEHANDLUNG VON INSULINBEDINGTEN ERKRANKUNGEN

GPR41 ET MODULATEURS DE CELUI-CI UTILISES DANS LE TRAITEMENT DE TROUBLES LIES A L'INSULINE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **03.11.2004 US 624867 P**

(43) Date of publication of application:
**01.08.2007 Bulletin 2007/31**

(73) Proprietor: **Arena Pharmaceuticals, Inc.**
**San Diego, CA 92121 (US)**

(72) Inventors:
- **LEONARD, James, N.**
  **San Diego, CA 92124 (US)**
- **CHU, Zhi Liang**
  **San Diego, CA 92128 (US)**
- **BRUCE, Marc, A.**
  **Escondido, CA 92029 (US)**
- **BOATMAN, P. Douglas**
  **San Diego, CA 92126 (US)**

(74) Representative: **Wytenburg, Wilhelmus Johannes et al**
**Mewburn Ellis LLP**
**33 Gutter Lane**
**London**
**EC2V 8AS (GB)**

(56) References cited:
**WO-A-01/61359**
**WO-A-02/100403**
**WO-A-2004/038421**
**CA-A- 2 482 915**
**CA-A1- 2 497 901**

- **BUXTON, DENIS B. ET AL: "The effects of cyclopropane carboxylic acid on hepatic pyruvate metabolism" METABOLISM, CLINICAL AND EXPERIMENTAL , 32(7), 736-44 CODEN: METAAJ; ISSN: 0026-0495, 1983, XP002391100**
- **STEWART, G. A.: "Pharmacological studies on oral hypoglycemic agents" DEUTSCH-ENGLISCHE MEDIZINISCHE RUNDSCHAU , 1(4), 334-47 CODEN: AMRWAX; ISSN: 0003-3332, 1962, XP002391101**
- **LEE DENNIS K ET AL: "Continued discovery of ligands for G protein-coupled receptors." LIFE SCIENCES. 5 DEC 2003, vol. 74, no. 2-3, 5 December 2003 (2003-12-05), pages 293-297, XP002391222 ISSN: 0024-3205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 812 080 B1

SEQUENCE LISTING

<110> Arena Pharmaceuticals Inc. Leonard, James N. chu, zhi-Liang Bruce, Marc A. Boatman, P. Douglas

<120> GPR41 AND MODULATORS THEREOF FOR THE TREATMENT OF INSULIN-RELATED DISORDERS

<130> 94.WO1

<160> 15

<170> PatentIn version 3.2

<210> 1
<211> 1041
<212> DNA
<213> Homo sapien

<400> 1

```
atggatacag gccccgacca gtcctacttc tccggcaatc actggttcgt cttctcggtg      60
taccttctca ctttcctggt ggggctcccc ctcaacctgc tggccctggt ggtcttcgtg     120
ggcaagctgc agcgccgccc ggtggccgtg gacgtgctcc tgctcaacct gaccgcctcg     180
gacctgctcc tgctgctgtt cctgcctttc cgcatggtgg aggcagccaa tggcatgcac     240
tggcccctgc ccttcatcct ctgcccactc tctggattca tcttcttcac caccatctat     300
ctcaccgccc tcttcctggc agctgtgagc attgaacgct tcctgagtgt ggcccaccca     360
ctgtggtaca agacccggcc gaggctgggg caggcaggtc tggtgagtgt ggcctgctgg     420
ctgttggcct ctgctcactg cagcgtggtc tacgtcatag aattctcagg ggacatctcc     480
cacagccagg gcaccaatgg gacctgctac ctggagttcc ggaaggacca gctagccatc     540
ctcctgcccg tgcggctgga gatggctgtg gtcctctttg tggtcccgct gatcatcacc     600
agctactgct acagccgcct ggtgtggatc ctcggcagag gggcagcca ccgccggcag      660
aggagggtgg cggggctgtt ggcggccacg ctgctcaact tccttgtctg ctttgggccc     720
tacaacgtgt cccatgtcgt gggctatatc tgcggtgaaa gcccggcatg gaggatctac     780
gtgacgcttc tcagcaccct gaactcctgt gtcgacccct ttgtctacta cttctcctcc     840
tccgggttcc aagccgactt tcatgagctg ctgaggaggt tgtgtgggct ctggggccag     900
tggcagcagg agagcagcat ggagctgaag gagcagaagg gaggggagga gcagagagcg     960
gaccgaccag ctgaaagaaa gaccagtgaa cactcacagg gctgtggaac tggtggccag    1020
gtggcctgtg ctgaaagcta g                                              1041
```

<210> 2
<211> 346
<212> PRT
<213> Homo sapien

<400> 2

```
Met Asp Thr Gly Pro Asp Gln Ser Tyr Phe Ser Gly Asn His Trp Phe
1               5                   10                  15

Val Phe Ser Val Tyr Leu Leu Thr Phe Leu Val Gly Leu Pro Leu Asn
            20                  25                  30

Leu Leu Ala Leu Val Val Phe Val Gly Lys Leu Gln Arg Arg Pro Val
        35                  40                  45

Ala Val Asp Val Leu Leu Leu Asn Leu Thr Ala Ser Asp Leu Leu Leu
    50                  55                  60

Leu Leu Phe Leu Pro Phe Arg Met Val Glu Ala Ala Asn Gly Met His
65                  70                  75                  80

Trp Pro Leu Pro Phe Ile Leu Cys Pro Leu Ser Gly Phe Ile Phe Phe
                85                  90                  95

Thr Thr Ile Tyr Leu Thr Ala Leu Phe Leu Ala Ala Val Ser Ile Glu
            100                 105                 110

Arg Phe Leu Ser Val Ala His Pro Leu Trp Tyr Lys Thr Arg Pro Arg
        115                 120                 125

Leu Gly Gln Ala Gly Leu Val Ser Val Ala Cys Trp Leu Leu Ala Ser
    130                 135                 140

Ala His Cys Ser Val Val Tyr Val Ile Glu Phe Ser Gly Asp Ile Ser
145                 150                 155                 160

His Ser Gln Gly Thr Asn Gly Thr Cys Tyr Leu Glu Phe Arg Lys Asp
                165                 170                 175

Gln Leu Ala Ile Leu Leu Pro Val Arg Leu Glu Met Ala Val Val Leu
            180                 185                 190

Phe Val Val Pro Leu Ile Ile Thr Ser Tyr Cys Tyr Ser Arg Leu Val
        195                 200                 205

Trp Ile Leu Gly Arg Gly Gly Ser His Arg Arg Gln Arg Arg Val Ala
    210                 215                 220

Gly Leu Leu Ala Ala Thr Leu Leu Asn Phe Leu Val Cys Phe Gly Pro
225                 230                 235                 240
```

Tyr Asn Val Ser His Val Val Gly Tyr Ile Cys Gly Glu Ser Pro Ala
245 250 255

Trp Arg Ile Tyr Val Thr Leu Leu Ser Thr Leu Asn Ser Cys Val Asp
260 265 270

Pro Phe Val Tyr Tyr Phe Ser Ser Ser Gly Phe Gln Ala Asp Phe His
275 280 285

Glu Leu Leu Arg Arg Leu Cys Gly Leu Trp Gly Gln Trp Gln Gln Glu
290 295 300

Ser Ser Met Glu Leu Lys Glu Gln Lys Gly Gly Glu Glu Gln Arg Ala
305 310 315 320

Asp Arg Pro Ala Glu Arg Lys Thr Ser Glu His Ser Gln Gly Cys Gly
325 330 335

Thr Gly Gly Gln Val Ala Cys Ala Glu Ser
340 345

<210> 3
<211> 20
<212> DNA
<213> Artificial

<220>
<223> primer

<400> 3
atggggacaa gcttctttct 20

<210> 4
<211> 20
<212> DNA
<213> Artificial

<220>
<223> primer

<400> 4
ctagctcgga cactccttgg 20

<210> 5
<211> 17
<212> DNA
<213> Artificial

<220>
<223> primer

<400> 5
gccggcgcaa gaggata 17

<210> 6

<211> 21
<212> DNA
<213> Artificial

<220>
<223> primer

<400> 6
ccgaagcaga cgaagaagat g 21

<210> 7
<211> 17
<212> DNA
<213> Artificial

<220>
<223> probe

<400> 7
ttcttgcagc cacactg 17

<210> 8
<211> 257
<212> DNA
<213> Artificial

<220>
<223> probe

<400> 8

```
gtggggctga gggttacaca cagaggtggc accttggtga tgtcgacact gggtgaggga     60
caggaaacca gggaggtagg caggaccacc tgcaggggag agcatgtgga gctatggtgg    120
tggggtgtag gcagtgtaga cagcaatctt gcctgatggg taagagtctc ccagtgaggg    180
aaccccaact ctcaacacat tcctctctgt ctcattagca tctgtgacca tggggacaag    240
cttctttctt ggcaatt                                                   257
```

<210> 9
<211> 18
<212> DNA
<213> Artificial

<220>
<223> primer

<400> 9
gtggggctga gggttaca 18

<210> 10
<211> 18
<212> DNA
<213> Artificial

<220>
<223> primer

<400> 10
aattgccaag aaagaagc 18

<210> 11
<211> 6
<212> PRT
<213> Artificial

<220>
<223> peptide

<400> 11

Thr Leu Glu Ser Ile Met
1 5

<210> 12
<211> 5
<212> PRT
<213> Artificial

<220>
<223> peptide

<400> 12

Glu Tyr Asn Leu Val
1 5

<210> 13
<211> 5
<212> PRT
<213> Artificial

<220>
<223> peptide

<400> 13

Asp Cys Gly Leu Phe
1 5

<210> 14
<211> 36
<212> DNA
<213> Artificial

<220>
<223> primer

<400> 14
gatcaagctt ccatggcgtg ctgcctgagc gaggag 36

<210> 15
<211> 53

<212> DNA
<213> Artificial

<220>
<223> primer

<400> 15
gatcggatcc ttagaacagg ccgcagtcct tcaggttcag ctgcaggatg gtg 53

## Description

### FIELD OF THE INVENTION

The present invention relates to methods for identifying a glycemic stabilizing compound, for example, a compound that controls insulin secretion, by determining whether a compound modulates GPR41 functionality. Accordingly, compounds of the present invention are useful in the prophylaxis or treatment of insulin-related disorders such as hypoglycemia, an insulin-secreting or insulin-dependent tumor, aging, insulin resistance, impaired glucose tolerance, or diabetes.

### BACKGROUND OF THE INVENTION

Cells use glucose as a main source of energy. Therefore, food is first broken down by the body to glucose prior to being utilized. Glucose is then released from the gut into the blood resulting in a rise in blood glucose levels. In response to this rise in glucose level, pancreatic ß-islet cells increase their production and secretion of insulin. Insulin circulates through the blood and acts as a messenger, sending a signal to insulin responsive organs such as the adipose tissue, muscle and liver, to increase their intake of glucose. In this way a rise in blood glucose is accompanied by a subsequent increase in insulin secretion from ß-cells. It is the rise in insulin that acts to return blood glucose levels to normal. In healthy individuals blood glucose levels are kept fairly constant. This state of equilibrium, called normoglycemia (normal glucose level) is tightly controlled by insulin.

In diseases such as diabetes this tight regulation of blood glucose level is lost, leading to the increased blood glucose levels observed in diabetics. A state of hyperglycemia (high glucose level) can occur due to an insufficient production of insulin by the pancreatic ß-cells and/or through inadequate uptake of glucose by target organs such as muscle, liver and fat. The end result is an increase in blood glucose level. Thus, diabetes can be thought of as the result of two types of impairment: impaired insulin secretion from the ß-cells and impaired insulin sensitivity by the major insulin responsive organs. This impaired insulin sensitivity, also known as insulin resistance (because the organs are resistant to the effects of insulin), means that more insulin is required in order for the target organs to increase their glucose uptake. Insulin resistance leads to increased pressure on the ß-cells because the ß-cells need to increase their insulin secretion to compensate for insulin resistance. This is an escalating problem leading first to impaired glucose tolerance and; eventually, complete loss of insulin secretion due to the inability of the pancreas to keep up with the ever-increasing demand for insulin.

Diabetes is a diagnostic term for a group of disorders characterized by abnormal glucose homeostasis resulting in elevated blood glucose. There are many types of diabetes, but the two most common are Type I, also referred to as insulin-dependent diabetes mellitus or IDDM, and Type II, also referred to as non-insulin-dependent diabetes mellitus or NIDDM. Type I diabetes is mainly a disease with a young age of onset, and is due to the destruction of the insulin secreting ß-cells in the pancreas by the immune system. In this case the body fails to recognize the pancreatic ß-cells as being self and destroys its own cells. With the destruction of the ß-cells there is a complete loss of insulin secretion and so affected individuals have an absolute dependency on insulin for survival. Type II diabetes is mainly a disease with a later age of onset, usually after the age of 40, but in recent years it is more common to find younger people being diagnosed with Type II diabetes. It is mainly characterized by insulin resistance and beta cell exhaustion and is often associated with obesity. Type II diabetes is more common than Type I diabetes and accounts for 90-95% of all diabetes cases diagnosed worldwide.

Chronic exposure of tissues to hyperglycemia can result in diverse complications including microvascular problems of neuropathy, retinopathy and nephropathy and the macrovascular complications of stroke, coronary heart disease, and peripheral vascular disease. Inappropriate control of blood glucose level is also a characteristic of diseases other than diabetes such as obesity and Syndrome X. For example, one of the characteristics of Syndrome X is insulin resistance or glucose intolerance. In addition, obesity is characterized by hyperinsulinemia and insulin resistance, a feature shared with NIDDM, hypertension and atherosclerosis. Further, obesity is a major risk factor for NIDDM. The risk of developing NIDDM is tripled in subjects 30% or more overweight, and three-quarters of NIDDM patients are overweight.

Obesity, which is the result of an imbalance between caloric intake and energy expenditure, is highly correlated with insulin resistance and diabetes in experimental animals and humans. However, the molecular mechanisms that are involved in obesity diabetes syndromes still under investigation. During early development of obesity, increased insulin secretion balances insulin resistance and protects patients from hyperglycemia (Le Stunff, et al., Diabetes 43:696-702 (1989)). However, over time, β cell function deteriorates and non-insulin-dependent diabetes develops in about 20% of obese individuals (Pederson, P., Diab. Metab. Rev. 5:505-509 (1989), and Brancati, F. L., et al., Arch. Intern. Med. 159:957-963 (1999)). Given its high prevalence in modem societies, obesity has thus become the leading risk factor for NIDDM (Hill, J. O., et al., Science 280:1371-1374 (1998)). However, the factors which predispose some

patients to alteration of insulin secretion in response to fat accumulation remain unknown. Unfortunately, effective long-term therapies to treat obesity are still not available.

Diabetes afflicts several million people worldwide. In the United States alone, there are more than 18 million diabetics, with 600,000 new cases diagnosed each year. People with diabetes are at higher risk for heart disease, blindness, kidney failure, infection, extremity amputations, and other conditions. It is estimated that the direct medical expenditures and indirect expenditures attributable to diabetes in the United States were $132 billion in 2002. Taken together, diabetes complications are one of the nation's leading causes of death.

Therapies do exist to treat diabetes, such as $\alpha$-glucosidase inhibitors, biguanides, thiazolidinediones, meglitinides, sulfonylureas and exogenous insulin. However, these therapies have limited effectiveness and are associated with significant safety and tolerability issues such as risk for hypoglycemic episodes, weight gain, gastrointestinal disturbances and anemia. In addition, many of the treatment options require injection or multiple daily dosings which present compliance challenges.

In addition to disorders that benefit from increasing insulin secretion such as diabetes, there are a number of disorders that can benefit from decreasing insulin secretion. For example, a decrease in insulin secretion can result in an increase in blood glucose which is needed during hypoglycemia. In addition, for example, decreasing insulin secretion can be useful for a patient with an insulinoma, which is a tumor that secretes excess insulin. Insulin can also serve as a growth factor for certain tumors. Further, caloric restriction is known to down-regulate insulin secretion and this may be a mediator of caloric restriction's favorable impact on longevity. Thus, a reduction in insulin secretion can be beneficial to treat aging. In all these cases, a reduction in insulin levels can be beneficial.

Thus, there exists a need for the identification of an agent which safely and effectively modulates insulin secretion and/or blood glucose levels for the treatment of insulin-related disorders such as hypoglycemia, an insulin-secreting or insulin-dependent tumor, aging, insulin resistance, impaired glucose tolerance, or diabetes. The present invention satisfies this need and provides related advantages as well.

## SUMMARY OF THE INVENTION

Applicants have unexpectedly found that GPR41 is expressed in pancreatic islet cell lines and GPR41 is upregulated in db/db diabetic mice. In addition, Applicants have identified agonist compounds that modulate GPR41 function and have found that these compounds decrease insulin secretion. Further, Applicants disclose inverse agonists or antagonists of GPR41 that can be used to increase insulin secretion for the treatment of insulin-related disorders such as insulin resistance, impaired glucose tolerance and diabetes.

In *a first* aspect, the invention features a method for identifying a glycemic stabilizing compound, comprising: a) contacting a candidate compound with GPR41, and b) determining whether GPR41 functionality is modulated, wherein a modulation in GPR41 functionality is indicative of the candidate compound being a glycemic stabilizing compound said GPR41 having an amino acid sequence as shown in SEQ ID NO:2, or a variant or ortholog thereof, wherein said variant is an allelic variant, splice variant or conservative amino acid substitution variant of GPR41. In some embodiments, said GPR41 is human. In some embodiments, said determining comprises a second messenger assay. In some embodiments, a glycemic stabilizing compound comprises a compound selected from the group consisting of: 2-methyl-4-(4-nitro-phenyl)-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, 2-Methyl-4-(3-nitro-phenyl)-5-oxo-1,4,5,6,7,8-hexahydro-quinoline-3-carboxylic acid o-tolylamide, 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3 -carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof

In some embodiments, said glycemic stabilizing compound is a GPR41 agonist, for example, a compound selected from the group consisting of: 2-methyl-4-(4-nitro-phenyl)-5-oxo-1,4,5,6,7,8-hexahydro-quinoiine-3-carboxylic acid o-tolylamide, 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-haxahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof. In some embodiments, said glycemic stabilizing compound is a GPR41 inverse agonist or antagonist, for example, a compound selected from the group consisting of: 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

In a further aspect, the invention features a method for preparing a composition comprising a glycemic stabilizing compound and including admixing said compound with a carrier, wherein said compound is one of the compounds disclosed above.

The invention further features a pharmaceutical composition comprising, consisting essentially of, or consisting of a compound provided above.

Described herein is a method for treating or preventing an insulin-related disorder in an individual in need thereof, comprising administering to said individual an effective amount of a compound provided above. In some instances, said insulin-related disorder is hypoglycemia, an insulin-secreting or insulin-dependent tumor, insulin resistance, impaired glucose tolerance, or diabetes. This method may further comprise administering to said individual an effective amount of an agent used for the treatment of diabetes, blood lipid disorders, or obesity in combination with an effective amount of a compound of the fourth aspect. The individual may be a mammal and preferably the individual is a human.

In a further aspect, the invention features a method for the manufacture of a medicament comprising a compound provided above for use as a glycemic stabilizing compound and a method for the manufacture of a medicament comprising said compound for use in the treatment of an insulin-related disorder.

Also described herein is a method for identifying a glycemic stabilizing compound, comprising: a) contacting a candidate compound with GPR41, and b) determining whether GPR41 functionality is increased, wherein an increase in GPR41 functionality is indicative of the candidate compound being a glycemic stabilizing compound. Preferably said GPR41 is human. Preferably said determining comprises a second messenger assay. A glycemic stabilizing compound may comprises a compound selected from the group consisting of: 2-methyl-4-(4-nitro-phenyl)-5-oxo-1,4,5,6,7,8-hexahydro-quinoline-3-carboxylic acid otolylamide, cyclopropanecarboxylic acid 4-[1,2,3]thiadiazol-4-yl-phenyl ester, cyclopropanecarboxylic acid; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

The glycemic stabilizing compound may be a GPR41 agonist, for example, a compound selected from the group consisting of: 2-methyl-4-(4-nitro-phenyl)-5-oxo-1,4,5,6,7,8-hexahydro-quinoline-3-carboxylic acid o-tolylamide, cyclopropanecarboxylic acid 4-[1,2,3]thiadiazol-4-yl-phenyl ester, cyclopropanecarboxylic acid; 4-Furan-3-yl-2-methyl-5-oxo-l,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

Also described herein is a method for identifying a glycemic stabilizing compound, comprising: a) contacting a candidate compound with GPR41, and b) determining whether GPR41 functionality is decreased, wherein a decrease in GPR41 functionality is indicative of the candidate compound being a glycemic stabilizing compound. Preferably, said GPR41 is human. Preferably said determining comprises a second messenger assay. The glycemic stabilizing compound may comprise a compound selected from the group consisting of 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxyphenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof

Preferably said glycemic stabilizing compound is a GPR41 inverse agonist or antagonist. The glycemic stabilizing compound may comprise a compound selected from the group consisting of: 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid otolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoroinethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

Further described herein is a method for increasing GPR41 function comprising contacting GPR41 with an effective amount of a GPR41 agonist. Preferably said agonist comprises a compound selected from the group consisting of: 2-methyl-4-(4-nitro-phenyl)-5-oxo-1,4,5,6,7,8-hexahydro-quinoline-3-carboxylic acid o-tolylamide, cyclopropanecarboxylic acid 4-[1,2,3]thiadiazol-4-yl-phenyl ester, cyclopropanecarboxylic acid; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl,2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

Also described herein is a method for decreasing GPR41 function comprising contacting GPR41 with an effective amount of a GPR41 inverse agonist or antagonist. Preferably said inverse agonist or antagonist comprises a compound selected from the group consisting of, 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

## BRIEF DESCRIPTION OF THE DRAWINGS

**Figure 1** shows dot blot analysis of human GPR41 expression in human adult and fetal tissues.
**Figure 2** shows RT-PCR and TaqMan quantitative PCR analysis of mouse GPR41 expression in selected tissues of normal and mutant mice.
**Figure 3** shows RNase protection assay analysis of mouse GPR41 expression in mouse cell types and tissues.
**Figure 4** shows coupling of GPR41 to G-protein G-alpha i.
**Figure 5** shows coupling of GPR41 to G-protein G-alpha 12/13.
**Figure 6** shows efficacy of GPR41 agonists in Gq/Gi co-transfected 293 cells.
**Figure 7** shows a GPR41 agonist inhibits insulin release in MIN6 insulinoma cells.
**Figure 8** shows that a GPR41 agonist, Compound 4, reverses the beneficial effect of an oral glucose tolerance test (oGTT) lowering compound, B111.

## DETAILED DESCRIPTION

Applicants have disclosed herein that human GPR41 is expressed predominantly in the pancreas (see Figure 1) and mouse GPR41 is expressed in the pancreas and pancreatic islet cell lines (see Figures 2 and 3). The pancreas is divided into lobules by connective tissue septae. Lobules are composed largely of grape-like clusters of exocrine cells called acini, which secrete digestive enzymes. Embedded within the pancreatic exocrine tissue are Islets of Langerhans, the endocrine component of the pancreas. Islets make up only 1% of the pancreas. Islets contain several cell types and are richly vascularized. The islet cell types include: alpha, beta, delta, A, B, C, D, and E. It is the beta islet cells that secrete insulin.

Applicants also disclose herein that mouse GPR41 is up-regulated in pancreatic islet cells from db/db diabetic mice compared to islets from C57 wild-type mice (see Figure 2). Further, Applicants have disclosed herein the G-protein coupling of GPCR GPR41 to G-alpha i and G-alpha 12/13 (see Figures 4 and 5). In addition, Applicants disclose herein that GPR41 agonists induce IP3 signaling in cells cotransfected with Gq/Gi (see Figure 6). Using MIN6 insulinoma cells, Applicants further disclose herein that a GPR41 agonist inhibits insulin secretion (Figure 7) and reversed the beneficial effect of an oral glucose tolerance test (oGTT) lowering compound (Figure 8).

Although a number of receptor classes exist in humans, the most abundant and currently therapeutically relevant is represented by the G protein-coupled receptor (GPCR) class. It is estimated that there are some 30,000-40,000 genes

within the human genome, and of these, approximately 2% are estimated to code for GPCRs. GPCRs represent an important area for the development of pharmaceutical products: from approximately 20 of the 100 known GPCRs, approximately 60% of all prescription pharmaceuticals have been developed.

GPCRs share a common structural motif, having seven sequences of between 22 to 24 hydrophobic amino acids that form seven alpha helices, each of which spans the membrane (each span is identified by number, i.e., transmembrane-1 (TM-1), transmembrane-2 (TM-2), etc.). The transmembrane helices are joined by strands of amino acids between transmembrane-2 and transmembrane-3, transmembrane-4 and transmembrane-5, and transmembrane-6 and transmembrane-7 on the exterior, or "extracellular" side, of the cell membrane (these are referred to as "extracellular" regions 1, 2 and 3 (EC-1, EC-2 and EC-3), respectively). The transmembrane helices are also joined by strands of amino acids between transmembrane-1 and transmembrane-2, transmembrane-3 and transmembrane-4, and transmembrane-5 and transmembrane-6 on the interior, or "intracellular" side, of the cell membrane (these are referred to as "intracellular" regions 1, 2 and 3 (IC-1, IC-2 and IC-3), respectively). The "carboxy" ("C") terminus of the receptor lies in the intracellular space within the cell, and the "amino" ("N") terminus of the receptor lies in the extracellular space outside of the cell.

Generally, when a ligand binds with the receptor (often referred to as "activation" of the receptor); there is a change in the conformation of the receptor that facilitates coupling between the intracellular region and an intracellular "G-protein." It has been reported that GPCRs are "promiscuous" with respect to G proteins, i.e., that a GPCR can interact with more than one G protein. See, Kenakin, T., 43 Life Sciences 1095 (1988). Although other G proteins exist, currently, Gq, Gs, Gi, Gz and Go are G proteins that have been identified. Ligand-activated GPCR coupling with the G-protein initiates a signaling cascade process (referred to as "signal transduction"). Under normal conditions, signal transduction ultimately results in cellular activation or cellular inhibition. Although not wishing to be bound to theory, it is thought that the IC-3 loop as well as the carboxy terminus of the receptor interact with the G protein.

There are also promiscuous G proteins, which appear to couple to several classes of GPCRs to the phospholipase C pathway, such as G$\alpha$15 or G$\alpha$16 (Offermanns & Simon, J Biol Chem 270:15175-80 (1995)), or chimeric G proteins designed to couple a large number of different GPCRs to the same pathway, e.g. phospholipase C (Milligan & Rees, Trends in Pharmaceutical Sciences 20:118-24 (1999)).

Gi-coupled GPCRs lower intracellular cAMP levels. The melanophore technology (see *infra*) is useful for identifying Gi-coupled GPCRs and also for identifying modulators of said Gi-coupled GPCRs.

Under physiological conditions, GPCRs exist in the cell membrane in equilibrium between two different conformations: an "inactive" state and an "active" state. A receptor in an inactive state is unable to link to the intracellular signaling transduction pathway to initiate signal transduction leading to a biological response. Changing the receptor conformation to the active state allows linkage to the transduction pathway (via the G-protein) and produces a biological response.

A receptor can be stabilized in an active state by a ligand or a compound such as a drug. Recent discoveries, including but not exclusively limited to modifications to the amino acid sequence of the receptor, provide means other than ligands or drugs to promote and stabilize the receptor in the active state conformation. These means effectively stabilize the receptor in an active state by simulating the effect of a ligand binding to the receptor. Stabilization by such ligand-independent means is termed "constitutive receptor activation."

The sequence of GPR41 was first published in the literature by Sawzdargo et al. (Sawzdargo et al., Biochem. Biophys. Res. Commun., 239:543-547 (1997)). Sawzdargo et al. amplified human genomic DNA using PCR with degenerate primers based on conserved sequences within the human and rat galanin receptor 1 (GALR1) and rat GALR2. One product contained a segment showing 100% homology to a portion of the 3-prime region of the human CD22 gene. Sawzdargo searched for open reading frames in this region and identified the GPR40 and GPR41 genes. The GPR41 gene has no introns. GPR41 encodes a predicted 346 amino acid GPCR containing 7 transmembrane domains, 1 glycosylation site, 1 PKC phosphorylation site, 2 PKA/PKC phosphorylation sites, and 1 palmitoylation site, which is located in the C-terminal domain. The GPR41 protein shares 98% amino acid identity with GPR42, but little similarity with GALRs. Sawzdargo et al. further reported that the GPR41 gene is located downstream of CD22, which was previously mapped to 19q13.1.

GPR41 was classified as an orphan receptor, meaning that no ligand had been identified for the receptor. Recently, Brown et al. have reported that GPR41 is activated by propionate and other short chain carboxylic acid anions (Brown et al., J. Biol. Chem.. 278:11312-11319 (2003)). In addition, Brown et al. indicate that GPR41 activates the Gi/o family proteins and GPR41 is primarily expressed in adipose tissue.

In contrast to the disclosure herein that GPR41 is expressed in the beta cells of the pancreas, WO 01/61359 (filing date February 19, 2001) indicates that GPR41 is restricted to adipose tissue. In addition, WO 01/61359 indicates that GPR41 can be used as a screening target for compounds that inhibit lipolysis. Since GPR41 is coupled to Gi, such compounds would be agonists of GPR41. Agonists of GPR41 are also hypothesized in WO 01/61359 to be useful, for example in the manufacture of a medicament for the treatment of dyslipidaemia and conditions associated with dyslipidemia, coronary heart disease, atherosclerosis, thrombosis or obesity, angina, chronic renal failure, peripheral vascular

disease, stroke, type II diabetes or metabolic syndrome. This is in contrast to the disclosure herein that an inverse agonist or antagonist of GPR41 would be useful, for example, in the manufacture of a medicament for treatment of an insulin-related disorder such as diabetes.

## DEFINITIONS

The scientific literature that has evolved around receptors has adopted a number of terms to refer to ligands having various effects on receptors. For clarity and consistency, the following definitions will be used throughout this patent document.

**AGONIST** shall mean material, for example, a ligand or candidate compound, that activates an intracellular response when it binds to the receptor. An intracellular response can be, for example, enhancement of GTP binding to membranes or modulation of the level of a second messenger such as cAMP or IP3. In some embodiments, an **AGONIST** is material not previously known to activate the intracellular response when it binds to the receptor (for example, to enhance GTPγS binding to membranes or to lower intracellular cAMP level). In some embodiments, an **AGONIST** is material not previously known to decrease blood glucose level when it binds to the receptor. The term **AGONIST** also includes **PARTIAL AGONISTS** which are materials, for example, ligands or candidate compounds, which activate the intracellular response when they bind to the receptor to a lesser degree or extent than do full agonists.

**ANTAGONIST** shall mean material, for example, ligands or candidate compounds that competitively bind to the receptor at the same site as an agonist but which does not activate an intracellular response, and can thereby inhibit an intracellular response elicited by the agonist. An **ANTAGONIST** does not diminish the baseline intracellular response in the absence of an agonist. In some embodiments, an **ANTAGONIST** is material not previously known to compete with an agonist to inhibit a cellular response when it binds to the receptor (for example, wherein the cellular response is GTPγS binding to membranes or to the lowering of intracellular cAMP level).

**ANTIBODY** is intended herein to encompass monoclonal antibodies and polyclonal antibodies. The term **ANTIBODY** is further intended to encompass IgG, IgA, IgD, IgE, and IgM. Antibodies include whole antibodies, including single-chain whole antibodies, and antigen binding fragments thereof, including Fab, Fab', F(ab)2 and F(ab')2. Antibodies can be from any natural or synthetic origin, for example, from human, murine, rabbit, goat, guinea pig, hamster, camel, donkey, sheep, horse or chicken. Antibodies can have binding affinities with a dissociation constant or Kd value, for example, less than 5x10-6M, 10-6M, 5x10-7M, 10-7M, 5x10-8M, 10-8M, 5x10-9M, 10-9M, 5x10-10M 10-10M, 5x10-11M, 10-11M, 5x10-12M, 10-12M, 5x10-13M, 10-13M, 5x10-14M 10-14M, 5x10-15M and 10-15M. Antibodies of the present invention can be prepared by any suitable method known in the art.

**CANDIDATE COMPOUND** shall mean a molecule (for example, a chemical compound) that is amenable to a screening technique. The term candidate compound specifically excludes any compound already known to modulate GPR41, for example, a known agonist of GPR41.

**COMPOSITION** shall mean a material comprising at least two compounds or two components; for example, a "pharmaceutical composition" is a composition.

**COMPOUND EFFICACY** shall mean a measurement of the ability of a compound to inhibit or stimulate receptor functionality, as opposed to receptor binding affinity. **CONSTITUTIVELY ACTIVATED RECEPTOR** shall mean a receptor subject to constitutive receptor activation.

**CONSTITUTIVE RECEPTOR ACTIVATION** shall mean stabilization of a receptor in the active state by means other than binding of the receptor with its endogenous ligand or a chemical equivalent thereof

**CONTACT** or **CONTACTING** shall mean bringing at least two moieties together, whether in an *in vitro* system or an *in vivo* system.

**DIABETES** as used herein is intended to encompass the usual diagnosis of diabetes made from any method including, for example, the following list: symptoms of diabetes (e.g., polyuria, polydipsia, polyphagia) plus casual blood glucose levels of greater than or equal to 200 mg/dl, wherein casual blood glucose is defined any time of the day regardless of the timing of meal or drink consumption; or 8 hour fasting blood glucose levels of greater than or equal to 126 mg/dl; or blood glucose levels of greater than or equal to 200 mg/dl two hours following oral administration of 75 g anhydrous glucose dissolved in water. In addition, the term diabetes as used herein also includes the "pre-diabetic" state as defined by the American Diabetes Association to be a fasting blood glucose level of 100-125 mg/dl or blood glucose levels of 140-199 mg/dl two hours following oral administration of glucose. Diabetes can be precipitated by several conditions including, for example, autoimmune destruction of beta islet cells, beta cell apoptosis, or pregnancy (gestational diabetes).

**ENDOGENOUS** shall mean a material that a mammal naturally produces. ENDOGENOUS in reference to, for example and not limitation, the term "receptor" shall mean that which is naturally produced by a mammal (for example, and not limitation, a human) or a virus. In contrast, the term **NON-ENDOGENOUS** in this context shall mean that which is not naturally produced by a mammal (for example, and not limitation, a human) or a virus. For example, and not limitation, a receptor which is not constitutively active in its endogenous form, but when manipulated becomes constitutively active, is most preferably referred to herein as a "non-endogenous, constitutively activated receptor." Both terms can be utilized to describe both "in vivo" and "in vitro" systems. For example, and not a limitation, in a screening approach, the endogenous or non-endogenous receptor can be in reference to an in vitro screening system.

**EFFECTIVE AMOUNT** means an amount of active compound or pharmaceutical composition that elicits the desired biological or medicinal response in a tissue, system, or individual that is being sought by the researcher or medical doctor or other clinician. For example, an effective dose can be an amount that can treat an insulin-related disorder. Also, for example, an effective dose can be an amount that can prevent an insulin-related disorder.

**GLYCEMIC STABILIZING COMPOUND** is intended to mean a compound that stabilizes blood glucose levels. Stabilization of blood glucose can be direct or indirect. For example, a glycemic-stabilizing compound can stabilize blood glucose levels in an individual with diabetes by increasing insulin secretion. In addition, for example, a glycemic-stabilizing compound can stabilize blood glucose levels in an individual with hypoglycemia by decreasing insulin secretion. Further, for example, a glycemic-stabilizing compound can stabilize blood glucose levels by increasing glucose sensitivity at an organ or tissue.

**IMPAIRED GLUCOSE TOLERANCE** (IGT) as used herein is intended to indicate that condition associated with insulin-resistance that is intermediate between frank, type 2 diabetes and normal glucose tolerance (NGT). IGT is diagnosed by a procedure wherein an affected person’s postprandial glucose response is determined to be abnormal as assessed by 2-hour postprandial plasma glucose levels. In this test, a measured amount of glucose is given to the patient and blood glucose levels are measured at regular intervals, usually every half hour for the first two hours and every hour thereafter. In a "normal" or non-IGT individual, glucose levels rise during the first two hours to a level less than 140 mg/dl and then drop rapidly. In an IGT individual, the blood glucose levels are higher and the drop-off level is at a slower rate.

**IN NEED OF PREVENTION OR TREATMENT** as used herein refers to a judgment made by a caregiver (e.g. physician, nurse, nurse practitioner, etc. in the case of humans; veterinarian in the case of animals, including non-human mammals) that an individual or animal requires or will benefit from treatment. This judgment is made based on a variety of factors that are in the realm of a caregiver’s expertise, but that include the knowledge that the individual or animal is ill, or will be ill, as the result of a condition that is treatable by the compounds of the invention.

**INDIVIDUAL** as used herein refers to any animal, including mammals, preferably mice, rats, other rodents, rabbits, dogs, cats, swine, cattle, sheep, horses, or primates, and most preferably humans.

**INHIBIT** or **INHIBITING,** in relationship to the term "response" shall mean that a response is decreased or prevented in the presence of a compound as opposed to in the absence of the compound.

**INSULIN-RELATED DISORDER** means a disorder related to the level of insulin in the blood or at an organ or tissue. As used herein, an insulin-related disorder can be the result of, for example, too little insulin secretion, too much insulin secretion, or even normal insulin secretion coupled with resistance of an organ to insulin. An insulin-related disorder is intended to include, for example, a disorder that would benefit from a decrease in insulin secretion, for example, hypoglycemia, an insulinoma, a tumor where insulin is a growth factor, or aging. In addition, an insulin-related disorder is intended to include, for example, a disorder that results in elevated blood glucose and would benefit from an increase in insulin secretion. Such disorders include, for example, insulin resistance, impaired glucose tolerance or diabetes such as Type I diabetes or Type II diabetes. Further, in some embodiments, the term insulin-related disorder can include diseases that are related to an elevated blood glucose level, for example, atherosclerosis, heart disease, stroke, hypertension, Syndrome X, obesity, and peripheral vascular disease.

**INSULIN RESISTANCE** as used herein is intended to encompass the usual diagnosis of insulin resistance made by any of a number of methods, including but not restricted to: the intravenous glucose tolerance test or measurement of the fasting insulin level. It is well known that there is a good correlation between the height of the fasting insulin level and the degree of insulin resistance. Therefore, one could use elevated fasting insulin levels as a surrogate marker for insulin resistance for the purpose of identifying which normal glucose tolerance (NGT) individuals have insulin resistance. A diagnosis of insulin resistance can also be made using the euglycemic glucose clamp test.

**INVERSE AGONIST** means material, for example, a ligand or candidate compound that binds either to the endogenous form or to the constitutively activated form of the receptor so as to reduce the baseline intracellular response of the receptor observed in the absence of an agonist. An intracellular response can be, for example, modulation of GTP binding to membranes or modulation of the level of a second messenger such as cAMP or IP3. In some embodiments, an **INVERSE AGONIST** is material not previously known to reduce the baseline intracellular response of the receptor observed in the absence of an agonist.

**LIGAND** shall mean an endogenous, naturally occurring molecule specific for an endogenous, naturally occurring receptor.

As used herein, the terms **MODULATE** or **MODULATING** shall mean to refer to an increase or decrease in the amount, quality, response or effect of a particular activity, function or molecule. A **GPR41 MODULATOR** is an agent that modulates the GPR41 receptor.

**PHARMACEUTICAL COMPOSITION** shall mean a composition comprising at least one compound and a

pharmaceutically acceptable carrier. For example, a pharmaceutical composition can comprise at least one active ingredient, whereby the composition is amenable to investigation for a specified, efficacious outcome in an animal (for example, a mammal such as a human). Those of ordinary skill in the art will understand and appreciate the techniques appropriate for determining whether an active ingredient has a desired efficacious outcome based upon the needs of the artisan.

**RECEPTOR FUNCTIONALITY** shall refer to the normal operation of a receptor to receive a stimulus and moderate an effect in the cell, including, but not limited to regulating gene transcription, regulating the influx or efflux of ions, effecting a catalytic reaction, and/or modulating activity through G-proteins. A GPR41 functionality can be, for example, binding a G-protein such as Gi or G12/13, signaling through a second messenger such as cAMP or IP3 (when using a chimeric G-protein), specifically binding to a GPR41-specific antibody, specifically binding to a compound such as a GPR41 agonist or inverse agonist, modulating insulin secretion or modulating blood glucose levels *in vivo.*

**SECOND MESSENGER** shall mean an intracellular response produced as a result of receptor activation. A second messenger can include, for example, inositol triphosphate (IP3), diacylglycerol (DAG), cyclic AMP (cAMP), cyclic GMP (cGMP), and Ca2+. Second messenger response can be measured for a determination of receptor activation. In addition, second messenger response can be measured for the direct identification of candidate compounds, including for example, inverse agonists, partial agonists, agonists, and antagonists.

The invention relates to a method for identifying a glycemic stabilizing compound, comprising: a) contacting a candidate compound with GPR41, and b) determining whether GPR41 functionality is modulated, wherein a modulation in GPR41 functionality is indicative of the candidate compound being a glycemic stabilizing compound. The screening method can be used to identify a compound which can be, for example, an agonist, inverse agonist, partial agonist, or antagonist of GPR41.

As used herein, "GPR41" refers to a polypeptide with the amino acid sequence as shown in SEQ ID NO:2, or a variant or ortholog of this sequence that retains substantially the function of a polypeptide with the amino acid sequence as referenced in SEQ ID NO:2, wherein said variant is an allelic variant, splice variant or conservative amino acid substitution variant of GPR41.

It is understood that limited variations or modifications to GPR41 can be made without destroying its function. For example, GPR41 is intended to include other GPR41 polypeptides, for example, mammalian species orthologs of the human GPR41 polypeptide. The sequences of species orthologs of human GPR41 are present in the database, for example, a mouse ortholog of GPR41 can be found in GenBank at Accession No. XM_145470 and a rat ortholog of GPR41 can be found in GenBank at Accession No. XM_344880. In addition, GPR41 includes variants such as allelic variants, splice variants and conservative amino acid substitution variants of GPR41. For example, GPR41 includes variants that retain substantially the function of the wild-type GPR41 polypeptide such as, for example, the ability to signal through G-alpha i or G-alpha 12/13, the ability to specifically bind to a GPR41-specific antibody, the ability to specifically bind to a compound such as a known ligand or agonist, the ability to specifically bind to agonist or inverse agonist compounds disclosed herein, or the ability to regulate insulin secretion or blood glucose levels. A GPR41 variant need not function to the same level as the wild-type GPR41, and need not contain every function of the wild-type GPR41.

Conservative and non-conservative amino acid changes, gaps, and insertions to an amino acid sequence can be compared to a reference sequence using available algorithms and programs such as the Basic Local Alignment Search Tool ("BLAST") using default settings [See, e.g., Karlin and Altschul, Proc Natl Acad Sci USA (1990) 87:2264-8; Altschul et al., J Mol Biol (1990) 215:403-410; Altschul et al., Nature Genetics (1993) 3:266-72; and Altschul et al., Nucleic Acids Res (1997) 25:3389-3402].

It is understood that a fragment of GPR41 which retains substantially a function of the entire polypeptide is included in the definition. For example, a signal generating domain of GPR41 or a compound binding domain of GPR41 can be used in lieu of the entire polypeptide. In addition, GPR41 can contain heterologous sequences such as an epitope tag or other fused polypeptide. Further, GPR41 can contain a label, for example, a radiolabel, fluorescent label or enzymatic label,

The methods described herein can be applied using a polypeptide comprising 99%, 98%, 95%, 92%, 90%, 85%, 80%, or 75% sequence identity to SEQ ID NO:2, where the polypeptide is not GPR42.

In some embodiments, said variant of GPR41 is a non-endogenous, constitutively activated mutant of GPR41. In one embodiment, said GPR41 is derived from a mammal. In another embodiment, said GPR41 is human.

In certain embodiments, said GPR41 is recombinant. In certain embodiments, said contacting comprises contacting with a host cell or with membrane of a host cell that expresses the GPCR, wherein the host cell comprises an expression vector comprising a polynucleotide encoding the receptor. In some embodiments, said contacting is carried out in the presence of a known agonist of the GPCR or an agonist as disclosed herein.

In certain embodiments, said method further comprises the step of comparing the modulation of the receptor caused by the candidate compound to a second modulation of the receptor caused by contacting the receptor with a known modulator of the receptor. In certain embodiments, said known modulator is an agonist.

In some embodiments, said determining comprises a second messenger assay, for example, determining is

through the measurement of GTPγS binding to membrane comprising said GPCR. In certain embodiments, said GTPγS is labeled with [$^{35}$S]. In certain embodiments, said determining is through the measurement of the level of a second messenger selected from the group consisting of cyclic AMP (cAMP), cyclic GMP (cGMP), inositol triphosphate (IP3), diacylglycerol (DAG), MAP kinase activity, and $Ca^{2+}$. In certain embodiments, said second messenger is cAMP. In certain embodiments, said measurement of cAMP is carried out using whole-cell adenylyl cyclase assay. In certain embodiments, said measurement of cAMP is carried out with membrane comprising said GPCR. In certain embodiments, said determining is through measurement of intracellular IP3. In certain embodiments, said determining further includes the use of a chimeric G-protein such as a Gq/Gi chimera. In certain embodiments, said second messenger is MAP kinase activity. In some embodiments, said determining is through CRE-reporter assay. In certain embodiments, said reporter is luciferase. In some embodiments, said reporter is β-galactosidase. In certain embodiments, said determining or said comparing is through measurement of intracellular $Ca^{2+}$.

In some embodiments, said determining is through measurement of glucose uptake by adipocytes obtained from a manunal. In some embodiments, said determining is through measurement of glucose uptake by skeletal muscle cells obtained from a mammal.

In certain embodiments, said determining is through the use of a melanophore assay.

In some embodiments, said glycemic stabilizing compound comprises a compound selected from the group consisting of: 2-methyl-4-(4-nitro-phenyl)-5-oxo-1,4,5,6,7,8-hexahydro-quinoline-3-carboxylic acid o-tolylamide, cyclopropanecarboxylic acid 4-[1,2,3]thiadiazol-4-yl-phenyl ester, 4-Furan-3-yl-2-methyl-5-oxo-1,4,5,6,7,8-hexahydro-quinoline-3-carboxylic acid (2,5-dichlorophenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-l,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, 2-Methyl-4-(3-nitro-phenyl)-5-oxo-1,4,5,6,7,8-hexahydro-quinoline-3-carboxylic acid o-tolylamide, 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

The invention also relates to a method of identifying a candidate compound as a modulator of insulin secretion, comprising a) contacting a candidate compound with GPR41, and b) determining whether GPR41 functionality is modulated, wherein a modulation in GPR41 functionality is indicative of the candidate compound being a modulator of insulin secretion. For example, a compound that decreases GPR41 functionality, such as a GPR41 antagonist or inverse agonist, can result in an increase in insulin secretion. An increase in insulin secretion can be desired, for example, in individuals with insulin resistance such as diabetics. A compound that increases GPR41 functionality, such as a GPR41 agonist, can result in a decrease in insulin secretion. A decrease in insulin secretion can be desired, for example, in individuals with hypoglycemia.

The invention also relates to a method of identifying a candidate compound as a modulator of blood glucose concentration, comprising a) contacting a candidate compound with GPR41, and b) determining whether GPR41 functionality is modulated, wherein a modulation in GPR41 functionality is indicative of the candidate compound being a modulator of blood glucose concentration. The invention also relates to a method of identifying a candidate compound as a modulator of insulin secretion, comprising a) contacting a candidate compound with GPR41, and b) determining whether GPR41 functionality is modulated, wherein a modulation in GPR41 functionality is indicative of the candidate compound being a modulator of insulin secretion. For example, a compound that decreases GPR41 functionality, such as a GPR41 inverse agonist or antagonist, can result in an increase in insulin secretion and a decrease in blood glucose concentration. A decrease in blood glucose can be desired, for example, in individuals with hyperglycemia such as diabetics. A compound that increases GPR41 functionality, such as a GPR41 agonist, can result in a decrease in insulin secretion and an increase in blood glucose concentration. An increase in blood glucose can be desired, for example, in individuals with hypoglycemia.

In certain embodiments, said GPR41 is recombinant. In certain embodiments, said contacting comprises contacting with a host cell or with membrane of a host cell that expresses the GPCR, wherein the host cell comprises an expression vector comprising a polynucleotide encoding the receptor. In some embodiments, said contacting is carried out in the presence of an agonist of the GPCR.

In the methods of the invention, control reactions can be performed to show specificity of the response. For example, mock-transfected cells can be compared to GPR41 transfected cells to show specificity of a response to the GPR41 receptor.

In the methods of the invention, in certain embodiments, said candidate compound is not an antibody or antigen-binding derivative thereof. In certain embodiments, said candidate compound is not a peptide. In certain embodiments,

said candidate compound is not a polypeptide.

As stated above, receptor fitnctionality refers to the normal operation of a receptor to receive a stimulus and moderate an effect in the cell, including, but not limited to regulating gene transcription, regulating the influx or efflux of ions, effecting a catalytic reaction, and/or modulating activity through G-proteins. A GPR41 functionality can be, for example, binding a G-protein such as Gi or G12/13, signaling through a second messenger such as cAMP or IP3 (when using a chimeric G-protein), specifically binding to a GPR41-specific antibody, specifically binding to a compound such as a GPR41 agonist or inverse agonist, modulating insulin secretion or modulating blood glucose levels *in vivo.*

In the methods of the invention, determining can comprise a second messenger assay. The initiation of an intracellular signal can be determined, for example, through the measurement of the level of a second messenger such as cyclic AMP (cAMP), cyclic GMP (cGMP), inositol triphosphate (IP3), diacylglycerol (DAG), MAP kinase, or calcium. Several assays are well known in the art for measuring these second messengers, for example, cAMP assays, IP3 assays, the FLIPR assay, the melanophore assay, or CRE-reporter assay. In addition, examples of second messenger assays are disclosed herein in Examples 12-17. In certain embodiments, said second messenger is cAMP. In other embodiments, said second messenger is IP3. In further embodiments said second messenger is calcium.

In one embodiment, said determining is through the measurement of GTPγS binding to membrane comprising said GPCR. Such assays are well known in the art and exemplified herein in Examples 12 and 14. In certain embodiments, said GTPγS is labeled with [$^{35}$S].

Also described herein are glycemic stabilizing compound identifiable according to the method of: a) contacting a candidate compound with GPR41, and b) determining whether GPR41 functionality is modulated, wherein a modulation in GPR41 functionality is indicative of the candidate compound being a glycemic stabilizing compound.

For example, a glycemic stabilizing compound may be identified according to the method of: a) contacting a candidate compound with GPR41, and b) determining whether GPR41 functionality is modulated, wherein a modulation in GPR41 functionality is indicative of the candidate compound being a glycemic stabilizing compound.

In one embodiment, said glycemic stabilizing compound is a GPR41 agonist. For example, said glycemic stabilizing compound comprises a compound selected from the group consisting of: 2-methyl-4-(4-nitro-phenyl)-5-oxo-1,4,5,6,7,3-hexahydro-quinoline-3-carboxylic acid o-tolylamide, cyclopropanecarboxylic acid 4-[1,2,3]thiadiazol-4-yl-phenyl ester, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

In some embodiments, said glycemic stabilizing compound is a GPR41 agonist. In some embodiments, said glycemic stabilizing compound is a GPR41 agonist with an EC50 of less than 10 μM, of less than 1 μM, of less than 100 nM, or of less than 10 nM. In some embodiments, said glycemic stabilizing compound is an agonist with an EC50 of a value selected from the interval of 1 nM to 10 μM. In some embodiments, said glycemic stabilizing compound is an agonist with an EC50 of a value selected from the interval of 1 nM to 1 μM. In some embodiments, said glycemic stabilizing compound is an agonist with an EC50 of a value selected from the interval of 1 nM to 100 nM. In some embodiments, said glycemic stabilizing compound is an agonist with an EC50 of a value selected from the interval of 1 nM to 10 nM.

In certain embodiments, said EC50 is determined using an assay selected from the group consisting of: IP3 assay carried out using transfected HEK293 cells expressing recombinant GPR41 polypeptide; and melanophore assay carried out using transfected melanophores expressing recombinant GPR 41 polypeptide. In some embodiments, said glycemic stabilizing compound is an agonist with an EC50 of less than 10 μM, of less than 1 μM, of less than 100 nM, or of less than 10 nM in said assay. In some embodiments, said glycemic stabilizing compound is an agonist with an EC50 of less than 10 μM, of less than 9 μM, of less than 8 μM, of less than 7 μM, of less than 6 μM, of less than 5 μM, of less than 4 μM, of less than 3 μM, of less than 2 μM, of less than 1 μM, of less than 900 nM, of less than 800 nM, of less than 700 nM, of less than 600 nM, of less than 500 nM, of less than 400 nM, of less than 300 nM, of less than 200 nM, of less than 100 nM, of less than 90 nM, of less than 80 nM, of less than 70 nM, of less than 60 nM, of less than 50 nM, of less than 40 nM, of less than 30 nM, of less than 20 nM, of less than 10 nM in said assay. In some embodiments, said glycemic stabilizing compound is an agonist with an EC50 in said assay of a value selected from the interval of 1 nM to 10 μM. In some embodiments, said glycemic stabilizing compound is an agonist with an EC50 in said assay of a value selected from the interval of 1 nM to 1 μM. In some embodiments, said glycemic stabilizing compound is an agonist with an EC50 in said assay of a value selected from the interval of 1 nM to 100 nM. In some embodiments, said glycemic stabilizing compound is an agonist with an EC50 in said assay of a value selected from the interval of 1 nM to 10 nM. In some embodiments, said glycemic stabilizing compound is selective for the GPCR.

In some embodiments, said glycemic stabilizing compound is a GPR41 inverse agonist or antagonist. For example, said glycemic compound can comprise a compound selected from the group consisting of: 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

In some embodiments, said glycemic stabilizing compound is a GPR41 inverse ' agonist or antagonist with an IC50 of less than 10 μM, of less than 1 μM, of less than 100 nM, or of less than 10 nM. In some embodiments, said glycemic stabilizing compound is an inverse agonist or antagonist with an IC50 of a value selected from the interval of 1 nM to 10 μM. In some embodiments, said glycemic stabilizing compound is an inverse agonist or antagonist with an IC50 of a value selected from the interval of 1 nM to 1 μM. In some embodiments, said glycemic stabilizing compound is an inverse agonist or antagonist with an IC50 of a value selected from the interval of 1 nM to 100 nM. In some embodiments, said glycemic stabilizing compound is an inverse agonist or antagonist with an IC50 of a value selected from the interval of 1 nM to 10 nM.

In certain embodiments, said IC50 is determined using an assay selected from the group consisting of: IP3 assay carried out using transfected HEK293 cells expressing recombinant GPR41 polypeptide; and melanophore assay carried out using transfected melanophores expressing recombinant GPR 41 polypeptide. In some embodiments, said glycemic stabilizing compound is an inverse agonist or antagonist with an IC50 of less than 10 μM, of less than 1 μM, of less than 100 nM, or of less than 10 nM in said assay. In some embodiments, said glycemic stabilizing compound is an inverse agonist or antagonist with an IC50 of less than 10 μM, of less than 9 μM, of less than 8 μM, of less than 7 μM, of less than 6 μM, of less than 5 μM, of less than 4 μM, of less than 3 μM, of less than 2 μM, of less than 1 μM, of less than 900 nM, of less than 800 nM, of less than 700 nM, of less than 600 nM, of less than 500 nM, of less than 400 nM, of less than 300 nM, of less than 200 nM, of less than 100 nM, of less than 90 nM, of less than 80 nM, of less than 70 nM, of less than 60 nM, of less than 50 nM, of less than 40 nM, of less than 30 nM, of less than 20 nM, of less than 10 nM in said assay. In some embodiments, said glycemic stabilizing compound is an inverse agonist or antagonist with an IC50 in said assay of a value selected from the interval of 1 nM to 10 μM. In some embodiments, said glycemic stabilizing compound is an inverse agonist or antagonist with an IC50 in said assay of a value selected from the interval of 1 nM to 1 μM. In some embodiments, said glycemic stabilizing compound is an inverse agonist or antagonist with an IC50 in said assay of a value selected from the interval of 1 nM to 100 nM. In some embodiments, said glycemic stabilizing compound is an inverse agonist or antagonist with an IC50 in said assay of a value selected from the interval of 1 nM to 10 nM. In some embodiments, said glycemic stabilizing compound is selective for the GPCR.

In some embodiments, said glycemic stabilizing compound is orally bioavailable. In some embodiments, said oral bioavailability is at least 1%, at least 5%, at least 10%, at least 15%, at least 20%, at least 25%, at least 30%, at least 35%, at least 40%, or at least 45% relative to intraperitoneal administration. In some embodiments, said oral bioavailability is at least 20%, at least 25%, at least 30%, at least 35%, at least 40%, or at least 45% relative to intraperitoneal administration. In some embodiments, said orally bioavailable glycemic stabilizing compound is further able to cross the blood-brain barrier.

In addition, described herein is a method for preparing a composition which comprises identifying a glycemic stabilizing compound and then admixing said compound with a carrier, wherein said compound is identifiable by the method of: a) contacting a candidate compound with GPR41, and b) determining whether GPR41 functionality is modulated, wherein a modulation in GPR41 functionality is indicative of the candidate compound being a glycemic stabilizing compound. For example, the invention provides a method for preparing a composition which comprises a glycemic stabilizing compound as listed above, said method including admixing said compound with a carrier. In addition, the invention provides a method for preparing a composition which comprises a glycemic stabilizing compound selected from the group consisting of: 2-methyl-4-(4-nitro-phenyl)-5-oxo-1,4,5,6,7,8-hexahydro-quinoline-3-carboxylic acid o-tolylamide, 4 Furan-3-yl-2 methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichlorophenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quilloline-3-carboxylic acid o-tolylamide, or a pharmaceutically acceptable salt thereof.

Also described is a pharmaceutical composition comprising, consisting essentially of, or consisting of the glycemic stabilizing compound identified according to the method of: a) contacting a candidate compound with GPR41, and b) determining whether GPR41 functionality is modulated, wherein a modulation in GPR41 functionality is indicative of the candidate compound being a glycemic stabilizing compound.

Some embodiments of the present invention include a method of producing a pharmaceutical composition comprising admixing at least one compound according to any of the compound embodiments disclosed herein and a pharmaceutically acceptable carrier.

A compound can be formulated into pharmaceutical compositions using techniques well known to those in the art. Suitable pharmaceutically-acceptable carriers, outside those mentioned herein, are available to those in the art; for example, see Remington's Pharmaceutical Sciences, 16th Edition, 1980, Mack Publishing Co., (Oslo et al., eds.).

While it is possible that, for use in the prophylaxis or treatment, a compound disclosed herein or identified by methods of the invention can in an alternative use be administered as a raw or pure chemical, it can be useful to present the compound or active ingredient as a pharmaceutical formulation or composition further comprising a pharmaceutically acceptable carrier.

The invention thus further provides pharmaceutical formulations comprising a compound disclosed herein or a pharmaceutically acceptable salt or derivative thereof together with one or more pharmaceutically acceptable carriers thereof and/or prophylactic ingredients. The carrier(s) are "acceptable" in the sense of being compatible with the other ingredients of the formulation and not overly deleterious to the recipient thereof.

Pharmaceutical formulations include those suitable for oral, rectal, nasal, topical (including buccal and sub-lingual), vaginal or parenteral (including intramuscular, subcutaneous and intravenous) administration or in a form suitable for administration by inhalation or insufflation.

The compounds of the invention, together with a conventional adjuvant, carrier, or diluent, can thus be placed into the form of pharmaceutical formulations and unit dosages thereof, and in such form can be employed as solids, such as tablets or filled capsules, or liquids such as solutions, suspensions, emulsions, elixirs, gels or capsules filled with the same, all for oral use, in the form of suppositories for rectal administration; or in the form of sterile injectable solutions for parenteral (including subcutaneous) use. Such pharmaceutical compositions and unit dosage forms thereof can comprise conventional ingredients in conventional proportions, with or without additional active compounds or principles, and such unit dosage forms can contain any suitable effective amount of the active ingredient commensurate with the intended daily dosage range to be employed.

For oral administration, the pharmaceutical composition can be in the form of, for example, a tablet, capsule, suspension or liquid. The pharmaceutical composition can be made in the form of a dosage unit containing a particular amount of the active ingredient. Examples of such dosage units are capsules, tablets, powders, granules or a suspension, with conventional additives such as lactose, mannitol, com starch or potato starch; with binders such as crystalline cellulose, cellulose derivatives, acacia, corn starch or gelatins; with disintegrators such as com starch, potato starch or sodium carboxymethyl-cellulose; and with lubricants such as talc or magnesium stearate. The active ingredient can also be administered by injection as a composition wherein, for example, saline, dextrose or water can be used as a suitable pharmaceutically acceptable carrier.

Described herein is a method for selectively activating a GPR41 receptor in a human host, comprising administering a compound that selectively activates the GPR41 gene product in a human host in need of such treatment. For example, the compound can be a GPR41 agonist such as 2-methyl-4-(4-nitro-phenyl)-5-oxo-1,4,5,6,7,8-hexahydro-quinoline-3-carboxylic acid o-tolylamide, cyclopropanecarboxylic acid 4-[1,3,3]thiadiazol-4-yl-phenyl ester, cyclopropanecarboxylic acid; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

Also described is a method for selectively inhibiting a GPR41 receptor in a human host, comprising administering a compound that selectively inhibits the GPR41 gene product in a human host in need of such treatment. For example, the compound can be a GPR41 inverse agonist such as 2-Methyl-4-[5-(2-nitro-4-trifluoromethyl-phenyl)-furan-2-yl]-5-oxo-1,4,5,6,7,8-hexahyclio-quinoline-3-carboxylic acid o-tolylamide, 4-(5-Biplienyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

Further described herein is a method for treating or preventing an insulin-related disorder in an individual in need thereof, comprising administering to said individual an effective amount of the compound identified according to the method of: a) contacting a candidate compound with GPR41, and b) determining whether GPR41 functionality is modulated, wherein a modulation in GPR41 functionality is indicative of the candidate compound being a glycemic stabilizing compound. Preferably, said insulin-related disorder is hypoglycemia, an insulin-secreting or insulin-dependent tumor, aging, insulin resistance, impaired glucose tolerance, or diabetes. In some instances, said insulin-related disorder includes a condition related to an elevated blood glucose concentration, such as atherosclerosis, heart disease, stroke, hypertension, obesity, Syndrome X or peripheral vascular disease. In some embodiments, said insulin-related disorder is Type II diabetes. Preferably, the compound administered comprises a GPR41 agonist. Alternatively, the compound

administered comprises a GPR41 inverse agonist or antagonist.

The method further comprises administering to said individual an effective amount of an agent used for the treatment of diabetes, blood lipid disorders, or obesity in combination with an effective amount of the pharmaceutical composition comprising, consisting essentially of, or consisting of the glycemic stabilizing compound identified according to the method of: a) contacting a candidate compound with GPR41, and b) determining whether GPR41 functionality is modulated, wherein a modulation in GPR41 functionality is indicative of the candidate compound being a glycemic stabilizing compound. For example, the method further comprises administering to said individual an effective amount of an agent used for the treatment of diabetes, blood lipid disorders, or obesity in combination with an effective amount of a pharmaceutical composition containing a GPR41 inverse agonist. The individual may be a mammal and preferably the individual is a human.

As used herein the term "treating" in reference to a disorder means a reduction in severity of one or more symptoms associated with a particular disorder. Therefore, treating a disorder does not necessarily mean a reduction in severity of all symptoms associated with a disorder and does not necessarily mean a complete reduction in the severity of one or more symptoms associated with a disorder. Similarly, the term "preventing" means prevention of the occurrence or onset of one or more symptoms associated with a particular disorder and does not necessarily mean the complete prevention of a disorder. The methods can be used to treat an insulin-related disorder including, for example, hypoglycemia or diabetes.

The dose when using the compounds disclosed herein or identified by methods can vary within wide limits, and as is customary and is known to the physician, it is to be tailored to the individual conditions in each individual case. It depends, for example, on the nature and severity of the illness to be treated, on the condition of the patient, on the compound employed or on whether an acute or chronic disease state is treated or prophylaxis is conducted or on whether further active compounds are administered in addition to the compounds disclosed herein or identified by methods of the invention. Representative doses of the present invention include, about 0.01 mg to about 1000 mg, about 0.01 to about 750 mg, about 0.01 to about 500 mg, 0.01 to about 250 mg, 0.01 mg to about 200 mg, about 0.01 mg to 150 mg, about 0.01 mg to about 100 mg, and about 0.01 mg to about 75 mg. Multiple doses can be administered during the day, especially when relatively large amounts are deemed to be needed, for example 2, 3 or 4, doses. If appropriate, depending on individual behavior and as appropriate from the patients physician or care-giver it can be necessary to deviate upward or downward from the daily dose.

The amount of active ingredient, or an active salt or derivative thereof, required for use in treatment will vary not only with the particular salt selected but also with the route of administration, the nature of the condition being treated and the age and condition of the patient and will ultimately be at the discretion of the attendant physician or clinician. In general, one skilled in the art understands how to extrapolate *in vivo* data obtained in a model system, typically an animal model, to another, such as a human. Typically, animal models include, but are not limited to, the rodent diabetes models as described in Example 19, *infra* (other animal models have been reported by Reed and Scribner in Diabetes, Obesity and Metabolism, 1:75-86 (1999)). In some circumstances, these extrapolations can merely be based on the weight of the animal model in comparison to another, such as a mammal, for example, a human, however, more often, these extrapolations are not simply based on weights, but rather incorporate a variety of factors. Representative factors include the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized, on whether an acute or chronic disease state is being treated or prophylaxis is conducted or on whether further active compounds are administered in addition to the compounds disclosed herein or identified by methods of the invention and as part of a drug combination. The dosage regimen for treating a disease condition with the compounds and/or compositions of this invention is selected in accordance with a variety factors as cited above. Thus, the actual dosage regimen employed can vary widely and therefore can deviate from a preferred dosage regimen and one skilled in the art will recognize that dosage and dosage regimen outside these typical ranges can be tested and, where appropriate, can be used.

The desired dose can conveniently be presented in a single dose or as divided doses administered at appropriate intervals, for example, as two, three, four or more sub-doses per day. The sub-dose itself can be further divided, e.g., into a number of discrete loosely spaced administrations. The daily dose can be divided, especially when relatively large amounts are administered as deemed appropriate, into several, for example 2, 3 or 4, part administrations. If appropriate, depending on individual behavior, it can be necessary to deviate upward or downward from the daily dose indicated.

The compounds disclosed herein can be administrated in a wide variety of oral and parenteral dosage forms. It will be obvious to those skilled in the art that the following dosage forms can comprise, as the active component, either a compound disclosed herein or identified by methods of the invention or a pharmaceutically acceptable salt of a compound disclosed herein.

For preparing pharmaceutical compositions from the compounds disclosed herein, the selection of a suitable pharmaceutically acceptable carrier can be either solid, liquid or a mixture of both. Solid form preparations include powders, tablets, pills, capsules, cachets, suppositories, and dispersible granules. A solid carrier can be one or more

substances which can also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component. In tablets, the active component is mixed with the carrier having the necessary binding capacity in suitable proportions and compacted to the desire shape and size.

The powders and tablets can contain varying percentage amounts of the active compound. A representative amount in a powder or tablet can contain from 0.5 to about 90 percent of the active compound; however, an artisan would know when amounts outside of this range are necessary. Suitable carriers for powders and tablets are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as an admixture of fatty acid glycerides or cocoa butter, is first melted and the active component is dispersed homogeneously therein, as by stirring. The molten homogenous mixture is then poured into convenient sized molds, allowed to cool, and thereby to solidify.

Formulations suitable for vaginal administration can be presented as pessaries, tampons, creams, gels, pastes, foams or sprays containing in addition to the active ingredient such carriers as are known in the art to be appropriate.

Liquid form preparations include solutions, suspensions, and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions can be formulated according to the known art using suitable dispersing or wetting agents and suspending agents. The sterile injectable preparation can also be a sterile injectable solution or suspension in a nontoxic parenterally acceptable diluent or solvent, for example, as a solution in 1,3-butanediol. Among the acceptable vehicles and solvents that can be employed are water, Ringer's solution, and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid find use in the preparation of injectables.

The compounds according to the present invention can thus be formulated for parenteral administration (e.g. by injection, for example bolus injection or continuous infusion) and can be presented in unit dose form in ampoules, pre-filled syringes, small volume infusion or in multi-dose containers with an added preservative. The pharmaceutical compositions can take such forms as suspensions, solutions, or emulsions in oily or aqueous vehicles, and can contain formulatory agents such as suspending, stabilizing and/or dispersing agents. Alternatively, the active ingredient can be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavors, stabilizing and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations can contain, in addition to the active component, colorants, flavors, stabilizers, buffers, artificial and natural sweeteners, dispersants, thickeners, solubilizing agents, and the like.

For topical administration to the epidermis the compounds according to the invention can be formulated as ointments, creams or lotions, or as a transdermal patch.

Ointments and creams can, for example, be formulated with an aqueous or oily base with the addition of suitable thickening and/or gelling agents. Lotions can be formulated with an aqueous or oily base and will in general also contain one or more emulsifying agents, stabilizing agents, dispersing agents, suspending agents, thickening agents, or coloring agents.

Formulations suitable for topical administration in the mouth include lozenges comprising active agent in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

Solutions or suspensions are applied directly to the nasal cavity by conventional means, for example with a dropper, pipette or spray. The formulations can be provided in single or multi-dose form. In the latter case of a dropper or pipette, this can be achieved by the patient administering an appropriate, predetermined volume of the solution or suspension. In the case of a spray, this can be achieved for example by means of a metering atomizing spray pump.

Administration to the respiratory tract can also be achieved by means of an aerosol formulation in which the active ingredient is provided in a pressurized pack with a suitable propellant. If the compounds disclosed herein or identified by methods of the invention or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds disclosed herein or identified by methods of the invention as an aerosol can be prepared by processes well-known to the person skilled in the art. For their preparation, for example, solutions or dispersions of the compounds disclosed herein or identified by methods of the invention in water, water/alcohol mixtures or suitable saline solutions can be employed using customary additives, for example benzyl alcohol or other suitable preservatives, absorption enhancers for increasing the bioavailability, solubilizers, dispersants and others, and, if appropriate, customary propellants, for example include carbon dioxide, CFC's, such as, dichlorodifluoromethane, trichlorofluoromethane, or dichlorotetrafluoroethane; and the like. The aerosol can conveniently also contain a surfactant such as lecithin. The dose of drug can be controlled by provision of a metered valve.

In formulations intended for administration to the respiratory tract, including intranasal formulations, the compound will generally have a small particle size for example of the order of 10 microns or less. Such a particle size can be obtained by means known in the art, for example by micronization. When desired, formulations adapted to give sustained release of the active ingredient can be employed.

Alternatively the active ingredients can be provided in the form of a dry powder, for example, a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition can be presented in unit dose form for example in capsules or cartridges of, for example, gelatin, or blister packs from which the powder can be administered by means of an inhaler.

The pharmaceutical preparations can be in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are particularly useful compositions.

Insulin-related disorders include, for example, hypoglycemia, an insulin-secreting or insulin-dependent tumor, aging, insulin resistance, impaired glucose tolerance, or diabetes.

Hypoglycemia is defined as abnormally low blood glucose. Hypoglycemia can result, for example, from excessive insulin or a poor diet. For example, hypoglycemia can occur when a person with diabetes has injected too much insulin, eaten too little food, or has exercised without extra food. Symptoms of hypoglycemia include, for example, a feeling of nervousness or weakness, headache, blurred vision, hunger, and excessive sweatiness.

Insulin-secreting tumors include, for example, insulinomas. An insulinoma is a tumor of the beta cells in areas of the pancreas called the islets of Langerhans. Although not usually cancerous, such tumors may cause the body to make extra insulin and may lead to a blood glucose level that is too low. In addition to insulin-secreting tumors, some tumors that do not secrete insulin can use insulin as a growth factor. While insulin may or may not be the sole growth factor used by the tumor, reduction in the amount of insulin in the body may reduce the growth of the tumor.

Aging is the physiological processes that occur in an organism as it gets older. Caloric restriction down-regulates insulin secretion and there is reason to suspect that these effects are key mediators of caloric restriction's favorable impact on longevity. Thus, strategies for down-regulating insulin can be useful to slow the process of aging and increase longevity.

Diabetes and related conditions such as insulin resistance and impaired glucose tolerance have been described above herein.

In addition, insulin resistance is a common feature of polycystic ovary syndrome (PCOS) and drugs such as rosiglitazone and metformin has been used in the treatment of PCOS (Sepilian and Nagamani J. Clin. Endocrinol Metab. Oct. 14, 2003; Baillargeon et al., Fertil. Steril. 82:893-902 (2004)). PCOS is characterized, for example, by bilaterally enlarged polycystic ovaries, amenorrhea, and infertility. It is inherited as an autosomal dominant condition. Other symptoms of the disease can include, for example, hirsutism and obesity. Hormonally, PCOS is characterized, for example, by increased secretion of leutinizing hormone, insulin and androgens.

Another indication for the compounds of the invention is treatment of lipodystrophy, for example, as caused by anti-retroviral therapy for HIV infection. Some patients on long term AIDS therapy known as highly active anti-retroviral therapy (HAART) are increasingly developing a syndrome called lipodystrophy. Symptoms include insulin sensitivity, the redistribution of fat from the face, arms and legs to the abdomen and upper back, and cholesterol changes. About 14 percent of people on HAART eventually develop type 2 diabetes. The drug rosiglitazone has been shown to improve insulin sensitivity in HIV-positive patients who received the treatment for three months. Patients had about a 20 percent

improvement on a standard test to measure insulin sensitivity and also increased their total body fat, particularly the amount of fat on their face, arms and legs, which went up by 24 percent. By comparison, patients taking the placebo had a 2 percent decrease in face, arm and leg fat.

In some embodiments, said insulin-related disorder includes a condition related to an elevated blood glucose concentration, such as atherosclerosis, heart disease, stroke, hypertension, obesity, Syndrome X and peripheral vascular disease.

Atherosclerosis is a process where deposits of fatty substances, cholesterol and other substances build up in the inner lining of an artery. This buildup is called plaque. Plaques that rupture cause blood clots to form that can block blood flow to the heart (heart attack) or the brain (stroke). Heart attack is the number one cause of death for both men and women in the United States and stroke is the number three cause of death [see, for example, Nature Medicine, Special Focus on Atherosclerosis, (2002) 8:1209-1262]. Abnormally high levels of circulating lipids are a major predisposing factor in development of atherosclerosis. Elevated levels of low density lipoprotein (LDL) cholesterol, elevated levels of triglycerides, or low levels of high density lipoprotein (HDL) cholesterol are, independently, risk factors for atherosclerosis and associated pathologies.

Heart disease includes, but is not limited to, cardiac insufficiency, coronary insufficiency, coronary artery disease, and high blood pressure (hypertension). Peripheral vascular disease refers to diseases of blood vessels outside the heart and brain. Organic peripheral vascular diseases are caused by structural changes in the blood vessels, such as inflammation and tissue damage. Peripheral artery disease is an example. Peripheral artery disease (PAD) is a condition similar to coronary artery disease and carotid artery disease. In PAD, fatty deposits build up along artery walls and affect blood circulation, mainly in arteries leading to the legs and feet. In its early stages a common symptom is cramping or fatigue in the legs and buttocks during activity. Such cramping subsides when the person stands still. This is called "intermittent claudication." People with PAD have a higher risk of death from stroke and heart attack, due to the risk of blood clots.

Syndrome X, also called metabolic syndrome, is characterized by a group of metabolic risk factors in one person. They include: central obesity (excessive fat tissue in and around the abdomen), atherogenic dyslipidemia (blood fat disorder - mainly high triglycerides and low HDL cholesterol), raised blood pressure (130/85 mmHg or higher), insulin resistance or glucose intolerance, prothrombotic state (e.g., high fibrinogen or plasminogen activator inhibitor [-1] in the blood), and proinflammatory state (e.g., elevated high-sensitivity C-reactive protein in the blood).

While the compounds disclosed herein can be administered as the sole active pharmaceutical agent as described herein above, they can also be used in combination with one or more agents including, for example, agents that are used for the treatment of diabetes, blood lipid disorders, or obesity. For example, a compound such as a GPR41 inverse agonist or antagonist can be used in combination with one or more agents belonging to the class of drugs known as α-glucosidase inhibitors, aldose reductase inhibitors, biguanides, thiazolidinediones, meglitinides, sulfonylureas, insulin, HMG-CoA reductase inhibitors, squalene synthesis inhibitors, fibrate compounds, LDL catabolism enhancers, angiotensin converting enzyme (ACE) inhibitors, lipase inhibitors, serotonin and/or noradrenaline releasers or reuptake inhibitors.

α-Glucosidase inhibitors belong to the class of drugs which competitively inhibit digestive enzymes such as α-amylase, maltase, α-dextrinase, sucrase, etc. in the pancreas and or small intestine. The reversible inhibition by α-glucosidase inhibitors retard, diminish or otherwise reduce blood glucose levels by delaying the digestion of starch and sugars. Some representative examples of α-glucosidase inhibitors include acarbose, N-(1,3-dihydroxy-2-propyl)valiolamine (generic name; voglibose), miglitol, and α-glucosidase inhibitors known in the art.

The class of aldose reductase inhibitors are drugs which inhibit the first-stage rate-limiting enzyme in the polyol pathway and thereby prevent or arrest diabetic complications. In the hyperglycemic state of diabetes, the utilization of glucose in the polyol pathway is increased and the excess sorbitol accumulated intracellularly as a consequence acts as a tissue toxin and hence evokes the onset of complications such as diabetic neuropathy, retinopathy, and nephropathy. Examples of the aldose reductase inhibitors include tolurestat; epalrestat; 3,4-dihydro-2,8-diisopropyl-3-thioxo-2H-1,4-benzoxazine-4-acetic acid; 2,7-difluorospiro(9H-fluorene-9,4'-imidazolidine)-2',5'-dione (generic name: imirestat); 3-[(4-bromo-2-flurophenyl)methy]-7-chloro-3,4-dihydro-2,4-dioxo-1(2H)-quinazoline acetic acid (generic name: zenarestat); 6-fluoro-2,3-dihydro-2',5'-dioxo-spiro[4H-1-benzopyran-4,4'-imidazolidine]-2-carboxamide (SNK-860); zopolrestat; sorbinil; and 1-[(3-bromo-2-benzofuranyl)sulfonyl]-2,4-imidazolidinedione (M-16209), and aldose reductase inhibitors known in the art.

The biguanides are a class of drugs that stimulate anaerobic glycolysis, increase the sensitivity to insulin in the peripheral tissues, inhibit glucose absorption from the intestine, suppress of hepatic gluconeogenesis, and inhibit fatty acid oxidation. Examples of biguanides include phenformin, metformin, buformin, and biguanides known in the art.

Insulin secretion enhancers belong to the class of drugs having the property to promote secretion of insulin from pancreatic β cells. Examples of the insulin secretion enhancers include sulfonylureas (SU). The sulfonylureas (SU) are drugs which promote secretion of insulin from pancreatic β cells by transmitting signals of insulin secretion via SU receptors in the cell membranes. Examples of the sulfonylureas include tolbutamide; chlorpropamide; tolazamide; acetohexamide; 4-chloro-N-[(1-pyrolidinylamino) carbonyl]-benzenesulfonamide (generic name: glycopyramide) or its ammonium salt; glibenclamide (glyburide); gliclazide; 1-butyl-3-metanilylurea; carbutamide; glibonuride; glipizide; gliquidone; glisoxepid; glybuthiazole; glibuzole; glyhexamide; glymidine; glypinamide; phenbutamide; tolcyclamide, glimepiride, and other insulin secretion enhancers known in the art. Other insulin secretion enhancers include N-[[4-(1-methylethyl)cyclohexyl)carbonyl]-D-phenylalanine (Nateglinide); calcium (2S)-2-benzyl-3-(cis-hexahydro-2-isoindolinylcarbonyl)propionate dihydrate (Mitiglinide, KAD-1229); and other insulin secretion enhancers known in the art.

Thiazolidinediones belong to the class of drugs more commonly known as TZDs. Thiazolidinediones are a class of drugs for type 2 diabetes that lower the blood sugar by increasing the sensitivity of cells to insulin. Insulin can then move glucose from the blood into cells for energy. These drugs can also increase HDL.

Examples of thiazolidinediones include rosiglitazone, pioglitazone, and thiazolidinediones known in the art. Rezulin (troglitazone) was the first drug in this class in the U.S., but was taken off the market because of liver toxicity. Sister compounds now available with a better safety profile include Actos (pioglitazone) and Avandia (rosiglitazone). The main contraindications to the use of these medications include liver disease and heart failure. These drugs can also cause a significant increase in fluid retention and thereby increase the risk of heart failure.

Meglitinides are used to stop the rapid rise in blood sugar that can occur immediately after a person with type 2 diabetes eats a meal. These compounds, which include, for example, repaglinide (Prandin) and nateglinide (Starlix), work by increasing the amount of insulin produced by the pancreas similar to the way sulfonyurea medications work. Meglitinides are taken before eating a meal. Side effects associated with this class of drugs includes low blood sugar, upper respiratory infections including sinus conditions, headache, joint and back pain, nausea, diarrhea and constipation.

The different types of insulin are categorized according to how fast they start to work (onset) and how long they continue to work (duration). The types now available include rapid-, short-, intermediate-, and long-acting insulin. There are premixed rapid- and intermediate-acting insulins available, including: 70% intermediate-acting (NPH) and 30% short-acting regular insulin, called 70/30 insulin; 50% intermediate-acting (NPH) and 50% short-acting regular insulin, called 50/50 insulin; 75% intermediate-acting (NPH) and 25% rapid-acting Humalog (lispro), called 75/25 insulin; 70% intermediate-acting (NPH); and 30% rapid-acting NovoLog (insulin aspart), called NovoLog Mix 70/30. Insulin usually is given as an injection into the tissues under the skin (subcutaneous). It can also be given through an insulin pump or jet injector, a device that sprays the medication into the skin.

Insulin lets sugar (glucose) enter cells, where it is used for energy. Without insulin, the blood sugar level rises above what is safe for the body. Usually, a rapid- or short-acting and an intermediate- or long-acting insulin is taken to provide the constant and variable levels of insulin that the body needs. The short-acting insulin reduces blood sugar levels quickly and then wears off. Some long-acting insulins start taking effect when rapid- or short-acting insulins begin to wear off. The new long-acting insulin, Lantus, starts to work within a few minutes after it is given and continues to work at the same rate for about 24 hours.

The combination of a rapid- or short-acting and intermediate- or long-acting insulin helps keep blood sugar levels within a range that is safe for the body throughout the day. Thus insulin can be used to treat people with type 1 diabetes, people with type 2 diabetes whose pancreas produces little or no insulin or whose oral medications do not control their blood sugar. These people may take insulin either alone or along with oral medication, people with type 2 diabetes whose blood sugar levels are high because of a severe illness or major surgery, women with type 2 diabetes who are pregnant or breast-feeding who cannot keep their blood sugar levels within a safe range with diet and exercise. Only one oral diabetes medication (glyburide) has been studied for use during pregnancy.

The major side effect of insulin can be a dangerously low blood sugar level (severe hypoglycemia). A very low blood sugar level can develop within 10 to 15 minutes. Insulin can contribute to weight gain, especially in people with type 2 who already are overweight. Other possible side effects of long-term insulin use include the loss of fatty tissue (lipodystrophy) where the insulin is injected and, rarely, allergic reactions that include swelling (edema).

Statin compounds belong to a class of drugs that lower blood cholesterol levels by inhibiting hydroxymethylglutalyl CoA (HMG-CoA) reductase. HMG-CoA reductase is the rate-limiting enzyme in cholesterol biosynthesis. A statin that inhibits this reductase lowers serum LDL concentrations by upregulating the activity of LDL receptors and responsible for clearing LDL from the blood. Examples of the statin compounds include rosuvastatin, pravastatin and its sodium salt, simvastatin, lovastatin, atorvastatin, fluvastatin, cerivastatin, and HMG-CoA reductase inhibitors known in the art.

Squalene synthesis inhibitors belong to a class of drugs that lower blood cholesterol levels by inhibiting synthesis of squalene. Examples of the squalene synthesis inhibitors include (S)-$\alpha$-[Bis[2,2-dimethyl-1-oxopropoxy)methoxy] phosphinyl]-3-phenoxybenzenebutanesulfonic acid, mono potassium salt (BMS-188494) and squalene synthesis inhibitors known in the art.

Fibrate compounds belong to a class of drugs that lower blood cholesterol levels by inhibiting synthesis and secretion of triglycerides in the liver and activating a lipoprotein lipase. Fibrates have been known to activate peroxisome proliferators-activated receptors and induce lipoprotein lipase expression. Examples of fibrate compounds include bezafibrate, beclobrate, binifibrate, ciplofibrate, clinofibrate, clofibrate, clofibric acid, etofibrate, fenofibrate, gemfibrozil, nicofibrate, pirifibrate, ronifibrate, simfibrate, theofibrate, and fibrates known in the art.

LDL (low-density lipoprotein) catabolism enhancers belong to a class of drugs that lower blood cholesterol levels by increasing the number of LDL (low-density lipoprotein) receptors, examples include LDL catabolism enhancers known in the art.

Angiotensin converting enzyme (ACE) inhibitors belong to the class of drugs that partially lower blood glucose levels as well as lowering blood pressure by inhibiting angiotensin converting enzymes. Examples of the angiotensin converting enzyme inhibitors include captopril, enalapril, alacepril, delapril; ramipril, lisinopril, imidapril, benazepril, ceronapril, cilazapril, enalaprilat, fosinopril, moveltopril, perindopril, quinapril, spirapril, temocapril, trandolapril, and angiotensin converting enzyme inhibitors known in the art.

Lipase inhibitors include, for example, anti-obesity compounds such as Orlistat (XENICAL™). Orlistat inhibits fat absorption directly but also tends to produce a high incidence of unpleasant gastric side-effects such as diarrhea and flatulence.

Another class of anti-obesity drugs includes serotonin and/or noradrenaline releasers or reuptake inhibitors. For example, sibutramine (Meridia™) is a mixed 5-HT/noradrenaline reuptake inhibitor. The main side effect of sibutramine can be an increase in blood pressure and heart rate in some patients. The serotonin releaser/reuptake inhibitors fenfluramine (Pondimin™) and dexfenfluramine (Redux™) have been reported to decrease food intake and body weight over a prolonged period (greater than 6 months). However, both products were withdrawn from use after reports of preliminary evidence of heart valve abnormalities associated with their use.

Some embodiments of the invention include, a pharmaceutical composition comprising a compound disclosed herein or a pharmaceutically acceptable salt thereof in combination with at least one member selected from the group consisting of an $\alpha$-glucosidase inhibitor, an aldose reductase inhibitor, a biguanide, a HMG-CoA reductase inhibitor, a squalene synthesis inhibitor, a fibrate compound, a LDL catabolism enhancer and an angiotensin converting enzyme inhibitor. In another embodiment, the HMG-CoA reductase inhibitor is selected from the group consisting of prevastatin, simvastatin, lovastatin, atorvastatin, fluvastatin and lipitor.

In accordance with the present invention, the combination can be used by mixing the respective active components either all together or independently with a physiologically acceptable carrier, excipient, binder, diluent, etc., as described herein above, and administering the mixture or mixtures either orally or non-orally as a pharmaceutical composition. When a compound or a mixture of compounds are administered as a combination therapy or prophylaxis with another active compound the therapeutic agents can be formulated as a separate pharmaceutical compositions given at the same time or at different times, or the therapeutic agents can be given as a single composition.

Described herein is a method for the manufacture of a medicament comprising a pharmaceutical composition comprising, consisting essentially of, or consisting of the glycemic stabilizing compound identified according to the method of: a) contacting a candidate compound with GPR41, and b) determining whether GPR41 functionality is modulated, wherein a modulation in GPR41 functionality is indicative of the candidate compound being a glycemic stabilizing compound, for use as a glycemic stabilizing compound.

Also described is a method for the manufacture of a medicament comprising a pharmaceutical composition comprising, consisting essentially of, or consisting of the glycemic stabilizing compound identified according to the method of: a) contacting a candidate compound with GPR41, and b) determining whether GPR41 functionality is modulated, wherein a modulation in GPR41 functionality is indicative of the candidate compound being a glycemic stabilizing compound, for use in the treatment of an insulin-related disorder.

The invention relates to a method for identifying a glycemic stabilizing compound, comprising: a) contacting a candidate compound with GPR41, and b) determining whether GPR41 functionality is increased, wherein an increase in GPR41 functionality is indicative of the candidate compound being a glycemic stabilizing compound.

In one embodiment, said GPR41 is derived from a mammal. In another embodiment, said GPR41 is human.

In certain embodiments, said GPR41 is recombinant. In certain embodiments, said contacting comprises contacting with a host cell or with membrane of a host cell that expresses the GPCR, wherein the host cell comprises an expression vector comprising a polynucleotide encoding the receptor. In some embodiments, said contacting is carried out in the presence of a known agonist of the GPCR or an agonist as disclosed herein.

In certain embodiments, said method further comprises the step of comparing the increase in functionality of the receptor caused by the candidate compound to a second increase in functionality of the receptor caused by contacting the receptor with a known ligand or agonist of the receptor.

In some embodiments, said determining comprises a second messenger assay, for example, determining is through the measurement of GTP$\gamma$S binding to membrane comprising said GPCR In certain embodiments, said GTP$\gamma$S is labeled with [$^{35}$S]. In certain embodiments, said determining is through the measurement of the level of a second messenger selected from the group consisting of cyclic AMP (cAMP), cyclic GMP (cGMP), inositol triphosphate (IP3), diacylglycerol (DAG), MAP kinase activity, and $Ca^{2+}$. In certain embodiments, said second messenger is cAMP. In certain embodiments, said measurement of cAMP is carried out using whole-cell adenylyl cyclase assay. In certain embodiments, said measurement of cAMP is carried out with membrane comprising said GPCR. In certain embodiments, said determining is through measurement of intracellular IP3. In certain embodiments, said determining further includes

the use of a chimeric G-protein such as a Gq/Gi chimera. In certain embodiments, said second messenger is MAP kinase activity. In some embodiments, said determining is through CRE-reporter assay. In certain embodiments, said reporter is luciferase. In some embodiments, said reporter is β-galactosidase. In certain embodiments, said determining or said comparing is through measurement of intracellular $Ca^{2+}$.

In some embodiments, said determining is through measurement of glucose uptake by adipocytes obtained from a mammal. In some embodiments, said determining is through measurement of glucose uptake by skeletal muscle cells obtained from a mammal.

In certain embodiments, said determining is through the use of a melanophore assay.

In some embodiments, said glycemic stabilizing compound comprises a compound selected from the group consisting of: 2-methyl-4-(4-nitro-phenyl)-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichlorophenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-anide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

The invention also relates to a method of identifying a candidate compound as an inhibitor of insulin secretion, comprising a) contacting a candidate compound with GPR41, and b) determining whether GPR41 functionality is increased, wherein an increase in GPR41 functionality is indicative of the candidate compound being an inhibitor of insulin secretion. For example, a compound that increases GPR41 functionality, such as a GPR41 agonist, can result in a decrease in insulin secretion. A decrease in insulin secretion can be desired, for example, in individuals with hypoglycemia.

The invention also relates to a method of identifying a candidate compound that results in an increase of blood glucose concentration, comprising a) contacting a candidate compound with GPR41, and b) determining whether GPR41 functionality is increased, wherein an increase in GPR41 functionality is indicative of the candidate compound resulting in an increase of blood glucose concentration. For example, a compound that increases GPR41 functionality, such as a GPR41 agonist, can result in a decrease in insulin secretion and an increase in blood glucose concentration. An increase in blood glucose can be desired, for example, in individuals with hypoglycemia.

In certain embodiments, said GPR41 is recombinant. In certain embodiments, said contacting comprises contacting with a host cell or with membrane of a host cell that expresses the GPCR, wherein the host cell comprises an expression vector comprising a polynucleotide encoding the receptor. In some embodiments, said contacting is carried out in the presence of an agonist of the GPCR.

In the methods of the invention, control reactions can be performed to show specificity of the response. For example, mock-transfected cells can be compared to GPR41 transfected cells to show specificity of a response to the GPR41 receptor.

In the methods of the invention, in certain embodiments, said candidate compound is not an antibody or antigen-binding derivative thereof. In certain embodiments, said candidate compound is not a peptide. In certain embodiments, said candidate compound is not a polypeptide.

In the methods of the invention, determining can comprise a second messenger assay. The initiation of an intracellular signal can be determined, for example, through the measurement of the level of a second messenger such as cyclic AMP (cAMP), cyclic GMP (cGMP), inositol triphosphate (IP3), diacylglycerol (DAG), MAP kinase, or calcium. Several assays are well known in the art for measuring these second messengers, for example, cAMP assays, IP3 assays, the FLIPR assay, the melanophore assay, or CRE-reporter assay. In addition, examples of second messenger assays are disclosed herein in Examples 12-17. In certain embodiments, said second messenger is cAMP. In other embodiments, said second messenger is IP3. In further embodiments said second messenger is calcium.

In one embodiment, said determining is through the measurement of GTPγS binding to membrane comprising said GPCR. Such assays are well known in the art and exemplified herein in Examples 12 and 14. In certain embodiments, said GTPγS is labeled with [$^{35}S$].

In one embodiment, said glycemic stabilizing compound is a GPR41 agonist. For example, said glycemic stabilizing compound comprises a compound selected from the group consisting of: 2-methyl-4-(4-nitro-phenyl)-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

In some embodiments, said glycemic stabilizing compound is a GPR41 agonist. In some embodiments, said glycemic stabilizing compound is a GPR41 agonist with an EC50 of less than 10 μM, of less than 1 μM, of less than

100 nM, or of less than 10 nM. In some embodiments, said glycemic stabilizing compound is an agonist with an EC50 of a value selected from the interval of 1 nM to 10 μM. In some embodiments, said glycemic stabilizing compound is an agonist with an EC50 of a value selected from the interval of 1 nM to 1 μM. In some embodiments, said glycemic stabilizing compound is an agonist with an EC50 of a value selected from the interval of 1 nM to 100 nM. In some embodiments, said glycemic stabilizing compound is an agonist with an EC50 of a value selected from the interval of 1 nM to 10 nM.

In certain embodiments, said EC50 is determined using an assay selected from the group consisting of: IP3 assay carried out using transfected HEK293 cells expressing recombinant GPR41 polypeptide; and melanophore assay carried out using transfected melanophores expressing recombinant GPR41 polypeptide. In some embodiments, said glycemic stabilizing compound is an agonist with an EC50 of less than 10 μM, of less than 1 μM, of less than 100 nM, or of less than 10 nM in said assay. In some embodiments, said glycemic stabilizing compound is an agonist with an EC50 of less than 10 μM, of less than 9 μM, of less than 8 μM, of less than 7 μM, of less than 6 μM, of less than 5 μM, of less than 4 μM, of less than 3 μM, of less than 2 μM, of less than 1 μM, of less than 900 nM, of less than 800 nM, of less than 700 nM, of less than 600 nM, of less than 500 nM, of less than 400 nM, of less than 300 nM, of less than 200 nM, of less than 100 nM, of less than 90 nM, of less than 80 nM, of less than 70 nM, of less than 60 nM, of less than 50 nM, of less than 40 nM, of less than 30 nM, of less than 20 nM, of less than 10 nM in said assay. In some embodiments, said glycemic stabilizing compound is an agonist with an EC50 in said assay of a value selected from the interval of 1 nM to 10 μM. In some embodiments, said glycemic stabilizing compound is an agonist with an EC50 in said assay of a value selected from the interval of 1 nM to 1 μM. In some embodiments, said glycemic stabilizing compound is an agonist with an EC50 in said assay of a value selected from the interval of 1 nM to 100 nM. In some embodiments, said glycemic stabilizing compound is an agonist with an EC50 in said assay of a value selected from the interval of 1 nM to 10 nM. In some embodiments, said glycemic stabilizing compound is selective for the GPCR.

In some embodiments, said glycemic stabilizing compound is orally bioavailable. In some embodiments, said oral bioavailability is at least 1%, at least 5%, at least 10%, at least 15%, at least 20%, at least 25%, at least 30%, at least 35%, at least 40%, or at least 45% relative to intraperitoneal administration. In some embodiments, said oral bioavailablity is at least 20%, at least 25%, at least 30%, at least 35%, at least 40%, or at least 45% relative to intraperitoneal administration. In some embodiments, said orally bioavailable glycemic stabilizing compound is further able to cross the blood-brain barrier.

In one embodiment, said glycemic stabilizing compound comprises a compound selected from the group consisting of: 2-methyl-4-(4-nitro-phenyl)-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide,4-Furan-2-yl-2-methyl-5-oxo-1,4,5,6,7,8-hexahydro-quinoline-3-carboxylicacid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

In addition, the invention relates to a method for preparing a composition which comprises a glycemic stabilizing compound as listed above and comprising admixing said compound with a carrier. In addition, the invention provides a method for preparing a composition which comprises a glycemic stabilizing compound and comprising admixing said compound with a carrier, wherein said compound comprises a compound selected from the group consisting of: 2-methyl-4-(4-nitro-phenyl)-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

Also described is a pharmaceutical composition comprising, consisting essentially of, or consisting of the glycemic stabilizing compound identified according to the method of: a) contacting a candidate compound with GPR41, and b) determining whether GPR41 functionality is increased, wherein an increase in GPR41 functionality is indicative of the candidate compound being a glycemic stabilizing compound.

Some embodiments of the present invention include a method of producing a Pharmaceutical composition comprising admixing at least one compound according to any of the compound embodiments disclosed herein and a pharmaceutically acceptable carrier.

A compound can be formulated into pharmaceutical compositions using techniques well known to those in the art and described herein.

While it is possible that, for use in the prophylaxis or treatment, a compound disclosed herein or identified by methods of the invention can in an alternative use be administered as a raw or pure chemical, it can be useful to present

the compound or active ingredient as a pharmaceutical formulation or composition further comprising a pharmaceutically acceptable carrier.

The invention thus further provides pharmaceutical formulations comprising a compound disclosed herein or a pharmaceutically acceptable salt or derivative thereof together with one or more phairnaceutically acceptable carriers thereof and/or prophylactic ingredients. The carrier(s) are "acceptable" in the sense of being compatible with the other ingredients of the formulation and not overly deleterious to the recipient thereof.

Pharmaceutical formulations, routes of administration, and dosages have been described above.

Described herein is a method for treating or preventing an insulin-related disorder in an individual in need thereof, comprising administering to said individual an effective amount of the compound identified according to the method of: a) contacting a candidate compound with GPR41, and b) determining whether GPR41 functionality is increased, wherein an increase in GPR41 functionality is indicative of the candidate compound being a glycemic stabilizing compound. In some instances, said insulin-related disorder is hypoglycemia, an insulin-secreting or insulin-dependent tumor, or aging. The compound administered may comprise a GPR41 agonist. Preferably the individual is a mammal and more preferably the individual is a human.

While the compounds disclosed herein or identified by the methods of the invention can be administered as the sole active pharmaceutical agent as described herein above, they can also be used in combination with one or more agents.

Also described herein is a method for the manufacture of a medicament comprising a pharmaceutical composition comprising, consisting essentially of, or consisting of the glycemic stabilizing compound identified according to the method of: a) contacting a candidate compound with GPR41, and b) determining whether GPR41 functionality is increased, wherein an increase in GPR41 functionality is indicative of the candidate compound being a glycemic stabilizing compound, for use as a glycemic stabilizing compound.

Further described is a method for the manufacture of a medicament comprising a pharmaceutical composition comprising, consisting essentially of, or consisting of the glycemic stabilizing compound identified according to the method of: a) contacting a candidate compound with GPR41, and b) determining whether GPR41 functionality is increased, wherein an increase in GPR41 functionality is indicative of the candidate compound being a glycemic stabilizing compound, for use in the treatment of an insulin-related disorder.

The invention relates to a method for identifying a glycemic stabilizing compound, comprising: a) contacting a candidate compound with GPR41, and b) determining whether GPR41 functionality is decreased, wherein a decrease in GPR41 functionality is indicative of the candidate compound being a glycemic stabilizing compound. In one embodiment, said glycemic stabilizing compound comprises a compound selected from the group consisting of: 2-Methyl-4-[5-(2-nitro-4-trifluoromethyl-phenyl)-furan-2-yl]-5-oxo-1,4, 5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

In one embodiment, said GPR41 is derived from a mammal. In another embodiment, said GPR41 is human.

In certain embodiments, said GPR41 is recombinant. In certain embodiments, said contacting comprises contacting with a host cell or with membrane of a host cell that expresses the GPCR, wherein the host cell comprises an expression vector comprising a polynucleotide encoding the receptor. In some embodiments, said contacting is carried out in the presence of a known agonist of the GPCR or an agonist as disclosed herein.

In some embodiments, said determining comprises a second messenger assay, for example, determining is through the measurement of GTP$\gamma$S binding to membrane comprising said GPCR. In certain embodiments, said GTP$\gamma$S is labeled with [$^{35}$S]. In certain embodiments, said determining is through the measurement of the level of a second messenger selected from the group consisting of cyclic AMP (cAMP), cyclic GMP (cGMP), inositol triphosphate (IP3), diacylglycerol (DAG), MAP kinase activity, and $Ca^{2+}$. In certain embodiments, said second messenger is cAMP. In certain embodiments, said measurement of cAMP is carried out using whole-cell adenylyl cyclase assay. In certain embodiments, said measurement of cAMP is carried out with membrane comprising said GPCR. In certain embodiments, said determining is through measurement of intracellular IP3. In certain embodiments, said determining further includes the use of a chimeric G-protein such as a Gq/Gi chimera. In certain embodiments, said second messenger is MAP kinase activity. In some embodiments, said determining is through CRE-reporter assay. In certain embodiments, said reporter is luciferase. In some embodiments, said reporter is $\beta$-galactosidase. In certain embodiments, said determining or said comparing is through measurement of intracellular $Ca^{2+}$.

In some embodiments, said determining is through measurement of glucose uptake by adipocytes obtained from a mammal. In some embodiments, said determining is through measurement of glucose uptake by skeletal muscle cells obtained from a mammal.

In certain embodiments, said determining is through the use of a melanophore assay.

The invention also relates to a method of identifying a candidate compound as a potentiator of insulin secretion, comprising a) contacting a candidate compound with GPR41, and b) determining whether GPR41 functionality is decreased, wherein a decrease in GPR41 functionality is indicative of the candidate compound being a potentiator of insulin secretion. For example, a compound that decreases GPR41 functionality, such as a GPR41 antagonist or inverse agonist, can result in an increase in insulin secretion. An increase in insulin secretion can be desired, for example, in individuals with insulin resistance such as diabetics.

The invention also relates to a method of identifying a candidate compound that results in a decrease in blood glucose concentration, comprising a) contacting a candidate compound with GPR41, and b) determining whether GPR41 functionality is decreased, wherein a decrease in GPR41 functionality is indicative of the candidate compound resulting in a decrease in blood glucose concentration. For example, a compound that decreases GPR41 functionality, such as a GPR41 inverse agonist or antagonist, can result in an increase in insulin secretion and a decrease in blood glucose concentration. A decrease in blood glucose can be desired, for example, in individuals with hyperglycemia such as diabetics.

In certain embodiments, said GPR41 is recombinant. In certain embodiments, said contacting comprises contacting with a host cell or with membrane of a host cell that expresses the GPCR, wherein the host cell comprises an expression vector comprising a polynucleotide encoding the receptor. In some embodiments, said contacting is carried out in the presence of an agonist of the GPCR.

In the methods of the invention, control reactions can be performed to show specificity of the response. For example, mock-transfected cells can be compared to GPR41 transfected cells to show specificity of a response to the GPR41 receptor.

In the methods of the invention, in certain embodiments, said candidate compound is not an antibody or antigen-binding derivative thereof. In certain embodiments, said candidate compound is not a peptide. In certain embodiments, said candidate compound is not a polypeptide.

In the methods of the invention, determining can comprise a second messenger assay. The initiation of an intracellular signal can be determined, for example, through the measurement of the level of a second messenger such as cyclic AMP (cAMP), cyclic GMP (cGMP), inositol triphosphate (IP3), diacylglycerol (DAG), MAP kinase, or calcium. Several assays are well known in the art for measuring these second messengers, for example, cAMP assays, IP3 assays, the FLIPR assay, the melanophore assay, or CRE-reporter assay. In addition, examples of second messenger assays are disclosed herein in Examples 12-17. In certain embodiments, said second messenger is cAMP. In other embodiments, said second messenger is IP3. In further embodiments said second messenger is calcium.

In one embodiment, said determining is through the measurement of GTPγS binding to membrane comprising said GPCR. Such assays are well known in the art and exemplified herein in Examples 12 and 14. In certain embodiments, said GTPγS is labeled with [$^{35}$S].

In one embodiment, said glycemic stabilizing compound is a GPR41 inverse agonist. In one embodiment, said glycemic stabilizing compound is a GPR41 antagonist. In one embodiment, said glycemic stabilizing compound comprises a compound selected from the group consisting of: 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

In some embodiments, said glycemic stabilizing compound is a GPR41 inverse agonist or antagonist with an IC50 of less than 10 μM, of less than 1 μM, of less than 100 nM, or of less than 10 nM. In some embodiments, said glycemic stabilizing compound is an inverse agonist or antagonist with an IC50 of a value selected from the interval of 1 nM to 10 μM. In some embodiments, said glycemic stabilizing compound is an inverse agonist or antagonist with an IC50 of a value selected from the interval of 1 nM to 1 μM. In some embodiments, said glycemic stabilizing compound is an inverse agonist or antagonist with an IC50 of a value selected from the interval of 1 nM to 100 nM. In some embodiments, said glycemic stabilizing compound is an inverse agonist or antagonist with an IC50 of a value selected from the interval of 1 nM to 10 nM.

In certain embodiments, said IC50 is determined using an assay selected from the group consisting of: IP3 assay carried out using transfected HEK293 cells expressing recombinant GPR41 polypeptide; and melanophore assay carried out using transfected melanophores expressing recombinant GPR 41 polypeptide. In some embodiments, said glycemic stabilizing compound is an inverse agonist or antagonist with an IC50 of less than 10 μM, of less than 1 μM, of less than 100 nM, or of less than 10 nM in said assay. In some embodiments, said glycemic stabilizing compound is an inverse agonist or antagonist with an IC50 of less than 10 μM, of less than 9 μM, of less than 8 μM, of less than 7 μM, of less than 6 μM, of less than 5 μM, of less than 4 μM, of less than 3 μM, of less than 2 μM, of less than 1 μM, of

less than 900 nM, of less than 800 nM, of less than 700 nM, of less than 600 nM, of less than 500 nM, of less than 400 nM, of less than 300 nM, of less than 200 nM, of less than 100 nM, of less than 90 nM, of less than 80 nM, of less than 70 nM, of less than 60 nM, of less than 50 nM, of less than 40 nM, of less than 30 nM, of less than 20 nM, of less than 10 nM in said assay. In some embodiments, said glycemic stabilizing compound is an inverse agonist or antagonist with an IC50 in said assay of a value selected from the interval of 1 nM to 10 μM. In some embodiments, said glycemic stabilizing compound is an inverse agonist or antagonist with an IC50 in said assay of a value selected from the interval of 1 nM to 1 μM. In some embodiments, said glycemic stabilizing compound is an inverse agonist or antagonist with an IC50 in said assay of a value selected from the interval of 1 nM to 100 nM. In some embodiments, said glycemic stabilizing compound is an inverse agonist or antagonist with an IC50 in said assay of a value selected from the interval of 1 nM to 10 nM. In some embodiments, said glycemic stabilizing compound is selective for the GPCR.

In some embodiments, said glycemic stabilizing compound is orally bioavailable. In some embodiments, said oral bioavailability is at least 1%, at least 5%, at least 10%, at least 15%, at least 20%, at least 25%, at least 30%, at least 35%, at least 40%, or at least 45% relative to intraperitoneal administration. In some embodiments, said oral bioavailablity is at least 20%, at least 25%, at least 30%, at least 35%, at least 40%, or at least 45% relative to intraperitoneal administration. In some embodiments, said orally bioavailable glycemic stabilizing compound is further able to cross the blood-brain barrier.

Further described herein is a method for preparing a composition which comprises identifying a glycemic stabilizing compound and then admixing said compound with a carrier, wherein said compound is identifiable by the method of: a)contacting a candidate compound with GPR41, and b) determining whether GPR41 functionality is decreased, wherein a decrease in GPR41 functionality is indicative of the candidate compound being a glycemic stabilizing compound. For example, the invention provides a method for preparing a composition which comprises identifying a glycemic stabilizing compound and then admixing said compound with a carrier, wherein said compound is identified by the method of: a) contacting a candidate compound with GPR41, and b) determining whether GPR41 functionality is decreased, wherein a decrease in GPR41 functionality is indicative of the candidate compound being a glycemic stabilizing compound.

Also described is a pharmaceutical composition comprising, consisting essentially of, or consisting of the glycemic stabilizing compound identified according to the method of: a) contacting a candidate compound with GPR41, and b) determining whether GPR41 functionality is decreased, wherein a decrease in GPR41 functionality is indicative of the candidate compound being a glycemic stabilizing compound.

Some embodiments of the present invention include a method of producing a pharmaceutical composition comprising admixing at least one compound according to any of the compound embodiments disclosed herein and a pharmaceutically acceptable carrier.

Pharmaceutical formulations, routes of administration, and dosages have been described above.

Described herein is a method for treating or preventing an insulin-related disorder in an individual in need thereof, comprising administering to said individual an effective amount of the compound identified according to the method of: a) contacting a candidate compound with GPR41, and b) determining whether GPR41 functionality is decreased, wherein a decrease in GPR41 functionality is indicative of the candidate compound being a glycemic stabilizing compound. In some instances, said insulin-related disorder is insulin resistance, impaired glucose tolerance, or diabetes. In some instances, said insulin-related disorder includes a condition related to an elevated blood glucose concentration, such as atherosclerosis, heart disease, stroke, hypertension, obesity, Syndrome X or peripheral vascular disease. In some embodiments, said insulin-related disorder is Type II diabetes. The compound administered may comprise a GPR41 inverse agonist or antagonist.

The method may further comprise administering to said individual an effective amount of an agent used for the treatment of diabetes, blood lipid disorders, or obesity in combination with an effective amount of the pharmaceutical composition comprising, consisting essentially of, or consisting of the glycemic stabilizing compound identified according to the method of: a) contacting a candidate compound with GPR41, and b) determining whether GPR41 functionality is decreased, wherein a decrease in GPR41 functionality is indicative of the candidate compound being a glycemic stabilizing compound. For example, the method further comprises administering to said individual an effective amount of an agent used for the treatment of diabetes, blood lipid disorders, or obesity in combination with an effective amount of a pharmaceutical composition containing a GPR41 inverse agonist.

Preferably, the individual is a mammal and more preferably the individual is a human.

While the compounds disclosed herein or identified by the methods of the invention can be administered as the sole active pharmaceutical agent as described herein above, they can also be used in combination with one or more agents including, for example, agents that are used for the treatment of diabetes, blood lipid disorders, or obesity. For example, a compound such as a GPR41 inverse agonist or antagonist can be used in combination with one or more agents belonging to the class of drugs known as α-glucosidase inhibitors, aldose reductase inhibitors, biguanides, thiazolidinediones, meglitinides, sulfonylureas, insulin, HMG-CoA reductase inhibitors, squalene synthesis inhibitors, fibrate compounds, LDL catabolism enhancers, angiotensin converting enzyme (ACE) inhibitors, lipase inhibitors, serotonin and/or noradrenaline releasers or reuptake inhibitors.

Some embodiments of the invention include, a pharmaceutical composition comprising a compound disclosed herein or a pharmaceutically acceptable salt thereof in combination with at least one member selected from the group consisting of an α-glucosidase inhibitor, an aldose reductase inhibitor, a biguanide, a HMG-CoA reductase inhibitor, a squalene synthesis inhibitor, a fibrate compound, a LDL catabolism enhancer and an angiotensin converting enzyme inhibitor. In another embodiment, the HMG-CoA reductase inhibitor is selected from the group consisting of prevastatin, simvastatin, lovastatin, atorvastatin, fluvastatin and lipitor.

In accordance with the present invention, the combination can be used by mixing the respective active components either all together or independently with a physiologically acceptable carrier, excipient, binder, diluent, etc., as described herein above, and administering the mixture or mixtures either orally or non-orally as a pharmaceutical composition. When a compound or a mixture of compounds are administered as a combination therapy or prophylaxis with another active compound the therapeutic agents can be formulated as a separate pharmaceutical compositions given at the same time or at different times, or the therapeutic agents can be given as a single composition.

Also described herein is a method for the manufacture of a medicament comprising a pharmaceutical composition comprising, consisting essentially of, or consisting of the glycemic stabilizing compound identified according to the method of: a) contacting a candidate compound with GPR41, and b) determining whether GPR41 functionality is decreased, wherein a decrease in GPR41 functionality is indicative of the candidate compound being a glycemic stabilizing compound, for use as a glycemic stabilizing compound.

Further described is a method for the manufacture of a medicament comprising a pharmaceutical composition comprising, consisting essentially of, or consisting of the glycemic stabilizing compound identified according to the method of: a) contacting a candidate compound with GPR41, and b) determining whether GPR41 functionality is decreased, wherein a decrease in GPR41 functionality is indicative of the candidate compound being a glycemic stabilizing compound, for use in the treatment of an insulin-related disorder such as insulin resistance, impaired glucose tolerance or diabetes.

Also described is a method for increasing GPR41 function, comprising contacting GPR41 with an effective amount of a GPR41 agonist, for example, a compound selected from the group selected from the group consisting of: 2-methyl-4-(4-nitro-phenyl)-5-oxo-1,4,5,6,7,8-hexahydro-quinoline-3-carboxylic acid o-tolylamide, cyclopropanecarboxylic acid 4-[1,2,3]thiadiazol-4-yl-phenyl ester, cyclopropanecarboxylic acid; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chlorophenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof. The structures of these compounds are shown in Table 1 below. Also contemplated is a method for increasing GPR41 function in a cell, comprising contacting a cell expressing GPR41 with an effective amount of a GPR41 agonist. The cell can be, for example, in an individual or the cell can be an isolated cell.

Table 1:

| Cmpd No. | Chemical Structure | Chemical Name |
|---|---|---|
| 1 | 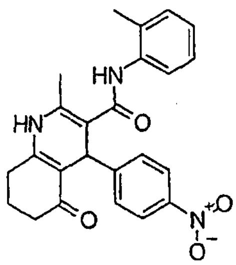 | 2-Methyl-4-(4-nitro-phenyl)-5-oxo-1,4,5,6,7,8-hexahydro-quinoline-3-carboxylic acid o-tolylamide |
| 2 | 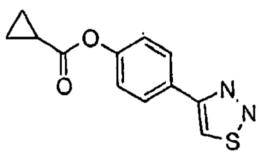 | Cyclopropanecarboxylic acid 4-[1,2,3]thiadiazol-4-yl-phenyl ester |
| 3 | 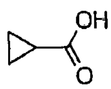 | Cyclopropanecarboxylic acid |

(continued)

| Cmpd No. | Chemical Structure | Chemical Name |
|---|---|---|
| 4 | 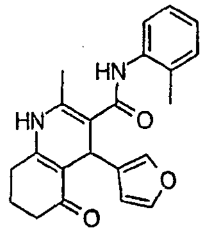 | 4-Furan-3-yl-2-methyl-5-oxo-1,4,5,6,7,8-hexahydro-quinoline-3-carboxylic acid o-tolylamide |
| 5 | 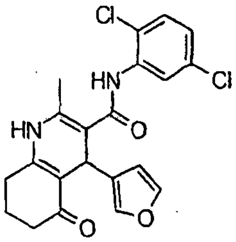 | 4-Furan-3-yl-2-methyl-5-oxo-1,4,5,6,7,8-hexahydro-quinoline-3-carboxylic acid (2,5-dichloro-phenyl)-amide |
| 6 | 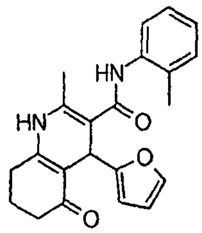 | 4-Furan-2-yl-2-methyl-5-oxo-1,4,5,6,7,8-hexahydro-quinoline-3-carboxylic acid o-tolylamide |
| 7 | 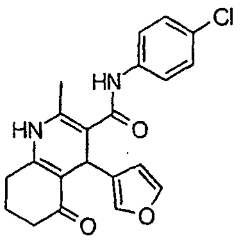 | 4-Furan-3-yl-2-methyl-5-oxo-1,4,5,6,7,8-hexahydro-quinoline-3-carboxylic acid (4-chloro-phenyl)-amide |
| 8 | 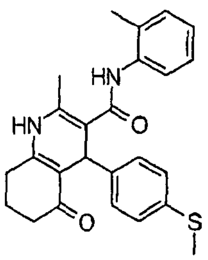 | 2-Methyl-4-(4-methylsulfanyl-phenyl)-5-oxo-1,4,5,6,7,8-hexahydro-quinoline-3-carboxylic acid o-tolylamide |
| 9 | 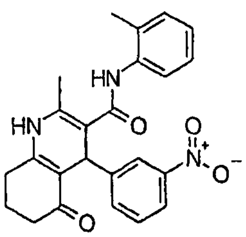 | 2-Methyl-4-(3-nitro-phenyl)-5-oxo-1,4,5,6,7,8-hexahydro-quinoline-3-carboxylic acid o-tolylamide |

Further described herein is a method for decreasing GPR41 function, comprising contacting GPR41 with an effective amount of a GPR41 inverse agonist or antagonist, for example, a compound selected from the group selected from the group consisting of 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof. The structures of these compounds are shown in Table 2 below. Also contemplated is a method for decreasing GPR41 function in a cell, comprising contacting a cell expressing GPR41 with an effective amount of a GPR41 inverse agonist or antagonist. The cell can be, for example, in an individual or the cell can be an isolated cell.

Table 2

| Cmpd No. | Chemical Structure | Chemical Name |
|---|---|---|
| 10 | 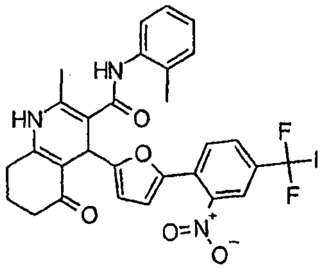 | 2-Methyl-4-[5-(2-nitro-4-trifluoromethylphenyl)-furan-2-yl]-5-oxo-1,4,5,6,7,8-hexahydro-quinoline-3-carboxylic acid o-tolylamide |
| 11 | 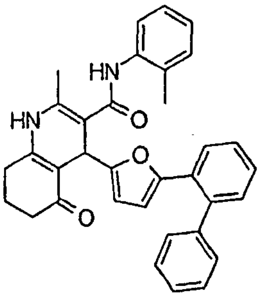 | 4-(5-Biphenyl-2-yl-furan-2-yl)-2-methyl-5-oxo-1,4,5,6,7,8-hexahydro-quinoline-3-carboxylic acid o-tolylamide |
| 12 | 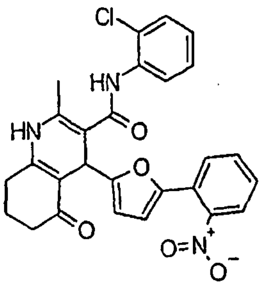 | 2-Methyl-4-[5-(2-nitro-phenyl)-furan-2-yl]-5-oxo-1,4,5,6,7,8-hexahydro-quinoline-3-carboxylic acid (2-chloro-phenyl)-amide |
| 13 | 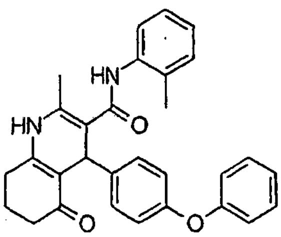 | 2-Methyl-5-oxo-4-(4-phenoxy-phenyl)-1,4,5,6,7,8-hexahydro-quinoline-3-carboxylic acid o-tolylamide |

(continued)

| Cmpd No. | Chemical Structure | Chemical Name |
|---|---|---|
| 14 | 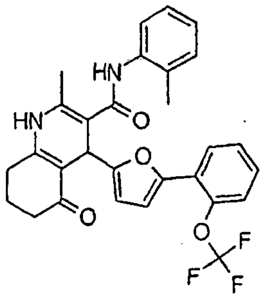 | 2-Methyl-5-oxo-4-[5-(2-trifluoromethoxyphenyl)-furan-2-yl]-1,4,5,6,7,8-hexahydro-quinoline-3-carboxylic acid o-tolylamide |
| 15 | 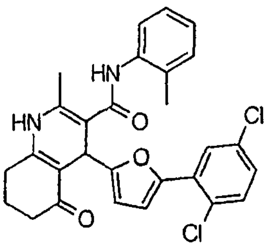 | 4-[5-(2,5-Dichloro-phenyl)-furan-2-yl]-2-methyl-5-oxo-1,4,5,6,7,8-hexahydro-quinoline-3-carboxylic acid o-tolylamide |

Described herein is a method for treating or preventing an insulin-related disorder, comprising administering to an individual in need thereof an effective amount of a GPR41 modulator. In one instance, said insulin-related disorder is hypoglycemia, an insulin-secreting or insulin-dependent tumor, or aging. Preferably, said modulator is an agonist. More preferably said agonist comprises a compound selected from the group selected from the group consisting of: 2-methyl-4-(4-nitro-phenyl)-5-oxo-1,4,5,6,7,8-hexahydro-quinoline-3-carboxylic acid o-tolylamide, cyclopropanecarboxylic acid 4-[1,2,3]thiadiazol-4-yl-phenyl ester, cyclopropanecarboxylic acid; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

The insulin-related disorder may be insulin resistance, impaired glucose tolerance, or diabetes and said modulator is an inverse agonist or antagonist. In one embodiment, said insulin-related disorder is Type II diabetes. In some cases, said insulin-related disorder includes a condition related to an elevated blood glucose concentration, such as atherosclerosis, heart disease, stroke, hypertension, obesity, Syndrome X or peripheral vascular disease. Preferably, said inverse agonist or antagonist comprises a compound selected from the group consisting of 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

The method may further comprise administering to said individual an effective amount of an agent used for the treatment of diabetes, blood lipid disorders, or obesity in combination with an effective amount of a GPR41 inverse agonist or antagonist. Preferably, the individual is a mammal and more preferably the individual is a human.

Further described is a method for treating or preventing a disorder treatable or preventable by increasing GPR41 function, comprising administering to an individual in need thereof an effective amount of a compound selected from the group selected from the group consisting of 2-methyl-4-(4-nitro-phenyl)-5-oxo-1,4,5,6,7,8-hexahydro-quinoline-3-carboxylic acid o-tolylamide, cyclopropanecarboxylic acid 4-[1,2,3]thiadiazol-4-yl-phenyl ester, cyclopropanecarboxylic acid; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chloro-phenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof. In one embodiment, said disorder is an insulin-related disorder. In some embodiments, said insulin-related disorder is hypoglycemia, an insulin-secreting or insulin-dependent tumor, or aging.

The invention also provides a compound for treating or preventing a disorder treatable or preventable by decreasing GPR41 function, comprising administering to an individual in need thereof an effective amount of 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof. In one embodiment, said disorder is an insulin-related disorder. In some embodiments, said insulin-related disorder is insulin resistance, impaired glucose tolerance, or diabetes. In one embodiment, said insulin-related disorder is type II diabetes. In some embodiments, said insulin-related disorder includes a condition related to an elevated blood glucose concentration, such as atherosclerosis, heart disease, stroke, hypertension, obesity, Syndrome X or peripheral vascular disease. In one embodiment, said method further comprises administering to said individual an effective amount of an agent used for the treatment of diabetes, blood lipid disorders, or obesity in combination with an effective amount of a GPR41 inverse agonist or antagonist. In one embodiment, the individual is a mammal and in another embodiment the individual is a human.

The invention also provides a compound for increasing blood glucose levels in an individual in need thereof, comprising administering to the individual an effective amount of a GPR41 agonist, where said agonist comprises a compound selected from the group selected from the group consisting of: 2-methyl-4-(4-nitrophenyl)-5-oxo-1,4,5,6,7,8-hexahydro-quinoline-3-carboxylic acid o-tolylamide, cyclopropanecarboxylic acid 4-[1,2,3]thiadiazol-4-yl-phenyl ester, cyclopropanecarboxylic acid; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chlorophenyl)-amide, 2-Methyl-4-(4-methylsulfanyl-phenyl)-5-oxo-1,4,5,6,7,8-hexahydro-quinoline-3-carboxylic acid tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

The invention also provides a compound for decreasing blood glucose levels in an individual in need thereof, comprising administering to the individual an effective amount of a GPR41 inverse agonist or antagonist, where said inverse agonist or antagonist comprises a compound selected from the group consisting of 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof..

In addition, the invention provides a compound for decreasing insulin secretion in an individual in need thereof, comprising administering to the individual an effective amount of a GPR41 agonist, where said agonist comprises a compound selected from the group selected from the group consisting of: 2-methyl-4-(4-nitro-phenyl)-5-oxo-1,4,5,6,7,8-hexahydro-quinoline-3-carboxylic acid o-tolylamide; 4-Furan-3-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (2,5-dichloro-phenyl)-amide, 4-Furan-2-yl-2-methyl-5-oxo-1,4,5,6,7,8-hexahydro-quinoline-3-carboxylic acid o-tolylamide, 4-Furan-3-yl-2-methyl-5-oxo-1,4,5,6,7,8-hexahydro-quinoline-3-carboxylic acid (4-chlorophenyl)-amide, 2-Methyl-4-(4-methylsulfanyl-phenyl)-5-oxo-1,4,5,6,7,8-hexahydro-quinoline-3-carboxylic acid o-tolylamide, and 2-Methyl-4-(3-nitro-phenyl)-5-oxo-1,4,5,6,7,8-hexahydro-quinoline-3-carboxylic acid o-tolylamide, or a pharmaceutically acceptable salt thereof.

The invention further provides a compound for increasing insulin secretion in an individual in need thereof, comprising administering to the individual an effective amount of a GPR41 inverse agonist or antagonist, where the GPR41 inverse agonist or antagonist can comprise a compound selected from the group consisting of 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and

4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

The invention further provides a compound for increasing insulin secretion in a glucose dependent manner in an individual in need thereof, comprising administering to the individual an effective amount of a GPR41 inverse agonist or antagonist, where the GPR41 inverse agonist or antagonist can comprise a compound selected from the group consisting of: 2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide, 4-(5-Biphenyl-2-yl-furan-2-yl)-2-methyl-5-oxo-1,4,5,6,7,8-hexahydro-quinoline-3-carboxylic acid o-tolylamide, 2-Methyl-4-[5-(2-nitro-phenyl)-furan-2-yl]-5-oxo-1,4,5,6,7,8-hexahydro-quinoline-3-carboxylic acid (2-chloro-phenyl)-amide, 2-Methyl-5-oxo-4-(4-phenoxy-phenyl)-1,4,5,6,7,8-hexahydro-quinoline-3-carboxylic acid o-tolylamide, 2-Methyl-5-oxo-4-[5-(2-trifluoromethoxy-phenyl)-furan-2-yl]-1,4,5,6,7,8-hexahydro-quinoline-3-carboxylic acid o-tolylamide, and 4-[5-(2,5-Dichloro-phenyl)-furan-2-yl]-2-methyl-5-oxo-1,4,5,6,7,8-hexahydro-quinoline-3-carboxylic acid o-tolylamide; or a pharmaceutically acceptable salt thereof.

The term "in a glucose dependent manner" means that insulin secretion is increased in response to a high concentration of glucose, but not in response to a low concentration of glucose. Some drugs that have been used for the treatment of diabetes increase insulin secretion regardless of the level of glucose in the blood. This is not desirable because these drugs increase insulin secretion even under conditions of hypoglycemia. The increased insulin then further exacerbates the hypoglycemia, sometimes to critical levels. A high concentration of glucose means that the concentration of glucose in the blood or around cells is higher than a normal glucose concentration range, for example, 16.8 mmol/L is a high concentration of glucose. A low concentration of glucose means that the concentration of glucose in the blood or around cells is lower than a normal glucose concentration range, for example, 3.3 mmol/L or less.

The cellular mechanism of action of insulin secretion is the increase in intracellular cAMP. As disclosed herein, GPR41 is expressed in pancreatic beta islet cells. GPR41 is coupled to Gi so an inverse agonist or antagonist of GPR41 will result in an increase in cAMP in pancreatic beta islet cells and an increase in insulin secretion.

One object of the invention relates to a method of (a) performing a method of the invention to identify a glycemic stabilizing compound and (b) optionally, determining the structure of the compound, and (c) providing the compound or the name or structure of the compound.

Another object of the present invention relates to radio-labeled compounds of Table 1 or Table 2 that would be useful not only in radio-imaging but also in assays, both in vitro and in vivo, for localizing and quantitating GPR41 in tissue samples, including human, and for identifying GPR41 ligands by inhibition binding of a radiolabelled compound. It is a further object of this invention to develop novel GPR41 assays of which comprise such radiolabelled compounds.

Suitable radionuclides that can be incorporated in compounds of the present invention include but are not limited to $^{3}H$ (also written as T), $^{11}C$, $^{14}C$, $^{18}F$, $^{125}I$, $^{82}Br$, $^{123}I$, $^{124}I$, $^{125}I$, $^{131}I$, $^{75}Br$, $^{76}Br$, $^{15}O$, $^{13}N$, $^{31}S$ and $^{77}Br$. The radionuclide that is incorporated in the instant radiolabelled compounds will depend on the specific application of that radiolabelled compound. Thus, for in vitro GPR41 labeling and competition assays, compounds that incorporate $^{3}H$, $^{14}C$, $^{125}I$, $^{131}I$, $^{35}S$ or $^{82}Br$ will generally be most useful. For radio-imaging applications $^{11}C$, $^{18}F$, $^{125}I$, $^{123}I$, $^{124}I$, $^{131}I$, $^{75}Br$, $^{76}Br$ or $^{77}Br$ will generally be most useful.

It is understood that a "radio-labelled " or "labelled compound" is a compound of Table 1 or Table 2 that has incorporated at least one radionuclide; in some embodiments the radionuclide is selected from the group consisting of $^{3}H$, $^{14}C$, $^{125}I$, $^{35}S$ and $^{82}Br$; in some embodiments the radionuclide $^{3}H$ or $^{14}C$. Moreover, it should be understood that all of the atoms represented in the compounds of the invention can be either the most commonly occurring isotope of such atoms or the more scarce radio-isotope or nonradioactive isotope.

Synthetic methods for incorporating radio-isotopes into organic compounds including those applicable to those compounds of the invention are well known in the art and include incorporating activity levels of tritium into target molecules include: **A.** Catalytic Reduction with Tritium Gas - This procedure normally yields high specific activity products and requires halogenated or unsaturated precursors. **B.** Reduction with Sodium Borohydride [$^{3}H$] - This procedure is rather inexpensive and requires precursors containing reducible functional groups such as aldehydes, ketones, lactones, esters, and the like. **C.** Reduction with Lithium Aluminum Hydride [$^{3}H$ ] - This procedure offers products at almost theoretical specific activities. It also requires precursors containing reducible functional groups such as aldehydes, ketones, lactones, esters, and the like. **D.** Tritium Gas Exposure Labeling - This procedure involves exposing precursors containing exchangeable protons to tritium gas in the presence of a suitable catalyst. **E.** N-Methylation using Methyl Iodide [$^{3}H$] - This procedure is usually employed to prepare O-methyl or N-methyl ($^{3}H$) products by treating appropriate precursors with high specific activity methyl iodide ($^{3}H$). This method in general allows for high specific activity, such as about 80-87 Ci/mmol.

Synthetic methods for incorporating activity levels of $^{125}I$ into target molecules include: **A.** Sandmeyer and like reactions - This procedure transforms an aryl or heteroaryl amine into a diazonium salt, such as a tetrafluoroborate salt, and subsequently to $^{125}I$ labelled compound using $Na^{125}I$. A represented procedure was reported by Zhu, D.-G. and co-workers in J. Org. Chem. 67:943-948 (2002)). **B.** Ortho $^{125}$Iodination of phenols - This procedure allows for the incorporation of $^{125}I$ at the ortho position of a phenol as reported by Collier, T. L. and co-workers in J. Labelled Compd

Radiopharm., 42: S264-S266 (1999)). C. Aryl and heteroaryl bromide exchange with $^{125}I$ - This method is generally a two step process. The first step is the conversion of the aryl or heteroaryl bromide to the corresponding tri-alkyltin intermediate using for example, a Pd catalyzed reaction [i.e. $Pd(Ph_3P)_4$] or through an aryl or heteroaryl lithium, in the presence of a tri-alkyltinhalide or hexaalkylditin [e.g., $(CH_3)_3SnSn(CH_3)_3$]. A represented procedure was reported by Bas, M.-D. and co-workers in J. Labelled Compd Radiopharm., 44:S280-S282 (2001)).

A radiolabelled GPR41 compound of Table 1 or Table 2 can be used in a screening assay to identify/evaluate compounds. In general terms, a newly synthesized or identified compound (i.e., candidate compound) can be evaluated for its ability to reduce binding of the "radio-labelled compound of Table 1 or Table 2" to the GPR41 receptor. Accordingly, the ability of a candidate compound to compete with the "radio-labelled compound of Table 1 or Table 2" for the binding to the GPR41 receptor directly correlates to its binding affinity.

One aspect of the present invention pertains to a glycemic stabilizing compound, mentioned above, for use in a method of treatment of the human or animal body by therapy.

Another aspect of the present invention pertains to a glycemic stabilizing compound mentioned above, for use in a method of treatment of an insulin related disorder, of the human or animal body by therapy.

Another aspect of the present invention pertains to a glycemic stabilizing compound, as mentioned above, for use in a method of treatment of an insulin related disorder of the human or animal body by therapy.

Applicants reserve the right to exclude any one or more candidate compounds from any of the embodiments of the invention. Applicants also reserve the right to exclude any one or more modulators from any of the embodiments of the invention. Applicants additionally reserve the right to exclude any insulin-related disorder, or any condition related to an elevated blood glucose concentration from any of the embodiments of the invention.

Other uses of the disclosed receptors and methods will become apparent to those in the art based upon, *inter alia,* a review of this patent document.

The following examples are given to illustrate the invention and are not intended to be inclusive in any manner:

## EXAMPLES

The examples are provided to further define the invention without, however, limiting the invention to the specifics of these examples.

### Example 1

<u>**Dot blot analysis of human GPR41 expression in human adult and fetal tissues**</u>

In this example, the expression level of human GPR41 was determined in several human adult and fetal tissues using a dot blot.

A dot blot containing human adult and fetal tissue mRNAs was purchased from Clontech (BD Bioscience, Palo Alto, CA). The order of the tissue mRNAs on the blot is: A1=total brain, A2=amygdala, A3=caudate nucleus, A4=cerebellum, A5=cerebral cortex, A6=frontal cortex, A7=hippocampus, A8=medulla oblongata, B1=occipital cortex, B2=putamen, B3=substantia nigra, B4=temporal cortex, B5=thalamus, B6=accumbens, B7=spinal cord, C1= heart, C2=aorta, C3=skeletal muscle, C4=colon, C5=bladder, C6=uterus, C7=prostate, C8=stomach, D1=testis, D2=ovary, D3=pancreas, D4=pituitary gland, D5=adrenal gland, D6=thyroid, D7=salivary gland, D8=mammary gland, El=kidney, E2=liver, E3=small intestine, E4=spleen, E5=thymus, E6=peripheral leukocyte, E7=lymph node, E8=bone marrow, F1=appendix, F2=lung, F3=trachea, F4=placenta, Gl=fetal brain, G2=fetal heart; G3=fetal kidney, G4=fetal liver, G5=fetal spleen, G6=fetal thymus, G7=fetal lung. The blot was hybridized with the GPR41 probe using Clontech "Express Hyb" under conditions recommended by Clontech. See Figure 1.

### Example 2

<u>**RT-PCR and Taqman Analysis of GPR41 Expresion in Mouse Tissues and Cells**</u>

In this example, the expression level of mouse GPR41 was determined in several mouse cell types and tissues using an RT-PCR assay and Taqman quantitiative PCR assay. As shown in Figure 2, the highest level expression of mouse GPR41 was observed in pancreas and pancreatic islet cells. In addition, as shown in Figure 2, GPR41 was up-regulated in pancreatic islets from db/db mice compared to wild-type and ob/ob mice.

GPR41 expression in mouse tissues was evaluated by RT-PCR using the following primers: 5'- ATG GGG ACA AGC TTC TTT CT-3' (SEQ ID NO:3) and 5'- CTA GCT CGG ACA CTC CTT GG-3' (SEQ ID NO:4). Mouse tissue cDNAs were synthesized from commercial poly A RNA purchased from Clontech using the BioRad iScript cDNA synthesis kit. cDNAs from mouse cell lines including insulin-producing pancreatic beta cell lines (NIT-1, βTC-6, AND MIN-6) were

prepared from total RNA isolated using Triazol (Invitrogen).

For the Taqman quantitative PCR experiment shown in the bottom panel of Figure 2, a 1X TaqMan supermix was made in a 5mL polypropylene tube. Forward and reverse primers were added to give a 300nM final concentration; appropriate amount of probe was added to give a final concentration of 200nM. Total volume per well was 20μL. 2μL was cDNA, while the other 18μL was supermix and nuclease-free water.

The primers were ordered from Proligo and the probe was synthesized by ABI. The sequence for primers and probe were:

5'Primer: GCCGGCGCAAGAGGATA (SEQ ID NO:5)
3'Primer: CCGAAGCAGACGAAGAAGATG 3' (SEQ ID NO:6)
Probe: TTCTTGCAGCCACACTG-MGBNFQ 3' (SEQ ID NO:7)

The thermo cycler conditions used is shown in the chart below (Table 3).

| Times and Temperatures | | | |
|---|---|---|---|
| Initial Steps | | Each of 40 Cycles | |
| | | Melt | Anneal/Extend |
| HOLD | HOLD | CYCLE | |
| 2 min* 50 °C | 10min** 95 °C | 15 sec 95 °C | 1 min 60 °C |
| *The 2 min hdd at 50 °C is required for optimal AmpErase UNG activity **The 10 min hdd at 95 °C is required for AmplTaq Gold DNA Polymerase activation | | | |

**Example 3**

**Mouse GPR41 RNase Protection Assay**

In this example, the expression level of mouse GPR41 was determined in several mouse cell types and tissues using an RNase protection assay. As shown in Figure 3, the highest level expression of mouse GPR41 was observed in pancreatic islets and pancreatic islet cell lines including MIN6, a mouse insulinoma cell line, NIT-1, and βTC-6.

Mouse tissue RNAs were commercially obtained (Clontech). Cells (as indicated in the figures) used for RNA isolation are pancreatic cell lines provided by ATCC (NIT-1: ATCC CRL-2055, and βTC-6:ATCC CRL-11506) or obtained from Jeffrey Pessin at SUNY at Stony Brook (MIN-6). RNAs were isolated using Trizol reagent (Invitrogen) according to manufacturer's instructions.

Briefly, a 257 bp fragment of mouse GPR41 was cloned into pCR II TOPO cloning vector (Invitrogen). The plasmid was linearized with Xho1 and gel purified using the Sephaglass Bandprep Kit (Amersham). After gel purification of the fragment, a riboprobe was made by *in vitro* transcription with using SP6 RNA polymerase (Ambion Maxiscript Kit). The probe was purified by acrylamide gel electrophoresis and hybridized with 20μg of total RNA at 47°C overnight. The hybrids were digested with Rnase the following day and run on a 5% acrylamide gel to detect the results (Ambion, RPA III kit). All the procedures for *in vitro* transcription and RPA reactions were following the manufacturer's instructions.

Mouse GPR41 sequence for RPA probe:

5'-
GTGGGGCTGAGGGTTACACACAGAGGTGGCACCTTGGTGATGTCGA
CACTGGGTGAGGGACAGGAAACCAGGGAGGTAGGCAGGACCACCTGCAGGG
GAGAGCATGTGGAGCTATGGTGGTGGGGTGTAGGCAGTGTAGACAGCAATC
TTGCCTGATGGGTAAGAGTCTCCCAGTGAGGGAACCCCAACTCTCAACACAT
TCCTCTCTGTCTCATTAGCATCTGTGACCATGGGGACAAGCTTCTTTCTTGGC
AATT-3' (SEQ ID NO:8)

Mouse GPR41 PCR primers for RPA probe

5'- GTGGGGCTGAGGGTTACA-3' (SEQ ID NO:9)
5'- AATTGCCAAGAAAGAAGC-3' (SEQ ID NO:10)

**Example 4**

**G-alpha i coupling of GPR41**

In this example, the coupling of GPR41 to G-alpha i (Gαi) was determined using a GqGi chimera. The function of GPR41 was measured using an IP3 assay as described below. See also Figure 4.

IP3 Assay of GPR41 Expressed in Gq/Gi transfected HEK 293 cells

Intracellular IP3 accumulation assays were performed using HEK293 cells transiently transfected with expression plasmids for both GPR41 and the Gq/Gi chimera (see Example 13 for structure of the Gq/Gi chimera). DNAs used in the transfection for this assay were GPR41 and GPR41(k) cloned into mammalian expression vector pCMV and Gq/Gi chimeras cDNA cloned into an expression vector pcDNA3.1(+) (Invitrogen). GPR41(k) is a single amino acid mutation of GPR41 where amino acid 224 is mutated to a lysine.

Transfections were performed using Lipofectamine transfection reagent (Invitrogen) and following manufacturer's recommendations. Briefly, on day 1 cells were plated on 96-well plates at a density of $3x10^6$ cells/plate. On the following day a DNA/Lipofectamine mixture was prepared for each plate as follows: 1μL of DNA (40ng of pCMV-GPR41) per 3 wells, or 2μL of DNA (20ng of pCMV-GPR41 combined with pcDNA-Gq/Gi) in 25μL of OPTI-MEM (Gibco) was gently mixed with 2μL of Lipofectamine reagent in 25μL of OPTI-MEM and the resulting solution was incubated for 30 minutes at room temperature. 96μL of OPTI-MEM was added to give a final volume of 150μL. Cells were then washed once with 100μL/well of PBS, and DNA-Liptofectamine mixture was then gently added to the plate (50μL/well). The cells were then incubated for 4 hours at 37°C in a humidified atmosphere containing 5%$CO_2$. Regular cell media was added to the transfection reagent. Cells were then incubated at 37°C/5%$CO_2$ overnight.

On day 3, regular growth media was carefully removed from wells and replaced with 100μL of inositol-free/serum-free DMEM (Gibco) containing 0.4uCi of [$^3$H]-myo-inositol (Perkin-Elmer). Cells were incubated overnight at 37°C with 5%$CO_2$. On day 4, [$^3$H]-myo-inositol-containing labeling media was removed and replaced with 100μL of inositol-free/serum-free DMEM containing 10μM parglyline (Sigma) and10mM lithium chloride (Sigma). Cells were incubated for 1 hour at 37°C /5%$CO_2$. Solution was then carefully removed and 160 μL per well of ice cold 0.1M formic acid was added to the cells. The cells were lysed by incubating plates at -80°C for at least 1 hour.

Separation of IP3 from cell lysates was performed using chromatography on AG1-X8 formate resin (Bio-Rad). 400μL of formate resin slurry (0.1g of resin in 1mL of water) was added per each well of Multiscreen filter plate (Millipore). Water was drained from the wells and resin was then washed with 200μL of water using Millipore filtration unit. Plates with lysed cells were thawed and lysates were transferred into Multiscreen filter plate, containing formate resin. Plates were incubated for 10 minutes at room temperature and lysates were then drained from filter plates with filtration unit. Plates were washed four times with water 9200μL/well) and thoroughly drained. Elution buffer was then added to the resin (180μL/well) and plates were incubated for 5 minutes at room temperature. Eluents were drained into 96 well collection plates using vacuum manifold, transferred into scintillation vials containing 5 ml of Optiphase HiSafe3 scintillation cocktail (Perkin-Elmer) and counted on Wallac Scintillation Counter.

**Example 5**

**G-alpha 12/13 coupling of GPR41**

In this example, the coupling of GPR41 to G-alpha 12/13 (Gα12/13) was determined using a cAMP assay as described below. See-also Figure 5.

Briefly, the experiment was performed as follows: 293 cells were transfected with the indicated CMV-driven expression plasmid indicated on the X-axis, along with one of the following chimeras: (a) "control": parental CMV expression plasmid, (b) "Gs/G12 chimera": CMV-Gs/G12 plasmid encoding human Gs in which the C-terminal 11 amino acids were switched to those corresponding to the C-terminus of G12, or (c) "Gs/G13 chimera": CMV-Gs/G13 plasmid encoding human Gs in which the C-terminal 11 amino acids were switched to those corresponding to the C-terminus of G13. 24-hours later, the cells were analyzed for cAMP levels using a "Flash Plate" kit as described below.

A Flash Plate™ Adenylyl Cyclase kit (New England Nuclear; Cat. No. SMP004A) designed for cell-based assays was modified for use with crude plasma membranes. The Flash Plate wells contained a scintillant coating which also contains a specific antibody recognizing cAMP. The cAMP generated in the wells was quantitated by a direct competition for binding of radioactive cAMP tracer to the cAMP antibody. The following serves as a brief protocol for the measurement of changes in cAMP levels in whole cells that express GPR41.

Transfected cells were harvested approximately twenty four hours after transient transfection. Media was carefully aspirated off and discarded. 5ml of cell dissociation buffer was added to each plate. Cells were pipetted off the plate and the cell suspension was collected into a 50ml conical centrifuge tube. Cells were then centrifuged at room temperature at 1,100 rpm for 5 minutes. The cell pellet was carefully re-suspended into an appropriate volume of Assay Buffer which consisted of ½ vol of PBS and ½ vol of stimulation buffer (about 3ml/plate). The cells were then counted using a hemocytometer and additional assay buffer was added to give the appropriate number of cells (with a final volume of about 50μl/well).

cAMP standards and Detection Buffer (comprising 1 μCi of tracer [$^{125}$I] cAMP (50μl) to 11ml Detection Buffer) was prepared and maintained in accordance with the manufacturer's instructions. The assay was initiated by addition of 50μl of cAMP standards to appropriate wells followed by addition of 50μl of PBS to wells H11 and H12 of a 96 well plate. 50ml of Stimulation Buffer was added to all standard wells. The cells were added to the appropriated wells. Forskolin was diluted with Assay Buffer into 2x stock, then added to the cells at 50μl /well and incubated for 60 minutes at room temperature. 100μl of Detection Mix containing tracer cAMP was then added to the wells. Plates were then incubated an additional 2 hours followed by counting in a Wallac MicroBeta scintillation counter. Values of cAMP/well were then extrapolated from a standard cAMP curve which was contained within each assay plate.

**Example 6**

**Identification of GPR41 Modulators**

In this example, GPR41 agonists were identified using a screening protocol in *Xenopus* melanophores.

Melanophore Technology

Melanophores are skin cells found in lower vertebrates. They contain pigmented organelles termed melanosomes. Melanophores are able to redistribute these melanosomes along a microtubule network upon G-protein coupled receptor (GPCR) activation. The result of this pigment movement is an apparent lightening or darkening of the cells. In melanophores, the decreased levels of intracellular cAMP that result from activation of a Gi-coupled receptor cause melanosomes to migrate to the center of the cell, resulting in a dramatic lightening in color. If cAMP levels are then raised, following activation of a Gs-coupled receptor, the melanosomes are re-dispersed and the cells appear dark again. The increased levels of diacylglycerol that result from activation of Gq-coupled receptors can also induce this re-dispersion. In addition, the technology is also suited to the study of certain receptor tyrosine kinases. The response of the melanophores takes place within minutes of receptor activation and results in a simple, robust color change. The response can be easily detected using a conventional absorbance microplate reader or a modest video imaging system. Unlike other skin cells, the melanophores derive from the neural crest and appear to express a full complement of signaling proteins. In particular, the cells express an extremely wide range of G-proteins and so are able to functionally express almost all GPCRs.

Melanophores can be utilized to identify compounds, including natural ligands, which bind to and/or activate GPCRs. This method can be conducted by introducing test cells of a pigment cell line capable of dispersing or aggregating their pigment in response to a specific stimulus and expressing an exogenous clone coding for the GPCR. An initial state of pigment disposition can be set using, for example, using melatonin, MSH or light. The test cells are then contacted

with chemical compounds, and it is determined whether the pigment disposition in the cells changed from the initial state of pigment disposition. Dispersion of pigments cells due to the candidate compound, including but not limited to a ligand, coupling to the GPCR will appear dark on a petri dish, while aggregation of pigments cells will appear light.

Materials and methods were followed according to the disclosure of U.S. Patent Number 5,462,856 and U.S. Patent Number 6,051,386. These patent disclosures are hereby incorporated by reference in their entirety.

Melanophores were transfected by electroporation with a plasmid which contained the coding sequence of human GPR41. The cells were plated in 96-well plates. 48 hours post-transfection, half of the cells on each plate were treated with 10nM melatonin. Melatonin activates an endogenous Gi-coupled receptor in the melanophores and causes them to aggregate their pigment. The remaining half of the cells were transferred to serum-free medium 0.7X L-15 (Gibco). After one hour, the cells in serum-free media remained in a pigment-dispersed state while the melatonin-treated cells were in a pigment-aggregated state. At this point, the cells were treated with different compounds from a proprietary compound library containing 140,000-150,000 organic small molecule compounds. If GPR41 bound to the compound, the melanophores would be expected to undergo a color change in response to the compound. Since the receptor can couple to Gi, the pigment-dispersed cells underwent a dose-dependent pigment aggregation.

**Example 7**

**<u>Efficacy of GPR41 Agonists in Gq/Gi Cotransfected Cells</u>**

In this example, the efficacy of selected GPR41 agonists 2-methyl-4-(4-nitrophenyl)-5-oxo-1,4,5,6,7,8-hexahydro-quinoline-3-carboxylic acid o-tolylamide (CPD1 in Figure 6) and cyclopropanecarboxylic acid 4-[1,2,3]thiadiazol-4-yl-phenyl ester (CPD2 in Figure 5) were tested in HEK 293 cells co-transfected with chimeric Galpha Gq/Gi.

Transfections were performed using Lipofectamine transfection reagent (Invitrogen) and following manufacturer's recommendations. Briefly, on day 1 cells were plated on 96-well plates at a density of $4x10^6$ cells/plate. On the following day a DNA/Lipofectamine mixture was prepared for each plate as follows: 2.5μL of DNA (250ng of pCMV-GPR41 or pCMV blank vector) per 8 wells and 2.5μL of DNA (250ng of pcDNA 3.1-Gq/Gi) in 200μL of DME (Gibco) was gently mixed with 2.5μL of Lipofectamine reagent in 200μL of DME and the resulting solution was incubated for 30 minutes at room temperature. Growth media was aspirated off from the cells and 55μl/well of DME were added. DNA-Liptofectamine mixture was then gently added to the plate (45μL/well). The cells were then incubated for 4 hours at 37°C in a humidified atmosphere containing 5%$CO_2$. Regular cell growth media was added to the transfection reagent. Cells were then incubated at 37°C /5%$CO_2$ overnight.

On day 3, regular growth media was carefully removed from wells and replaced with 100μL of inositol-free/serum-free DMEM (Gibco) containing 0.4μCi of [$^3$H]-myo-inositol (Perkin-Elmer). Cells were incubated overnight at 37°C with 5%$CO_2$. On day 4, [$^3$H]-myo-inositol-containing labeling media was removed and replaced with 100μL of inositol-free/serum-free DMEM containing 10μM parglyline (Sigma) and10mM lithium chloride (Sigma) with/out compound. Cells were incubated for 3 hours at 37°C /5%$CO_2$. Solution was then carefully removed and 160 μL per well of ice cold 0.1M formic acid was added to the cells. The cells were lysed by incubating plates at -80°C for at least 1 hour.

Separation of IP3 from cell lysates was performed using chromatography on AG1-X8 formate resin (Bio-Rad). 400μL of formate resin slurry (0.1g of resin in 1mL of water) was added per each well of Multiscreen filter plate (Millipore). Water was drained from the wells and resin was then washed with 200μL of water using Millipore filtration unit. Plates with lysed cells were thawed and lysates were transferred into Multiscreen filter plate, containing formate resin. Plates were incubated for 10 minutes at room temperature and lysates were then drained from filter plates with filtration unit. Plates were washed four times with water (200μL/well) and thoroughly drained. Elution buffer was then added to the resin (180μL/well) and plates were incubated for 5 minutes at room temperature. Eluents were drained into 96 well collection plates using vacuum manifold, 60μl transferred into Whatman Unifilter GF/C 96 well plate (Perkin Elemer 1450-525) and 50μl of Optima Gold (Perkin Elmer) was added. Counting was performed on a Wallac Scintillation Counter.

**Example 8**

A **<u>GPR41 Agonist Inhibits Insulin Secretion in MIN6 Insulinoma Cells</u>**

In this example, a selected GPR41 agonist, cyclopropanecarboxylic acid (CPC) was assayed for its effect on insulin secretion in an insulinoma cell line. See Figure 7.

Mouse insulinoma line, MIN6, was plated into multi-well tissue culture dishes and cultured 2 days in Dulbecco's Minimum Essential Media (DMEM) supplemented with 15% fetal bovine serum. The cells were rinsed and fasted in low glucose (10mg/dl) Krebs-Ringers Bicarbonate buffer for several hours before a 30-minute challenge with 300mg/dl glucose and the compounds of interest: glucagon-like peptide 1 (GLP-1) and cyclopropanecarboxylic acid (CPC). GLP-1, 7-36 amide, a known inhibitor of insulin secretion, was purchased from Sigma and used at 25 nM concentration and

CPC was purchased from Aldrich and was used at 5 μM or 1 μM. Control wells with glucose only (both 10mg/dl and 300mg/dl) were run for comparison. Supernatants from the glucose challenge were harvested and frozen. These were evaluated by ELISA (Crystal Chem, Inc.) for insulin.

**Example 9**

**Oral Glucose Tolerance Test**

A GPR41 modulator such as an agonist, antagonist or inverse agonist can be tested for its effect on plasma glucose after oral glucose administration is tested.

For example, male C57bl/6 mice at age 67 days can be fasted for 18 hours and randomly grouped to receive a GPR41 modulator at selected doses, or vehicle (PET which contains 80% PEG, 10% Tween80, and 10% Ethanol): The GPR41 modulator is delivered orally via a gavage needle (p.o. volume at 100 μl). Thirty minutes after administration of the GPR41 modulator or vehicle, mice are administered orally with dextrose at 3 g/kg dose. Levels of blood glucose are determined at several time points using Glucometer Elite XL (Bayer).

Glucose tolerance can also be tested using i.p. delivery of glucose. For example, 68 day old male C57Bl/6 mice are treated with a GPR41 modulator at 100 mg/kg or with PET vehicle after 18 hours of fasting. Thirty minutes after administration of the GPR41 modulator or vehicle, mice are administered i.p. with dextrose at 2 g/kg dose. Levels of blood glucose are determined at the selected time points using Glucometer Elite XL (Bayer).

**Example 10**

**GPR41 agonist reverses the beneficial effect of an oral glucose tolerance test (oGTT) lowering compound**

A compound having a glycemic lowering effect in the oGTT, (2-Fluoro-4-methanesulfonyl-phenyl)-{6-[4-(3-isopropyl-[1,2,4]oxadiazol-5-yl)-piperidin-1-yl]-5-nitro-pyrimidin-4-yl}-amine, which is referenced as Compound B111 in WO 2004/065380 A1 filed January 14, 2004, was used alone or in conjunction with the GPR41 agonist Compound 4, disclosed herein. As shown in Figure 8, the GPR41 agonist reversed the glycemic lowering effect of Compound B111, causing an increase in plasma glucose. These results indicate that a GPR41 antagonist or inverse agonist can have a glycemic lowering effect.

For the oGTT, male C57bl/6 mice were fasted for about 18 hours and randomly grouped to receive the compounds indicated in Figure 8 at the indicated doses, or vehicle (PET which contains 80% PEG, 10% Tween80, and 10% Ethanol). The indicated compound(s) were delivered orally via a gavage needle (p.o. volume at 100 μl). Thirty minutes after administration of the compound(s) or vehicle, mice were administered orally with dextrose at 3 g/kg dose. Levels of blood glucose were determined at several time points using Glucometer Elite XL (Bayer).

**Example 11**

**Compound Synthesis**

Compounds disclosed herein were either commercially available or prepared by procedures known in the art. For example, Compound **1** [i.e., 2-methyl-4-(4-nitro-phenyl)-5-oxo-1,4,5,6,7,8-hexahydro-quinoline-3-carboxylic acid o-tolylamide] was purchased from ASINEX Ltd. 5 Gabrichevskogo St. Bldg 8, Moscow 123367, Russia; Compound **2** (cyclopropanecarboxylic acid 4-[1,2,3]thiadiazol-4-yl-phenyl ester) was purchased from Tripos, Inc., 1699 South Hanley Road, St. Louis, MO 63144-2319; and Compound **3** (cyclopropanecarboxylic acid) was purchased from Sigma-Aldrich, 3050 Spruce St., St. Louis, MO 63103. Compounds **4** to **9** were prepared by methods known in the art, for example, the 3-component Hantzsch Dihydropyridine Synthesis of dihydropyridines as essentially described by Carroll, et al. Journal of Medicinal Chemistry 47: 3180-3192 (2004) (e.g., heating the components to reflux in isopropanol for 18-36 hours). The general reaction scheme for the preparation of certain compounds of the invention is shown below:

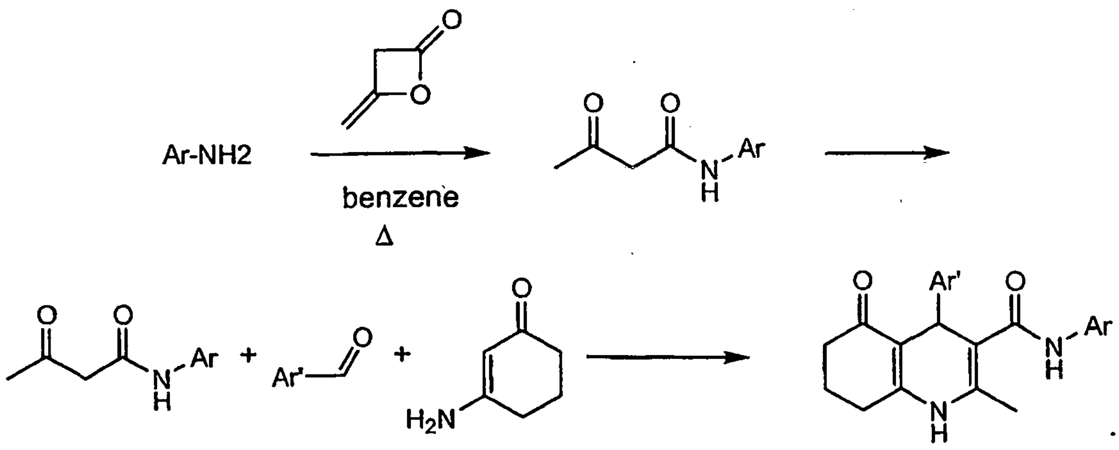


Certain compounds disclosed herein also required a further coupling step, for example, a Suzuki coupling step. The Suzuki coupling reaction is well know in the art and a variety of conditions and variations have been reported, for example, the procedure by Snieckus, et al. Journal of Organic Chemistry 56: 3763-3768 (1991). The general reaction is shown below:

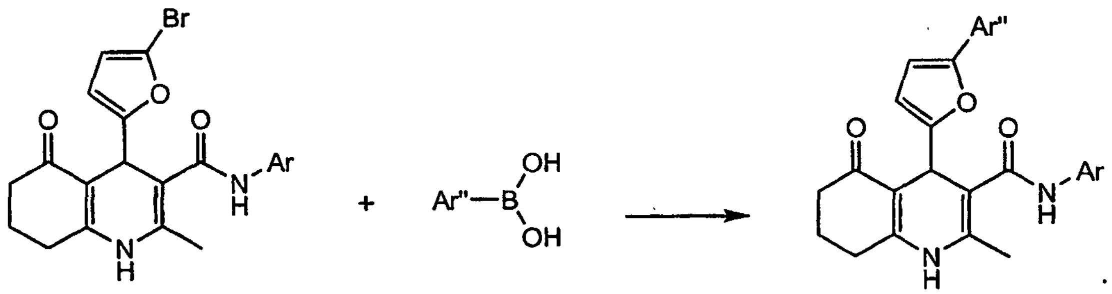

**Example 12**

**Assays for Determination of GPCR Activation**

A variety of approaches are available for assessment of activation of human GPCRs. The following are illustrative; those of ordinary skill in the art are credited with the ability to determine those techniques that are preferentially beneficial for the needs of the artisan.

1. Membrane Binding Assays: [$^{35}$S]GTPγS Assay

When a G protein-coupled receptor is in its active state, either as a result of ligand binding or constitutive activation, the receptor couples to a G protein and stimulates the release of GDP and subsequent binding of GTP to the G protein. The alpha subunit of the G protein-receptor complex acts as a GTPase and slowly hydrolyzes the GTP to GDP, at which point the receptor normally is deactivated. Activated receptors continue to exchange GDP for GTP. The non-hydrolyzable GTP analog, [$^{35}$S]GTPγS, can be utilized to demonstrate enhanced binding of [$^{35}$S]GTPγS to membranes expressing activated receptors. The advantage of using [$^{35}$S]GTPγS binding to measure activation is that: (a) it is generically applicable to all G protein-coupled receptors; (b) it is proximal at the membrane surface making it less likely to pick-up molecules which affect the intracellular cascade.

The assay utilizes the ability of G protein coupled receptors to stimulate [$^{35}$S]GTPγS binding to membranes expressing the relevant receptors. The assay can, therefore, be used in the direct identification method to screen candidate compounds to endogenous GPCRs and non-endogenous, constitutively activated GPCRs. The assay is generic and has application to drug discovery at all G protein-coupled receptors.

The [$^{35}$S]GTPγS assay is incubated in 20 mM HEPES and between 1 and about 20mM $MgCl_2$ (this amount can be adjusted for optimization of results, although 20mM is preferred) pH 7.4, binding buffer with between about 0.3 and about 1.2 nM [$^{35}$S]GTPγS (this amount can be adjusted for optimization of results, although 1.2 is preferred) and 12.5 to 75 μg membrane protein (e.g, 293 cells expressing the GPR41; this amount can be adjusted for optimization)

and 10 μM GDP (this amount can be changed for optimization) for 1 hour. Wheatgerm agglutinin beads (25 μl; Amersham) are then added and the mixture incubated for another 30 minutes at room temperature. The tubes are then centrifuged at 1500 x g for 5 minutes at room temperature and then counted in a scintillation counter.

2. Adenylyl Cyclase

A Flash Plate™ Adenylyl Cyclase kit (New England Nuclear; Cat. No. SMP004A) designed for cell-based assays can be modified for use with crude plasma membranes. The Flash Plate wells can contain a scintillant coating which also contains a specific antibody recognizing cAMP. The cAMP generated in the wells can be quantitated by a direct competition for binding of radioactive cAMP tracer to the cAMP antibody. The following serves as a brief protocol for the measurement of changes in cAMP levels in whole cells that express a receptor.

Transfected cells are harvested approximately twenty four hours after transient transfection. Media is carefully aspirated off and discarded. 10ml of PBS is gently added to each dish of cells followed by careful aspiration. 1ml of Sigma cell dissociation buffer and 3ml of PBS are added to each plate. Cells are pipetted off the plate and the cell suspension is collected into a 50ml conical centrifuge tube. Cells are then centrifuged at room temperature at 1,100 rpm for 5 minutes. The cell pellet is carefully re-suspended into an appropriate volume of PBS (about 3ml/plate). The cells are then counted using a hemocytometer and additional PBS is added to give the appropriate number of cells (with a final volume of about 50μl/well).

cAMP standards and Detection Buffer (comprising 1μCi of tracer [$^{125}$I] cAMP (50μl) to 11ml Detection Buffer) is prepared and maintained in accordance with the manufacturer's instructions. Assay Buffer is prepared fresh for screening and contains 50μl of Stimulation Buffer, 3μl of candidate compound (12μM final assay concentration) and 50μl cells. Assay Buffer is stored on ice until utilized. The assay, preferably carried out, for example, in a 96-well plate, is initiated by addition of 50μl of cAMP standards to appropriate wells followed by addition of 50μl of PBSA to wells H11 and H12. 50μl of Stimulation Buffer is added to all wells. DMSO (or selected candidate compounds) is added to appropriate wells using a pin tool capable of dispensing 3μl of compound solution, with a final assay concentration of 12μM candidate compound and 100μl total assay volume. The cells are then added to the wells and incubated for 60 minutes at room temperature. 100μl of Detection Mix containing tracer cAMP is then added to the wells. Plates are then incubated additional 2 hours followed by counting in a Wallac MicroBeta scintillation counter. Values of cAMP/well are then extrapolated from a standard cAMP curve which is contained within each assay plate.

3. Cell-Based cAMP for Gi Coupled Target GPCRs

TSHR is a Gs coupled GPCR that causes the accumulation of cAMP upon activation. TSHR can be constitutively activated by mutating amino acid residue 623 (i.e., changing an alanine residue to an isoleucine residue). A Gi coupled receptor is expected to inhibit adenylyl cyclase, and, therefore, decrease the level of cAMP production, which can make assessment of cAMP levels challenging. An effective technique for measuring the decrease in production of cAMP as an indication of activation of a Gi coupled receptor can be accomplished by co-transfecting, non-endogenous, constitutively activated TSHR (TSHR-A623I) (or an endogenous, constitutively active Gs coupled receptor) as a "signal enhancer" with a Gi linked target GPCR to establish a baseline level of cAMP. Upon creating an endogenous or non-endogenous version of the Gi coupled receptor, the target GPCR is then co-transfected with the signal enhancer, and it is this material that can be used for screening. In some embodiments, this approach is preferably used in the direct identification of candidate compounds against Gi coupled receptors. It is noted that for a Gi coupled GPCR, when this approach is used, an inverse agonist of the target GPCR will increase the cAMP signal and an agonist will decrease the cAMP signal.

On day one, $2x10^4$ 293 cells/well are plated out. On day two, two reaction tubes are prepared (the proportions to follow for each tube are per plate): tube A is prepared by mixing 2μg DNA of each receptor transfected into the mammalian cells, for a total of 4μg DNA (e.g., pCMV vector; pCMV vector with mutated THSR (TSHR-A623I); TSHR-A623I and GPCR, etc.) in 1.2ml serum free DMEM (Irvine Scientific, Irvine, CA); tube B is prepared by mixing 120μl lipofectamine (Gibco BRL) in 1.2ml serum free DMEM. Tubes A and B are then admixed by inversions (several times), followed by incubation at room temperature for 30-45minutes. The admixture is referred to as the "transfection mixture". Plated 293 cells are washed with 1XPBS, followed by addition of 10ml serum free DMEM. 2.4ml of the transfection mixture is then added to the cells, followed by incubation for 4 hours at 37°C/5% $CO_2$. The transfection mixture is then removed by aspiration, followed by the addition of 25ml of DMEM/10% Fetal Bovine Serum. Cells are then incubated at 37°C/5% $CO_2$. After 24 hours incubation, cells are harvested and utilized for analysis.

A Flash Plate™ Adenylyl Cyclase kit (New England Nuclear; Cat. No. SMP004A) is designed for cell-based assays, but can be modified for use with crude plasma membranes depending on the need of the skilled artisan. The Flash Plate wells contain a scintillant coating which also contains a specific antibody recognizing cAMP. The cAMP generated in the wells can be quantitated by a direct competition for binding of radioactive cAMP tracer to the cAMP antibody. The following serves as a brief protocol for the measurement of changes in cAMP levels in whole cells that

express a receptor of interest.

Transfected cells are harvested approximately twenty four hours after transient transfection. Media is carefully aspirated off and discarded. 10ml of PBS is gently added to each dish of cells followed by careful aspiration. 1ml of Sigma cell dissociation buffer and 3ml of PBS is added to each plate. Cells are pipetted off the plate and the cell suspension is collected into a 50ml conical centrifuge tube. Cells are then centrifuged at room temperature at 1,100 rpm for 5 minutes. The cell pellet is carefully re-suspended into an appropriate volume of PBS (about 3ml/plate). The cells are then counted using a hemocytometer and additional PBS is added to give the appropriate number of cells (with a final volume of about 50μl/well).

cAMP standards and Detection Buffer (comprising 1μCi of tracer [$^{125}$I] cAMP (50μl) to 11ml Detection Buffer) is prepared and maintained in accordance with the manufacturer's instructions. Assay Buffer should be prepared fresh for screening and contain 50μl of Stimulation Buffer, 3μl of candidate compound (12μM final assay concentration) and 50μl cells. Assay Buffer can be stored on ice until utilized. The assay can be initiated by addition of 50μl of cAMP standards to appropriate wells followed by addition of 50μl of PBSA to wells H-11 and H12. Fifty μl of Stimulation Buffer is added to all wells. Selected compounds (e.g., TSH) are added to appropriate wells using a pin tool capable of dispensing 3μl of compound solution, with a final assay concentration of 12μM candidate compound and 100μl total assay volume. The cells are then added to the wells and incubated for 60 minutes at room temperature. 100μl of Detection Mix containing tracer cAMP is then added to the wells. Plates are then incubated additional 2 hours followed by counting in a Wallac MicroBeta scintillation counter. Values of cAMP/well are extrapolated from a standard cAMP curve which is contained within each assay plate.

4. Reporter-Based Assays

a. CRE-LUC Reporter Assay (Gs-associated receptors)

293 or 293T cells are plated-out on 96 well plates at a density of 2 x $10^4$ cells per well and are transfected using Lipofectamine Reagent (BRL) the following day according to manufacturer instructions. A DNA/lipid mixture is prepared for each 6-well transfection as follows: 260ng of plasmid DNA in 100μl of DMEM is gently mixed with 2μl of lipid in 100μl of DMEM (the 260ng of plasmid DNA consists of 200ng of a 8xCRE-Luc reporter plasmid, 50ng of pCMV comprising endogenous receptor or non-endogenous receptor or pCMV alone, and 10ng of a GPRS expression plasmid (GPRS in pcDNA3 (Invitrogen)). The 8XCRE-Luc reporter plasmid is prepared as follows: vector SRIF-β-gal is obtained by cloning the rat somatostatin promoter (-71/+51) at BglV-HindIII site in the pβgal-Basic Vector (Clontech). Eight (8) copies of cAMP response element are obtained by PCR from an adenovirus template AdpCF126CCRE8 (see, Suzuki et al., Hum Gene Ther 7:1883-1893 (1996); the disclosure of which is hereby incorporated by reference in its entirety) and cloned into the SRIF-β-gal vector at the Kpn-BglV site, resulting in the 8xCRE-β-gal reporter vector. The 8xCRE-Luc reporter plasmid is generated by replacing the beta-galactosidase gene in the 8xCRE-β-gal reporter vector with the luciferase gene obtained from the pGL3-basic vector (Promega) at the HindIII-BamHI site. Following 30 minutes incubation at room temperature, the DNA/lipid mixture is diluted with 400 μl of DMEM and 100μl of the diluted mixture is added to each well. 100 μl of DMEM with 10% FCS are added to each well after a four hour incubation in a cell culture incubator. The following day the transfected cells are changed with 200 μl/well of DMEM with 10% FCS. Eight (8) hours later, the wells are changed to 100 μl /well of DMEM without phenol red, after one wash with PBS. Luciferase activity is measured the next day using the LucLite™ reporter gene assay kit (Packard) following manufacturer instructions and read on a 1450 MicroBeta™ scintillation and luminescence counter (Wallac).

b. AP1 reporter assay (Gq-associated receptors)

A method to detect Gq stimulation depends on the known property of Gq-dependent phospholipase C to cause the activation of genes containing AP1 elements in their promoter. A Pathdetect™ AP-1 cis-Reporting System (Stratagene, Catalogue No. 219073) can be utilized following the protocol set forth above with respect to the CREB reporter assay, except that the components of the calcium phosphate precipitate are 410 ng pAP1-Luc, 80 ng pCMV-receptor expression plasmid, and 20 ng CMV-SEAP.

c. SRF-LUC Reporter Assay (Gq- associated receptors)

One method to detect Gq stimulation depends on the known property of Gq-dependent phospholipase C to cause the activation of genes containing serum response factors in their promoter. A Pathdetect™ SRF-Luc-Reporting System (Stratagene) can be utilized to assay for Gq coupled activity in, for example, COS7 cells. Cells are transfected with the plasmid components of the system and the indicated expression plasmid encoding endogenous or non-endogenous GPCR using a Mammalian Transfection™ Kit (Stratagene, Catalogue #200285) according to the manufacturer's

instructions. Briefly, 410 ng SRF-Luc, 80 ng pCMV-receptor expression plasmid and 20 ng CMV-SEAP (secreted alkaline phosphatase expression plasmid; alkaline phosphatase activity is measured in the media of transfected cells to control for variations in transfection efficiency between samples) are combined in a calcium phosphate precipitate as per the manufacturer's instructions. Half of the precipitate is equally distributed over 3 wells in a 96-well plate and kept on the cells in a serum free media for 24 hours. The last 5 hours the cells are incubated with, for example, 1μM, candidate compound. Cells are then lysed and assayed for luciferase activity using a Luclite™ Kit (Packard, Cat. No. 6016911) and "Trilux 1450 Microbeta" liquid scintillation and luminescence counter (Wallac) as per the manufacturer's instructions. The data can be analyzed using GraphPad Prism™ 2.0a (GraphPad Software Inc.).

d. Intracellular IP3 Accumulation Assay (Gq-associated receptors)

On day 1, cells comprising the receptor of interest (endogenous or non-endogenous) can be plated onto 24 well plates, usually 1x$10^5$ cells/well (although his number can be optimized). On day 2 cells can be transfected by first mixing 0.25μg DNA in 50 μl serum free DMEM/well and 2 μl lipofectamine in 50 μl serum free DMEM/well. The solutions are gently mixed and incubated for 15-30 minutes at room temperature. Cells are washed with 0.5 ml PBS and 400 μl of serum free media is mixed with the transfection media and added to the cells. The cells are then incubated for 3-4 hours at 37°C/5%$CO_2$ and then the transfection media is removed and replaced with 1ml/well of regular growth media. On day 3 the cells are labeled with $^3$H-myo-inositol. Briefly, the media is removed and the cells are washed with 0.5 ml PBS. Then 0.5 ml inositol-free/serum free media (GIBCO BRL) is added/well with 0.25 μCi of $^3$H-myo-inositol/ well and the cells are incubated for 16-18 hours overnight at 37°C/5%$CO_2$. On Day 4 the cells are washed with 0.5 ml PBS and 0.45 ml of assay medium is added containing inositol-free/serum free media, 10 μM pargyline, 10 mM lithium chloride or 0.4 ml of assay medium and 50μl of 10x ketanserin (ket) to final concentration of 10μM, if using a control construct containing a serotonin receptor. The cells are then incubated for 30 minutes at 37°C. The cells are then washed with 0.5 ml PBS and 200μl of fresh/ice cold stop solution (1M KOH; 18 mM Na-borate; 3.8 mM EDTA) is added/well. The solution is kept on ice for 5-10 minutes or until cells were lysed and then neutralized by 200 μl of fresh/ice cold neutralization sol. (7.5 % HCL). The lysate is then transferred into 1.5 ml eppendorf tubes and 1 ml of chloroform/methanol (1:2) is added/tube. The solution is vortexed for 15 seconds and the upper phase is applied to a Biorad AG1-X8™ anion exchange resin (100-200 mesh). Firstly, the resin is washed with water at 1:1.25 W/V and 0.9 ml of upper phase is loaded onto the column. The column is washed with 10 mls of 5 mM myo-inositol and 10 ml of 5 mM Naborate/60mM Na-formate. The inositol tris phosphates are eluted into scintillation vials containing 10 ml of scintillation cocktail with 2 ml of 0.1 M formic acid/ 1 M ammonium formate. The columns are regenerated by washing with 10 ml of 0.1 M formic acid/3M ammonium formate and rinsed twice with dd $H_2O$ and stored at 4°C in water.

**Example 13**

**Fusion Protein Preparation**

a. GPCR:Gs Fusion Constuct

The design of the GPCR-G protein fusion construct can be accomplished as follows: both the 5' and 3' ends of the rat G protein Gsα (long form; Itoh, H. et al., Proc. Natl. Acad. Sci. 83:3776 (1986)) are engineered to include a HindIII sequence thereon. Following confirmation of the correct sequence (including the flanking HindIII sequences), the entire sequence is shuttled into pcDNA3.1(-) (Invitrogen, cat. no. V795-20) by subcloning using the HindIII restriction site of that vector. The correct orientation for the Gsα sequence is determined after subcloning into pcDNA3.1(-). The modified pcDNA3.1(-) containing the rat Gsα gene at HindIII sequence is then verified; this vector is now available as a "universal" Gsα protein vector. The pcDNA3.1(-) vector contains a variety of well-known restriction sites upstream of the HindIII site, thus beneficially providing the ability to insert, upstream of the Gs protein, the coding sequence of a receptor of interest. This same approach can be utilized to create other "universal" G protein vectors, and, of course, other commercially available or proprietary vectors known to the artisan can be utilized the important criteria is that the sequence for the GPCR be upstream and in-frame with that of the G protein.

b. Gq(6 amino acid deletion)/Gi Fusion Construct

The design of a Gq(del)/Gi fusion construct can be accomplished as follows: the N-terminal six (6) amino acids (amino acids 2 through 7, having the sequence of TLESIM (SEQ ID NO:11)) of Gαq-subunit is deleted and the C-terminal five (5) amino acids having the sequence EYNLV (SEQ ID NO:12) is replaced with the corresponding amino acids of the Gαi Protein, having the sequence DCGLF (SEQ ID NO:13). This fusion construct can be obtained by PCR using the following primers:

5'-gatcAAGCTTCCATGGCGTGCTGCCTGAGCGAGGAG-3' (SEQ ID NO:14) and

5'-gatcGGATCCTTAGAACAGGCCGCAGTCCTTCAGGTTCAGCTGCAGGATGGTG-3' (SEQ ID NO:15)

and Plasmid 63313 which contains the mouse Gαq-wild type version with a hemagglutinin tag as template. Nucleotides in lower caps are included as spacers.

TaqPlus Precision DNA polymerase (Stratagene) can be utilized for the amplification by the following cycles, with steps 2 through 4 repeated 35 times: 95°C for 2 min; 95°C for 20 sec; 56°C for 20 sec; 72°C for 2 min; and 72°C for 7 min. The PCR product can be cloned into a pCRII-TOPO vector (Invitrogen) and sequenced using the ABI Big Dye Terminator kit (P.E. Biosystems). Inserts from a TOPO clone containing the sequence of the fusion construct can be shuttled into the expression vector pcDNA3.1(+) at the HindIII/BamHI site by a 2 step cloning process. Also see, PCT Application Number PCT/US02/05625 published as WO02068600 on 6 September 2002, the disclosure of which is hereby incorporated by reference in its entirety.

**Example 14**

**[$^{35}$S]GTPγS Assay**

A. Membrane Preparation

In some embodiments membranes comprising the Target GPCR of interest for use in the identification of candidate compounds as, e.g.,. agonists, inverse agonists or antagonists, are prepared as follows:

a. Materials

"Membrane Scrape Buffer" is comprised of 20mM HEPES and 10mM EDTA, pH 7.4; "Membrane Wash Buffer" is comprised of 20mM HEPES and 0.1mM EDTA, pH 7.4; "Binding Buffer" is comprised of 20mM HEPES, 100 mM NaCl, and 10 mM $MgCl_2$, pH 7.4.

b. Procedure

All materials are kept on ice throughout the procedure. Firstly, the media is aspirated from a confluent monolayer of cells, followed by rinsing with 10ml cold PBS, followed by aspiration. Thereafter, 5ml of Membrane Scrape Buffer is added to scrape cells; this is followed by transfer of cellular extract into 50ml centrifuge tubes (centrifuged at 20,000 rpm for 17 minutes at 4°C). Thereafter, the supernatant is aspirated and the pellet is resuspended in 30ml Membrane Wash Buffer followed by centrifuge at 20,000 rpm for 17 minutes at 4°C. The supernatant is then aspirated and the pellet resuspended in Binding Buffer. This is then homogenized using a Brinkman Polytron™ homogenizer (15-20 second bursts until the all material is in suspension). This is referred to herein as "Membrane Protein".

Bradford Protein Assay

Following the homogenization, protein concentration of the membranes is determined using the Bradford Protein Assay (protein can be diluted to about 1.5mg/ml, aliquoted and frozen (-80°C) for later use; when frozen, protocol for use will be as follows: on the day of the assay, frozen Membrane Protein is thawed at room temperature, followed by vortex and then homogenized with a Polytron at about 12 x 1,000 rpm for about 5-10 seconds; it is noted that for multiple preparations, the homogenizer should be thoroughly cleaned between homogenization of different preparations).

a. Materials

Binding Buffer (as per above); Bradford Dye Reagent; Bradford Protein Standard is utilized, following manufacturer instructions (Biorad, cat. no. 500-0006).

b. Procedure

Duplicate tubes are prepared, one including the membrane, and one as a control "blank". Each tube contains 800μl Binding Buffer. Thereafter, 10μl of Bradford Protein Standard (1mg/ml) is added to each tube, and 10μl of membrane Protein is then added to just one tube (not the blank). Thereafter, 200μl of Bradford Dye Reagent is added to each tube, followed by vortexing of each tube. After five (5) minutes, the tubes are revortexed and the material therein is transferred to cuvettes. The cuvettes are read using a CECIL 3041 spectrophotometer, at wavelength 595.

Identification Assay

a. Materials

GDP Buffer consists of 37.5ml Binding Buffer and 2mg GDP (Sigma, cat. no. G-7127), followed by a series of dilutions in Binding Buffer to obtain 0.2 μM GDP (final concentration of GDP in each well is 0.1 μM GDP); each well comprising a candidate compound has a final volume of 200μl consisting of 100μl GDP Buffer (final concentration, 0.1 μM GDP), 50μl Membrane Protein in Binding Buffer, and 50μl [$^{35}$S]GTPγS (0.6 nM) in Binding Buffer (2.5 μl [$^{35}$S]GTPγS per 10ml Binding Buffer).

b. Procedure

Candidate compounds can be screened using a 96-well plate format (these can be frozen at -80°C). Membrane Protein (or membranes with expression vector excluding the Target GPCR, as control), are homogenized briefly until in suspension. Protein concentration is be determined using the Bradford Protein Assay set forth above. Membrane Protein (and control) is diluted to 0.25mg/ml in Binding Buffer (final assay concentration, 12.5μg/well). Thereafter, 100μl GDP Buffer is added to each well of a Wallac Scintistrip™ (Wallac). A 5μl pin-tool is used to transfer 5 μl of a candidate compound into such well (i.e., 5μl in total assay volume of 200 μl is a 1:40 ratio such that the final screening concentration of the candidate compound is 10μM). Again, to avoid contamination, after each transfer step the pin tool should be rinsed in three reservoirs comprising water (1X), ethanol (1X) and water (2X) - excess liquid should be shaken from the tool after each rinse and dried with paper and kimwipes. Thereafter, 50μl of Membrane Protein is added to each well (a control well comprising membranes without the Target GPCR is also utilized), and pre-incubated for 5-10 minutes at room temperature. Thereafter, 50μl of [$^{35}$S]GTPγS (0.6 nM) in Binding Buffer is added to each well, followed by incubation on a shaker for 60 minutes at room temperature (plates are covered with foil). The assay is then stopped by spinning of the plates at 4000 RPM for 15 minutes at 22°C. The plates are aspirated with an 8 channel manifold and sealed with plate covers. The plates are read on a Wallac 1450 using setting "Prot. #37" (as per manufacturer's instructions).

**Example 15**

**Cyclic AMP Assay**

Another assay approach for identifying candidate compounds as, e.g., agonists, inverse agonist, or antagonists, can accomplished by utilizing a cyclase-based assay. In addition to direct identification, this assay approach can be utilized as an independent approach to provide confirmation of the results from the [$^{35}$S]GTPγS approach as set forth in the above example.

A modified Flash Plate™ Adenylyl Cyclase kit (New England Nuclear; Cat. No. SMP004A) can be utilized for direct identification of candidate compounds as inverse agonists and agonists to a receptor of interest in accordance with the following protocol.

Transfected cells are harvested approximately three days after transfection. Membranes are prepared by homogenization of suspended cells in buffer containing 20mM HEPES, pH 7.4 and 10mM $MgCl_2$. Homogenization is performed on ice using a Brinkman Polytron™ for approximately 10 seconds. The resulting homogenate is centrifuged at 49,000 X g for 15 minutes at 4°C. The resulting pellet is then resuspended in buffer containing 20mM HEPES, pH 7.4 and 0.1 mM EDTA, homogenized for 10 seconds, followed by centrifugation at 49,000 x g for 15 minutes at 4°C. The resulting pellet is then stored at -80°C until utilized. On the day of direct identification screening, the membrane pellet is slowly thawed at room temperature, resuspended in buffer containing 20mM HEPES, pH 7.4 and 10mM $MgCl_2$, to yield a final protein concentration of 0.60mg/ml (the resuspended membranes are placed on ice until use).

cAMP standards and Detection Buffer (comprising 2μCi of tracer [$^{125}$I]cAMP (100μl) to 11ml Detection Buffer] are prepared and maintained in accordance with the manufacturer's instructions. Assay Buffer is prepared fresh for screening and contains 20mM HEPES, pH 7.4, 10mM $MgCl_2$, 20mM phospocreatine (Sigma), 0.1 units/ml creatine phosphokinase (Sigma), 50 μM GTP (Sigma), and 0.2 mM ATP (Sigma); Assay Buffer is then stored on ice until utilized.

Candidate compounds are added to, for example, 96-well plate wells (3μl/well; 12μM final assay concentration), together with 40 μl Membrane Protein (30μg/well) and 50μl of Assay Buffer. This admixture is then incubated for 30 minutes at room temperature, with gentle shaking.

Following the incubation, 100μl of Detection Buffer is added to each well, followed by incubation for 2-24 hours. Plates are then counted in a Wallac MicroBeta™ plate reader using "Prot. #31" (as per manufacturer's instructions).

**Example 16**

**Fluorometric Imaging Plate Reader (FLIPR) Assay for the Measurement of Intracellular Calcium Concentration**

Target Receptor (experimental) and pCMV (negative control) stably transfected cells from respective clonal lines are seeded into poly-D-lysine pretreated 96-well plates (Becton-Dickinson, #356640) at $5.5x10^4$ cells/well with complete culture medium (DMEM with 10% FBS, 2mM L-glutamine, 1mM sodium pyruvate) for assay the next day. Because GPR41 is Gi coupled, the cells comprising GPR41 can further comprise Gα15, Gα16, or the chimeric Gq/Gi alpha subunit. However, since GPR41 is also coupled to Gα12/13 (see Example 5 and Figure 5), a promiscuous G protein such as Gα15, Gα16, or the chimeric Gq/Gi alpha subunit may not be required in order to cause a detectable calcium flux. To prepare Fluo4-AM (Molecular Probe, #F14202) incubation buffer stock, 1 mg Fluo4-AM is dissolved in 467μl DMSO and 467#μl Pluoronic acid (Molecular Probe, #P3000) to give a 1mM stock solution that can be stored at -20°C for a month. Fluo4-AM is a fluorescent calcium indicator dye.

Candidate compounds are prepared in wash buffer (1X HBSS/2.5mM Probenicid/20mM HEPES at pH 7.4).

At the time of assay, culture medium is removed from the wells and the cells are loaded with 100μl of 4μM Fluo4-AM/2.5 mM Probenicid (Sigma, #P8761)/20mM HEPES/complete medium at pH 7.4. Incubation at 37°C/5% $CO_2$ is allowed to proceed for 60 minutes.

After the 1 hour incubation, the Fluo4-AM incubation buffer is removed and the cells are washed 2X with 100 μl wash buffer. In each well is left 100 μl wash buffer. The plate is returned to the incubator at 37°C/5% $CO_2$ for 60 minutes.

FLIPR (Fluorometric Imaging Plate Reader; Molecular Device) is programmed to add 50 μl candidate compound on the 30th second and to record transient changes in intracellular calcium concentration ([Ca2+]) evoked by the candidate compound for another 150 seconds. Total fluorescence change counts are used to determine agonist activity using the FLIPR software. The instrument software normalizes the fluorescent reading to give equivalent initial readings at zero.

Although the foregoing provides a FLIPR assay for agonist activity using stably transfected cells, a person of ordinary skill in the art would readily be able to modify the assay in order to characterize antagonist activity. Said person of ordinary skill in the art would also readily appreciate that, alternatively, transiently transfected cells could be used.

**Example 17**

**MAP Kinase Assay**

MAP kinase (mitogen activated kinase) can be monitored to evaluate receptor activation. MAP kinase can be detected by several approaches. One approach is based on an evaluation of the phosphorylation state, either unphosphorylated (inactive) or phosphorylated (active). The phosphorylated protein has a slower mobility in SDS-PAGE and can therefore be compared with the unstimulated protein using Western blotting. Alternatively, antibodies specific for the phosphorylated protein are available (New England Biolabs) which can be used to detect an increase in the phosphorylated kinase. In either method, cells are stimulated with the candidate compound and then extracted with Laemmli buffer. The soluble fraction is applied to an SDS-PAGE gel and proteins are transferred electrophoretically to nitrocellulose or Immobilin. Immunoreactive bands are detected by standard Western blotting technique. Visible or chemiluminescent signals are recorded on film and can be quantified by densitometry.

Another approach is based on evalulation of the MAP kinase activity via a phosphorylation assay. Cells are stimulated with the candidate compound and a soluble extract is prepared. The extract is incubated at 30°C for 10 minutes with gamma-$^{32}P$-ATP, an ATP regenerating system, and a specific substrate for MAP kinase such as phosphorylated heat and acid stable protein regulated by insulin, or PHAS-I. The reaction is terminated by the addition of $H_3PO_4$ and samples are transferred to ice. An aliquot is spotted onto Whatman P81 chromatography paper, which retains the phosphorylated protein. The chromatography paper is washed and counted for $^{32}P$ is a liquid scintillation counter. Alternatively, the cell extract is incubated with gamma-$^{32}P$-ATP, an ATP regenerating system, and biotinylated myelin basic proein bound by streptavidin to a filter support. The myelin basic protein is a substrate for activated MAP kinase. The phosphorylation reaction is carried out for 10 minutes at 30°C. The extract can then be aspirated through the filter, which retains, the phosphorylated myelin basic protein. The filter is washed and counted for $^{32}P$ by liquid scintillation counting.

**Example 18**

**Receptor Binding Assay**

In addition to the methods described herein, another means for evaluating a candidate compound is by determining binding affinities to the GPR41 receptor. This type of assay generally requires a radiolabelled ligand to the GPR41 receptor. In addition to the use of known ligands for the GPR41 receptor and radiolabels thereof, GPR41 agonist compounds disclosed herein can be labelled with a radioisotope and used in an assay for evaluating the affinity of a candidate compound to the GPR41 receptor.

A radiolabelled GPR41 compound such as a GPR41 agonist disclosed herein can be used in a screening assay to identify/evaluate compounds. In general terms, a newly synthesized or identified compound (i.e., candidate compound) can be evaluated for its ability to reduce binding of the radiolabelled GPR41 agonist to the GPR41 receptor. Accordingly, the ability to compete with the radiolabelled GPR41 agonist for the binding to the GPR41 receptor directly correlates to the binding affinity of the candidate compound to the GPR41 receptor.

**ASSAY PROTOCOL FOR DETERMINING RECEPTOR BINDING FOR GPR41:**

**A. GPR41 RECEPTOR PREPARATION**

For example, HEK293 cells (human kidney, ATCC) can be transiently or stably transfected with GPR41 as described herein. For example, 293 cells can be transiently transfected with 10 μg human GPR41 receptor and 60 μl Lipofectamine (per 15-cm dish), and grown in the dish for 24 hours (75% confluency) with a media change. Cells are removed with 10ml/dish of Hepes-EDTA buffer (20mM Hepes + 10 mM EDTA, pH 7.4). The cells are then centrifuged in a Beckman Coulter centrifuge for 20 minutes, 17,000 rpm (JA-25.50 rotor). Subsequently, the pellet is resuspended in 20mM Hepes + 1 mM EDTA, pH 7.4 and homogenized with a 50- ml Dounce homogenizer and again centrifuged. After removing the supernatant, the pellets are stored at -80°C, until used in binding assay. When used in the assay, membranes are thawed on ice for 20 minutes and then 10mL of incubation buffer (20mM Hepes, 1mM $MgCl_2$,100mM NaCl, pH 7.4) is added. The membranes are then vortexed to resuspend the crude membrane pellet and homogenized with a Brinkmann PT-3100 Polytron homogenizer for 15 seconds at setting 6. The concentration of membrane protein is determined using the BRL Bradford protein assay.

**B. BINDING ASSAY**

For total binding, a total volume of 50μl of appropriately diluted membranes (diluted in assay buffer containing 50mM Tris HCl (pH 7.4), 10mM $MgCl_2$, and 1mM EDTA; 5-50μg protein) is added to 96-well polyproylene microtiter plates followed by addition of 100μl of assay buffer and 50μl of radiolabelled GPR41 agonist. For nonspecific binding, 50μl of assay buffer is added instead of 100μl and an additional 50μl of 10μM cold GPR41 is added before 50μl of radiolabelled GPR41 agonist is added. Plates are then incubated at room temperature for 60-120 minutes. The binding reaction is terminated by filtering assay plates through a Microplate Devices GF/C Unifilter filtration plate with a Brandell 96-well plate harvester followed by washing with cold 50 mM Tris HCl, pH 7.4 containing 0.9% NaCl. Then, the bottom of the filtration plates are sealed, 50μl of Optiphase Supermix is added to each well, the top of the plates are sealed, and plates are counted in a Trilux MicroBeta scintillation counter. For compound competition studies, instead of adding 100μl of assay buffer, 100μl of appropriately diluted candidate compound is added to appropriate wells followed by addition of 50μl of radiolabelled GPR41 agonist.

**C. CALCULATIONS**

The candidate compounds are initially assayed at 1 and 0.1μM and then at a range of concentrations chosen such that the middle dose would cause about 50% inhibition of a radiolabelled GPR41 agonist binding (i.e., $IC_{50}$). Specific binding in the absence of candidate compound ($B_O$) is the difference of total binding ($B_T$) minus non-specific binding (NSB) and similarly specific binding (in the presence of candidate compound) (B) is the difference of displacement binding ($B_D$) minus non-specific binding (NSB). $IC_{50}$ is determined from an inhibition response curve, logit-log plot of % $B/B_O$ vs concentration of candidate compound.

$K_i$ is calculated by the Cheng and Prustoff transformation:

$$K_i = IC_{50} / (1 + [L]/K_D)$$

where [L] is the concentration of a radiolabelled GPR41 agonist used in the assay and $K_D$ is the dissociation constant of a radiolabelled GPR41 agonist determined independently under the same binding conditions.

**Example 19**

**Rodent Diabetes Model**

Rodent models of type 2 diabetes associated with obesity and insulin resistance have been developed. Genetic models such as db/db and ob/ob [see Diabetes (1982) 31:1-6] in mice and fa/fa in zucker rats have been developed for understanding the pathophysiology of disease and for testing candidate therapeutic compounds [Diabetes (1983) 32:830-838; Annu Rep Sankyo Res Lab (1994) 46:1-57]. The homozygous animals, C57 BL/KsJ-db/db mice developed by Jackson Laboratory are obese, hyperglycemic, hyperinsulinemic and insulin resistant [J Clin Invest (1990) 85:962-967], whereas heterozygotes are lean and normoglycemic. In the db/db model, mice progressively develop insulinopenia with age, a feature commonly observed in late stages of human type 2 diabetes when sugar levels are insufficiently controlled. Since this model resembles that of human type 2 diabetes, the compounds of the present invention are tested for activities including, but not limited to, lowering of plasma glucose and triglycerides. Zucker (fa/fa) rats are severely obese, hyperinsulinemic, and insulin resistant {Coleman, Diabetes (1982) 31:1; E Shafrir in Diabetes Mellitus, H Rifkin and D Porte, Jr, Eds [Elsevier Science Publishing Co, New York, ed. 4, (1990), pp. 299-340]}, and the fa/fa mutation may be the rat equivalent of the murine db mutation [Friedman et al, Cell (1992) 69:217-220; Truett et al, Proc Natl Acad Sci USA (1991) 88:7806]. Tubby (tub/tub) mice are characterized by obesity, moderate insulin resistance and hyperinsulinemia without significant hyperglycemia [Coleman et al, Heredity (1990) 81:424].

The present invention encompasses the use GPR41 modulators for reducing the insulin resistance and hyperglycemia in any or all of the above rodent diabetes models, in humans with type 2 diabetes or other preferred insulin-related disorders or disorders of lipid metabolism described previously, or in models based on other mammals. Plasma glucose and insulin levels can be tested, as well as other factors including, but not limited to, plasma free fatty acids and triglycerides.

In Vivo Assay for Anti-Hyperglycemic Activity of GPR41 modulators

Genetically altered obese diabetic mice (db/db) (male, 7-9 weeks old) are housed (7-9 mice/cage) under standard laboratory conditions at 22°C and 50% relative humidity, and maintained on a diet of Purina rodent chow and water ad libitum. Prior to treatment, blood is collected from the tail vein of each animal and blood glucose concentrations are determined using One Touch Basic Glucose Monitor System (Lifescan). Mice that have plasma glucose levels between 250 to 500 mg/dl are used. Each treatment group consists of seven mice that are distributed so that the mean glucose levels are equivalent in each group at the start of the study. db/db mice are dosed by micro-osmotic pumps, inserted using isoflurane anesthesia, to provide compounds of the invention, saline, or an irrelevant compound to the mice subcutaneously (s.c.). Blood is sampled from the tail vein at intervals thereafter and analyzed for blood glucose concentrations. Significant differences between groups (comparing compounds of the interest to saline-treated) are evaluated using Student t-test.

The foregoing is provided by way of illustration and not limitation. Other illustrative rodent models for type 2 diabetes have been described [Moller DE, Nature (2001) 414:821-7 and references therein; and Reed MJ et al., Diabetes, Obesity and Metabolism (1999) 1:75-86 and reference therein; the disclosure of each of which is hereby incorporated by reference in its entirety].

**Example 20**

**Mouse Atherosclerosis Model**

Adiponectin-deficient mice generated through knocking out the adiponectin gene have been shown to be predisposed to atherosclerosis and to be insulin resistant. The mice are also a suitable model for ischemic heart disease [Matsuda, M et al. J Biol Chem (2002) July, and references cited therein, the disclosures of which are incorporated herein by reference in their entirety].

Adiponectin knockout mice are housed (7-9 mice/cage) under standard laboratory conditions at 22°C and 50% relative humidity. The mice are dosed by micro-osmotic pumps, inserted using isoflurane anesthesia, to provide compounds of the invention, saline, or an irrelevant compound to the mice subcutaneously (s.c.). Neointimal thickening and ischemic heart disease are determined for different groups of mice sacrificed at different time intervals. Significant differences between groups (comparing compounds of the interest to saline-treated) are evaluated using Student t-test.

The foregoing mouse model of atherosclerosis is provided by way of illustration and not limitation. By way of

further example, Apolipoprotein E-deficient mice have also been shown to be predisposed to atherosclerosis [Plump AS et al., Cell (1992) 71:343-353; the disclosure of which is hereby incorporated by reference in its entirety].

Another model that can be used is that of diet-induced atherosclerosis in C57BL/6J mice, an inbred strain known to be susceptible to diet-induced atherosclerotic lesion formation. This model is well known to persons of ordinary skill in the art [Kamada N et al., J Atheroscler Thromb (2001) 8:1-6; Garber DW et al., J Lipid Res (2001) 42:545-52; Smith JD et al., J Intern Med (1997) 242:99-109; the disclosure of each of which is hereby incorporated by reference in its entirety].

**Example 21**

**In Vivo Pig Model of HDL-Cholesterol and Atherosclerosis**

The utility of a compound of interest as a medical agent in the prevention or treatment of a high total cholesterol/HDL-cholesterol ratio and conditions relating thereto is demonstrated, for example, by the activity of the compound in lowering the ratio of total cholesterol to HDL-cholesterol, in elevating HDL-cholesterol, or in protection from atherosclerosis in an *in vivo* pig model. Pigs are used as an animal model because they reflect human physiology, especially lipid metabolism, more closely than most other animal models. An illustrative *in vivo* pig model not intended to be limiting is presented here.

Yorkshire albino pigs (body weight 25.5 ± 4 kg) are fed a saturated fatty acid rich and cholesterol rich (SFA-CHO) diet during 50 days (1 kg chow 35 kg-1 pig weight), composed of standard chow supplemented with 2% cholesterol and 20% beef tallow [Royo T et al., European Journal of Clinical Investigation (2000) 30:843-52]. Saturated to unsaturated fatty acid ratio is modified from 0.6 in normal pig chow to 1.12 in the SFA-CHO diet. Animals are divided into two, groups, one group (n = 8) fed with the SFA-CHO diet and treated with placebo and one group (n = 8) fed with the SFA-CHO diet and treated with the modulator (3.0 mg kg-1). Control animals are fed a standard chow for a period of 50 days. Blood samples are collected at baseline (2 days after the reception of the animals), and 50 days after the initiation of the diet. Blood lipids are analyzed. The animals are sacrificed and necropsied.

Alternatively, the foregoing analysis comprises a plurality of groups each treated with a different dose of the compound of interest. Doses include, for example: 0.1 mg kg-1, 0.3 mg kg-1, 1.0 mg kg-1, 3.0 mg kg-1, 10 mg kg-1, 30 mg kg-1 and 100 mg kg-1. Alternatively, the foregoing analysis is carried out at a plurality of timepoints, for example, 10 weeks, 20 weeks, 30 weeks, 40 weeks, and 50 weeks.

HDL-Cholesterol

Blood is collected in trisodium citrate (3.8%, 1:10). Plasma is obtained after centrifugation (1200 g 15 min) and immediately processed. Total cholesterol, HDL-cholesterol, and LDL-cholesterol are measured using the automatic analyzer Kodak Ektachem DT System (Eastman Kodak Company, Rochester, NY, USA). Samples with value parameters above the range are diluted with the solution supplied by the manufacturer and then re-analyzed. The total cholesterol/HDL-cholesterol ratio is determined. Comparison is made of the level of HDL-cholesterol between groups. Comparison is made of the total cholesterol/HDL-cholesterol ratio between groups.

Elevation of HDL-cholesterol or reduction of the total cholesterol/HDL-cholesterol ratio on administration of the compound of interest is taken as indicative of the compound having the aforesaid utility.

Atherosclerosis

The thoracic and abdominal aortas are removed intact, opened longitudinally along the ventral surface, and fixed in neutral-buffered formalin after excision of samples from standard sites in the thoracic and abdominal aorta for histological examination and lipid composition and synthesis studies. After fixation, the whole aortas are stained with Sudan IV and pinned out flat, and digital images are obtained with a TV camera connected to a computerized image analysis system (Image Pro Plus; Media Cybernetics, Silver Spring, MD) to determine the percentage of aortic surface involved with atherosclerotic lesions [Gerrity RG et al, Diabetes (2001) 50:1654-65; Comhill JF et al, Arteriosclerosis, Thrombosis, and Vascular Biology (1985) 5:415-26; which disclosures are hereby incorporated by reference in their entirety]. Comparison is made between groups of the percentage of aortic surface involved with atherosclerotic lesions.

Reduction of the percentage of aortic surface involved with atherosclerotic lesions on administration of the compound of interest is taken as indicative of the compound having the aforesaid utility.

Plasma Free Fatty Acids

It would be readily apparent to one of ordinary skill in the art that the foregoing *in vivo* pig model is easily

modified in order to address, without limitation, the activity of a compound in lowering plasma free fatty acids.

## Claims

1. A method for identifying a glycemic stabilizing compound, comprising:

    a) contacting a candidate compound with GPR41, and
    b) determining whether GPR41 functionality is modulated,

    wherein a modulation in GPR41 functionality is indicative of the candidate compound being a glycemic stabilizing compound;
    said GPR41 having an amino acid sequence as shown in SEQ ID NO:2, or a variant or ortholog thereof, wherein said variant is an allelic variant, splice variant or conservative amino acid substitution variant of GPR41.

2. A method according to claims 1, wherein said variant retains the function of a polypeptide with the amino acid sequence as shown in SEQ ID NO:2.

3. A method according to claim 1, wherein said GPR41 is human.

4. A method according to claim 1, wherein said determining comprises a second messenger assay.

5. A glycemic stabilizing compound identified by the method according to claim 1 selected from the following compounds and pharmaceutically acceptable salts thereof:

    2-methyl-4-(4-nitro-phenyl)-5-oxo-1,4,5,6,7,8-hexahydro-quinoline-3-carboxylic acid o-tolylamide,
    4-Furan-3-yl-2-methyl-5-oxo-1,4,5,6,7,8-hexahydro-quinoline-3-carboxylic acid o-tolylamide,
    4-Furan-3-yl-2-methyl-5-oxo-1,4,5,6,7,8-hexahydro-quinoline-3-carboxylic acid (2,5-dichloro-phenyl)-amide,
    4-Furan-2-yl-2-methyl-5-oxo-1,4,5,6,7,8-hexahydro-quinoline-3-carboxylic acid o-tolylamide,
    4-Furan-3-yl-2-methyl-5-oxo-1,4,5,6,7,8-hexahydro-quinoline-3-carboxylic acid (4-chloro-phenyl)-amide,
    2-Methyl-4-(4-methylsulfanyl-phenyl)-5-oxo-1,4,5,6,7,8-hexahydroquinoline-3-carboxylic acid o-tolylamide,
    2-Methyl-4-(3-nitro-phenyl)-5-oxo-1,4,5,6,7,8-hexahydro-quinoline-3-carboxylic acid o-tolylamide,
    2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide,
    4-((5-Biphenyl-2-yl-furan-2-yl)-2-methyl-5-oxo-1,4,5,6,7,8-hexahydroquinoline-3-carboxylic acid o-tolylamide,
    2-Methyl-4-[5-(2-nitro-phenyl)-furan-2-yl]-5-oxo-1,4,5,6,7,8-hexahydroquinoline=3-carboxylic acid (2-chloro-phenyl)-amide,
    2-Methyl-5-oxo-4-(4-phenoxy-phenyl)-1,4,5,6,7,8-hexahydro-quinoline-3-carboxylic acid o-tolylamide,
    2-Methyl-5-oxo-4-[5-(2-trifluoromethoxy-phenyl)-furan-2-yl]-1,4,5,6,7,8-hexahydro-quinoline-3-carboxylic acid o-tolylamide, and
    4-[5-(2,5-Dichloro-phenyl)-furan-2-yl]-2-methyl-5-oxo-1,4,5,6,7,8-hexahydro-quinoline-3-carboxylic acid o-tolylamide.

6. A compound according to claim 5, wherein said glycemic stabilizing compound is a GPR41 agonist.

7. A compound according to claim 6, wherein said glycemic stabilizing compound is a compound selected from the following compounds and pharmaceutically acceptable salts thereof:

    2-methyl-4-(4-nitro-phenyl)-5-oxo-1,4,5,6,7,8-hexahydro-quinoline-3-carboxylic acid o-tolylamide,
    4-Furan-3-yl-2-methyl-5-oxo-1,4,5,6,7,8-hexahydro-quinoline-3-carboxylic acid o-tolylamide,
    4-Furan-3-yl-2-methyl-5-oxo-1,4,5,6,7,8-hexahydro-quinoline-3-carboxylic acid (2,5-dichloro-phenyl)-amide,

4-Furan-2-yl-2-methyl-5-oxo-1,4,5,6,7,8-hexahydro-quinoline-3-carboxylic acid o-tolylamide,
4-Furan-3-yl-2-methyl-5-oxo-1,4,5,6,7,8-hexahydro-quinoline-3-carboxylic acid (4-chloro-phenyl)-amide,
2-Methyl-4-(4-methylsulfanyl-phenyl)-5-oxo-1,4,5,6,7,8-hexahydroquinoline-3-carboxylic acid o-tolylamide, and
2-Methyl-4-(3-nitro-phenyl)-5-oxo-1,4,5,6,7,8-hexahydro-quinoline-3-carboxylic acid o-tolylamide.

**8.** A compound according to claim 5, wherein said glycemic stabilizing compound is a GPR41 inverse agonist or antagonist.

**9.** A compound according to claim 8, wherein said glycemic stabilizing compound comprises a compound selected from the following compounds and pharmaceutically acceptable salts thereof:

2-Methyl-4-[5-(2-nitro-4-trifluoromethyl-phenyl)-furan-2-yl]-5-oxo-1,4,5,6,7,8-hexahydro-quinoline-3-carboxylic acid o-tolylamide,
4-((5-Biphenyl-2-yl-furan-2-yl)-2-methyl-5-oxo-1,4,5,6,7,8-hexahydro-quinoline-3-carboxylic acid o-tolylamide,
2-Methyl-4-[5-(2-nitro-phenyl)-furan-2-yl]-5-oxo-1,4,5,6,7,8-hexahydro-quinoline-3-carboxylic acid (2-chloro-phenyl)-amide,
2-Methyl-5-oxo-4-(4-phenoxy-phenyl)-1,4,5,6,7,8-hexahydro-quinoline-3-carboxylic acid o-tolylamide,
2-Methyl-5-oxo-4-[5-(2-trifluoromethoxy-phenyl)-furan-2-yl]-1,4,5,6,7,8-hexahydro-quinoline-3-carboxylic acid o-tolylamide, and
4-[5-(2,5-Dichloro-phenyl)-furan-2-yl]-2-methyl-5-oxo-1,4,5,6,7,8-hexahydro-quinoline-3-carboxylic acid o-tolylamide.

**10.** A method for preparing a composition comprising a glycemic stabilizing compound said method including admixing said compound with a carrier, wherein said compound is a compound according to any one of claims 5 to 9.

**11.** A pharmaceutical composition comprising, consisting essentially of, or consisting of a compound according to any one of claims 5 to 9.

**12.** A compound according to any one of claims 5 to 9 for use in a method of treatment of human or animal body by therapy.

**13.** A compound according to any one of claims 5 to 9 for use in a method of treatment of an insulin-related disorder.

**14.** Use of a compound according to any one of claims 5 to 9 in the manufacture of a medicament for the treatment of an insulin-related disorder.

**15.** A compound according to claim 5 for use in a method of treatment of hypoglycemia, an insulin-secreting or insulin-dependent tumor, insulin resistance, impaired glucose tolerance, or diabetes.

**16.** Use of a compound according to claim 5 in the manufacture of a medicament for the treatment of hypoglycemia, an insulin-secreting or insulin-dependent tumor, insulin resistance, impaired glucose tolerance, or diabetes.

**17.** A method according to any one of claims 1 to 4 wherein step b) comprises determining whether GPR41 functionality is decreased,
wherein a decrease in GPR41 functionality is indicative of the candidate compound being a glycemic stabilizing compound.

**18.** A method according to claim 17 wherein said glycemic stabilizing compound is capable of increasing insulin secretion.

**19.** A compound according to claim 12 for use in a method of treatment of the human body by therapy.

**Patentansprüche**

**1.** Verfahren zur Identifizierung einer Glykämie-stabilisierenden Verbindung, das Folgendes umfasst:

a) das Kontaktieren einer Kandidatenverbindung mit GPR41 und
b) das Bestimmen, ob die Funktionalität von GPR41 moduliert wird,

worin eine Modulation der Funktionalität von GPR41 ein Indikator dafür ist, dass es sich bei der Kandidatenverbindung um eine Glykämie-stabilisierende Verbindung handelt;
wobei der GPR41 eine Aminosäuresequenz wie in Seq.-ID Nr. 2 gezeigt oder eine Variante oder ein Ortholog davon aufweist, worin die Variante eine Allelvariante, eine Spleißvariante oder eine Variante von GPR41 mit konservativer Aminosäuresubstitution ist.

**2.** Verfahren nach Anspruch 1, worin die Variante die Funktion eines Polypeptids mit der in Seq.-ID Nr. 2 gezeigten Aminosäuresequenz beibehält.

**3.** Verfahren nach Anspruch 1, worin der GPR41 menschlich ist.

**4.** Verfahren nach Anspruch 1, worin das Bestimmen einen Test mit zweitem Messenger umfasst.

**5.** Glykämie-stabilisierende Verbindung, identifiziert durch ein Verfahren nach Anspruch 1 und ausgewählt aus den nachstehenden Verbindungen und pharmazeutisch annehmbaren Salzen davon:

2-Methyl-4-(4-nitrophenyl)-5-oxo-1,4,5,6,7,8-hexahydrochinolin-3-carbonsäure-o-tolylamid,
4-Furan-3-yl-2-methyl-5-oxo-1,4,5,6,7,8-hexahydrochinolin-3-carbonsäure-o-tolylamid,
4-Furan-3-yl-2-methyl-5-oxo-1,4,5,6,7,8-hexahydrochinolin-3-carbonsäure-(2,5-dichlorphenyl)amid,
4-Furan-2-yl-2-methyl-5-oxo-1,4,5,6,7,8-hexahydrochinolin-3-carbonsäure-o-tolylamid,
4-Furan-3-yl-2-methyl-5-oxo-1,4,5,6,7,8-hexahydrochinolin-3-carbonsäure-(4-chlorphenyl)amid,
2-Methyl-4-(4-methylsulfanylphenyl)-5-oxo-1,4,5,6,7,8-hexahydrochinolin-3-carbonsäure-o-tolylamid,
2-Methyl-4-(3-nitrophenyl)-5-oxo-1,4,5,6,7,8-hexahydrochinolin-3-carbonsäure-o-tolylamid,
2-Methyl-4-[5-nitro-4-trifluormethylphenyl)furan-2-yl]-5-oxo-1,4,5,6,7,8-hexahydrochinolin-3-carbonsäure-o-tolylamid,
4-((5-Biphenyl-2-ylfuran-2-yl)-2-methyl-5-oxo-1,4,5,6,7,8-hexahydrochinolin-3-carbonsäure-o-tolylamid,
2-Methyl-4-[5-(2-nitrophenyl)furan-2-yl]-5-oxo-1,4,5,6,7,8-hexahydrochinolin-3-carbonsäure-o-tolylamid,
2-Methyl-5-oxo-4-(4-phenoxyphenyl)-1,4,5,6,7,8-hexahydrochinolin-3-carbonsäure-o-tolylamid,
2-Methyl-5-oxo-4-[5-(2-trifluormethoxyphenyl)furan-2-yl]-1,4,5,6,7,8-hexahydrochinolin-3-carbonsäure-o-tolylamid und
4-[5-(2,5-Dichlorphenyl)furan-2-yl]-2-methyl-5-oxo-1,4,5,6,7,8-hexahydrochinolin-3-carbonsäure-o-tolylamid.

**6.** Verbindung nach Anspruch 5, worin die Glykämie-stabilisierende Verbindung ein GPR41-Agonist ist.

**7.** Verbindung nach Anspruch 6, worin die Glykämie-stabilisierende Verbindung eine aus der aus den nachstehenden Verbindungen und pharmazeutisch annehmbaren Salzen davon bestehenden Gruppe ausgewählte Verbindung ist:

2-Methyl-4-(4-nitrophenyl)-5-oxo-1,4,5,6,7,8-hexahydrochinolin-3-carbonsäure-o-tolylamid,
4-Furan-3-yl-2-methyl-5-oxo-1,4,5,6,7,8-hexahydrochinolin-3-carbonsäure-o-tolylamid,
4-Furan-3-yl-2-methyl-5-oxo-1,4,5,6,7,8-hexahydrochinolin-3-carbonsäure-(2,5-dichlorphenyl)amid,
4-Furan-2-yl-2-methyl-5-oxo-1,4,5,6,7,8-hexahydrochinolin-3-carbonsäure-o-tolylamid,
4-Furan-3-yl-2-methyl-5-oxo-1,4,5,6,7,8-hexahydrochinolin-3-carbonsäure-(4-chlorphenyl)amid,
2-Methyl-4-(4-methylsulfanylphenyl)-5-oxo-1,4,5,6,7,8-hexahydrochinolin-3-carbonsäure-o-tolylamid,
2-Methyl-4-(3-nitrophenyl)-5-oxo-1,4,5,6,7,8-hexahydrochinolin-3-carbonsäure-o-tolylamid.

**8.** Verbindung nach Anspruch 5, worin die Glykämie-stabilisierende Verbindung ein inverser Agonist oder Antagonist von GPR41 ist.

**9.** Verbindung nach Anspruch 8, worin die Glykämie-stabilisierende Verbindung eine aus der aus den nachstehenden Verbindungen und pharmazeutisch annehmbaren Salzen davon bestehenden Gruppe ausgewählte Verbindung umfasst:

2-Methyl-4-[5-nitro-4-trifluormethylphenyl)furan-2-yl]-5-oxo-1,4,5,6,7,8-hexahydrochinolin-3-carbonsäure-o-tolylamid,
4-((5-Biphenyl-2-ylfuran-2-yl)-2-methyl-5-oxo-1,4,5,6,7,8-hexahydrochinolin-3-carbonsäure-o-tolylamid,
2-Methyl-4-[5-(2-nitrophenyl)furan-2-yl]-5-oxo-1,4,5,6,7,8-hexahydrochinolin-3-carbonsäure-o-tolylamid,
2-Methyl-5-oxo-4-(4-phenoxyphenyl)-1,4,5,6,7,8-hexahydrochinolin-3-carbonsäure-o-tolylamid,
2-Methyl-5-oxo-4-[5-(2-trifluormethoxyphenyl)furan-2-yl]-1,4,5,6,7,8-hexa-hydrochinolin-3-carbonsäure-o-tolylamid und
4-[5-(2,5-Dichlorphenyl)furan-2-yl]-2-methyl-5-oxo-1,4,5,6,7,8-hexahydrochinolin-3-carbonsäure-o-tolylamid.

**10.** Verfahren zur Herstellung einer Zusammensetzung, die eine Glykämie-stabilisierende Verbindung umfasst, wobei das Verfahren das Vermischen der Verbindung mit einem Träger umfasst, worin die Verbindung eine Verbindung nach einem der Ansprüche 5 bis 9 ist.

**11.** Pharmazeutische Zusammensetzung, die eine Verbindung nach einem der Ansprüche 5 bis 9 umfasst, im Wesentlichen daraus besteht oder daraus besteht.

**12.** Verbindung nach einem der Ansprüche 5 bis 9 zur Verwendung in einem Verfahren zur Behandlung des menschlichen oder tierischen Körpers durch Therapie.

**13.** Verbindung nach einem der Ansprüche 5 bis 9 zur Verwendung in einem Verfahren zur Behandlung einer insulinbedingten Erkrankung.

**14.** Verwendung einer Verbindung nach einem der Ansprüche 5 bis 9 bei der Herstellung eines Medikaments zur Behandlung einer insulinbedingten Erkrankung.

**15.** Verbindung nach Anspruch 5 zur Verwendung in einem Verfahren zur Behandlung von Hypoglykämie, einem insulinsekretierenden oder insulinabhängigen Tumor, Insulinresistenz, gestörter Glucosetoleranz oder Diabetes.

**16.** Verwendung einer Verbindung nach Anspruch 5 bei der Herstellung eines Medikaments zur Behandlung von Hypoglykämie, einem insulinsekretierenden oder insulinabhängigen Tumor, Insulinresistenz, gestörter Glucosetoleranz oder Diabetes.

**17.** Verfahren nach einem der Ansprüche 1 bis 4, worin Schritt b) das Bestimmen dessen umfasst, ob die Funktionalität von GPR41 zurückgegangen ist,
worin ein Rückgang der Funktionalität von GPR41 ein Indikator dafür ist, dass die Kandidatenverbindung eine Glykämie-stabilisierende Verbindung ist.

**18.** Verfahren nach Anspruch 17, worin die Glykämie-stabilisierende Verbindung in der Lage ist, die Insulinsekretion zu erhöhen.

**19.** Verbindung nach Anspruch 12 zur Verwendung in einem Verfahren zur Behandlung des menschlichen Körpers durch Therapie.

## Revendications

**1.** Procédé pour identifier un composé stabilisant la glycémie, qui consiste à :

a) mettre en contact un composé candidat avec GPR41, et
b) déterminer si la fonctionnalité de GPR41 est modulée,

où une modulation de la fonctionnalité de GPR41 indique que le composé candidat est un composé stabilisant la glycémie ;
ledit GPR41 ayant une séquence d'acides aminés telle que montrée dans SEQ ID NO: 2, ou un variant ou un orthologue de celle-ci, où ledit variant est un variant allélique, un variant d'épissage ou un variant de GPR41 présentant une substitution d'acide aminé conservatrice.

**2.** Procédé selon la revendication 1, dans lequel ledit variant conserve la fonction d'un polypeptide ayant la séquence d'acides aminés telle que montrée dans SEQ ID NO: 2.

**3.** Procédé selon la revendication 1, dans lequel ledit GPR41 est humain.

**4.** Procédé selon la revendication 1, dans lequel ladite détermination comprend un essai de messager secondaire.

**5.** Composé stabilisant la glycémie identifié par le procédé selon la revendication 1, sélectionné parmi les composés suivants et sels pharmaceutiquement acceptables de ceux-ci :

le o-tolylamide d'acide 2-méthyl-4-(4-nitro-phényl)-5-oxo-1,4,5,6,7,8-hexahydro-quinoline-3-carboxylique,
le o-tolylamide d'acide 4-furan-3-yl-2-méthyl-5-oxo-l,4,5,6,7,8-hexahydro-quinoline-3-carboxylique,
le (2,5-dichloro-phényl)-amide d'acide 4-furan-3-yl-2-méthyl-5-oxo-1,4,5,6,7,8-hexahydro-quinoline-3-carboxylique,
le o-tolylamide d'acide 4-furan-2-yl-2-méthyl-5-oxo-1,4,5,6,7,8-hexahydro-quinoline-3-carboxylique,
le (4-chloro-phényl)-amide d'acide 4-furan-3-yl-2-méthyl-5-oxo-1,4,5,6,7,8-hexahydro-quinoline-3-carboxylique,
le o-tolylamide d'acide 2-méthyl-4-(4-méthylsulfanyl-phényl)-5-oxo-1,4,5,6,7,8-hexahydro-quinoline-3-carboxylique,
le o-tolylamide d'acide 2-méthyl-4-(3-nitro-phényl)-5-oxo-1,4,5,6,7,8-hexahydro-quinoline-3-carboxylique,
le o-tolylamide d'acide 2-méthyl-4-[5-(2-nitro-4-trifluorométhyl-phényl)-furan-2-yl]-5-oxo-1,4,5,6,7,8-hexahydro-quinoline-3-carboxylique,
le o-tolylamide d'acide 4-(5-biphényl-2-yl-furan-2-yl)-2-méthyl-5-oxo-1,4,5,6,7,8-hexahydro-quinoline-3-carboxylique,
le (2-chloro-phényl)-amide d'acide 2-méthyl-4-[5-(2-nitro-phényl)-furan-2-yl]-5-oxo-1,4,5,6,7,8-hexahydroquinoline-3-carboxylique,
le o-tolylamide d'acide 2-méthyl-5-oxo-4-(4-phénoxyphényl)-1,4,5,6,7,8-hexahydro-quinoline-3-carboxylique,
le o-tolylamide d'acide 2-méthyl-5-oxo-4-[5-(2-trifluorométhoxy-phényl)-furan-2-yl]-5-oxo-1,4,5,6,7,8-hexahydro-quinoline-3-carboxylique, et
le o-tolylamide d'acide 4-[5-(2,5-dichloro-phényl)-furan-2-yl]-2-méthyl-5-oxo-1,4,5,6,7,8-hexahydro-quinoline-3-carboxylique.

**6.** Composé selon la revendication 5, où ledit composé stabilisant la glycémie est un agoniste de GPR41.

**7.** Composé selon la revendication 6, où ledit composé stabilisant la glycémie est un composé sélectionné parmi les composés suivants et sels pharmaceutiquement acceptables de ceux-ci :

le o-tolylamide d'acide 2-méthyl-4-(4-nitro-phényl)-5-oxo-1,4,5,6,7,8-hexahydro-quinoline-3-carboxylique,
le o-tolylamide d'acide 4-furan-3-yl-2-méthyl-5-oxo-1,4,5,6,7,8-hexahydro-quinoline-3-carboxylique,
le (2,5-dichloro-phényl)-amide d'acide 4-furan-3-yl-2-méthyl-5-oxo-1,4,5,6,7,8-hexahydro-quinoline-3-carboxylique,
le o-tolylamide d'acide 4-furan-2-yl-2-méthyl-5-oxo-1,4,5,6,7,8-hexahydro-quinoline-3-carboxylique,
le (4-chloro-phényl)-amide d'acide 4-furan-3-yl-2-méthyl-5-oxo-1,4,5,6,7,8-hexahydro-quinoline-3-carboxylique,
le o-tolylamide d'acide 2-méthyl-4-(4-méthylsulfanyl-phényl)-5-oxo-1,4,5,6,7,8-hexahydro-quinoline-3-carboxylique, et
le o-tolylamide d'acide 2-méthyl-4-(3-nitro-phényl)-5-oxo-1,4,5,6,7,8-hexahydro-quinoline-3-carboxylique.

**8.** Composé selon la revendication 5, où ledit composé stabilisant la glycémie est un agoniste inverse ou un antagoniste de GPR41.

**9.** Composé selon la revendication 8, où ledit composé stabilisant la glycémie comprend un composé sélectionné parmi les composés suivants et sels pharmaceutiquement acceptables de ceux-ci :

le o-tolylamide d'acide 2-méthyl-4-[5-(2-nitro-4-trifluorométhyl-phényl)-furan-2-yl]-5-oxo-1,4,5,6,7,8-hexahydro-quinoline-3-carboxylique,
le o-tolylamide d'acide 4-(5-biphényl-2-yl-furan-2-yl)-2-méthyl-5-oxo-1,4,5,6,7,8-hexahydro-quinoline-3-carboxylique,
le (2-chloro-phényl)-amide d'acide 2-méthyl-4-[5-(2-nitro-phényl)-furan-2-yl]-5-oxo-1,4,5,6,7,8-hexahydroquinoline-3-carboxylique,
le o-tolylamide d'acide 2-méthyl-5-oxo-4-(4-phénoxyphényl)-1,4,5,6,7,8-hexahydro-quinoline-3-carboxylique,
le o-tolylamide d'acide 2-méthyl-5-oxo-4-[5-(2-trifluorométhoxy-phényl)-furan-2-yl]-5-oxo-1,4,5,6,7,8-hexahydro-quinoline-3-carboxylique, et
le o-tolylamide d'acide 4-[5-(2,5-dichloro-phényl)-furan-2-yl]-2-méthyl-5-oxo-1,4,5,6,7,8-hexahydro-quinoline-3-carboxylique.

**10.** Procédé pour préparer une composition comprenant un composé stabilisant la glycémie, ledit procédé consistant à mélanger ledit composé avec un véhicule, où ledit composé est un composé selon l'une quelconque des revendications 5 à 9.

**11.** Composition pharmaceutique comprenant, consistant essentiellement en, ou consistant en un composé selon l'une quelconque des revendications 5 à 9.

**12.** Composé selon l'une quelconque des revendications 5 à 9, destiné à être utilisé dans un procédé de traitement du corps humain ou animal par une thérapie.

**13.** Composé selon l'une quelconque des revendications 5 à 9, destiné à être utilisé dans un procédé de traitement d'un trouble lié à l'insuline.

**14.** Utilisation d'un composé selon l'une quelconque des revendications 5 à 9 dans la fabrication d'un médicament destiné au traitement d'un trouble associé à l'insuline.

**15.** Composé selon la revendication 5 destiné à être utilisé dans un procédé de traitement de l'hypoglycémie, d'une tumeur sécrétant de l'insuline ou insulino-dépendante, de l'insulinorésistance, de l'intolérance au glucose, ou du diabète.

**16.** Utilisation d'un composé selon la revendication 5 dans la fabrication d'un médicament destiné au traitement de l'hypoglycémie, d'une tumeur sécrétant de l'insuline ou insulino-dépendante, de l'insulinorésistance, de l'intolérance au glucose, ou du diabète.

**17.** Procédé selon l'une quelconque des revendications 1 à 4, dans lequel l'étape b) consiste à déterminer si la fonctionnalité de GPR41 est réduite,
où une réduction de la fonctionnalité de GPR41 indique que le composé candidat est un composé stabilisant la glycémie.

**18.** Procédé selon la revendication 17, dans lequel ledit composé stabilisant la glycémie est capable d'augmenter la sécrétion d'insuline.

**19.** Composé selon la revendication 12 destiné à être utilisé dans un procédé de traitement du corps humain par une thérapie.

# GPR41 Dot Blot Expression In Pancreas

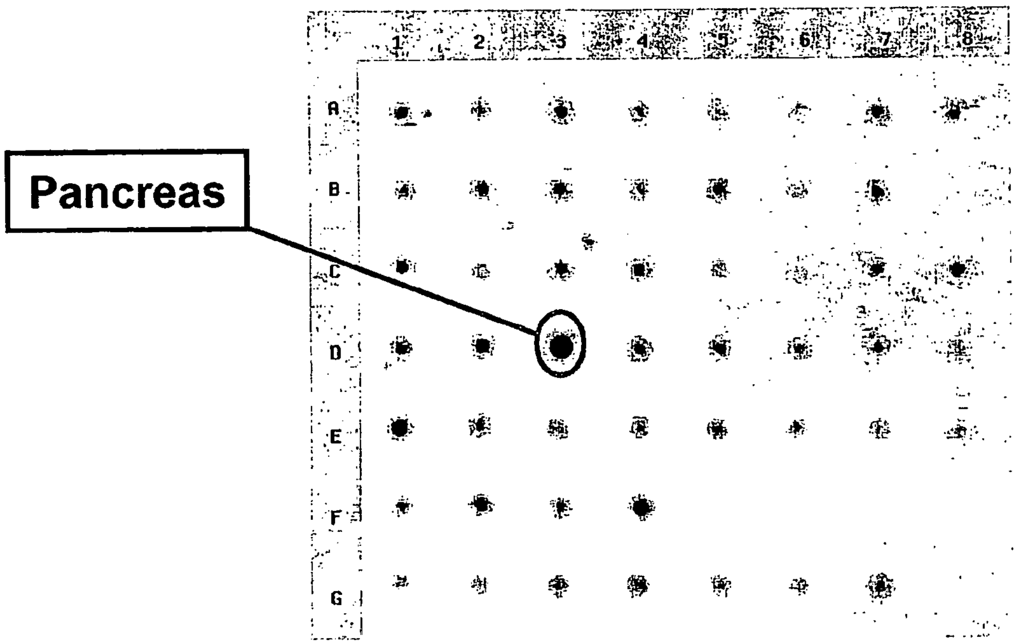


Figure 1

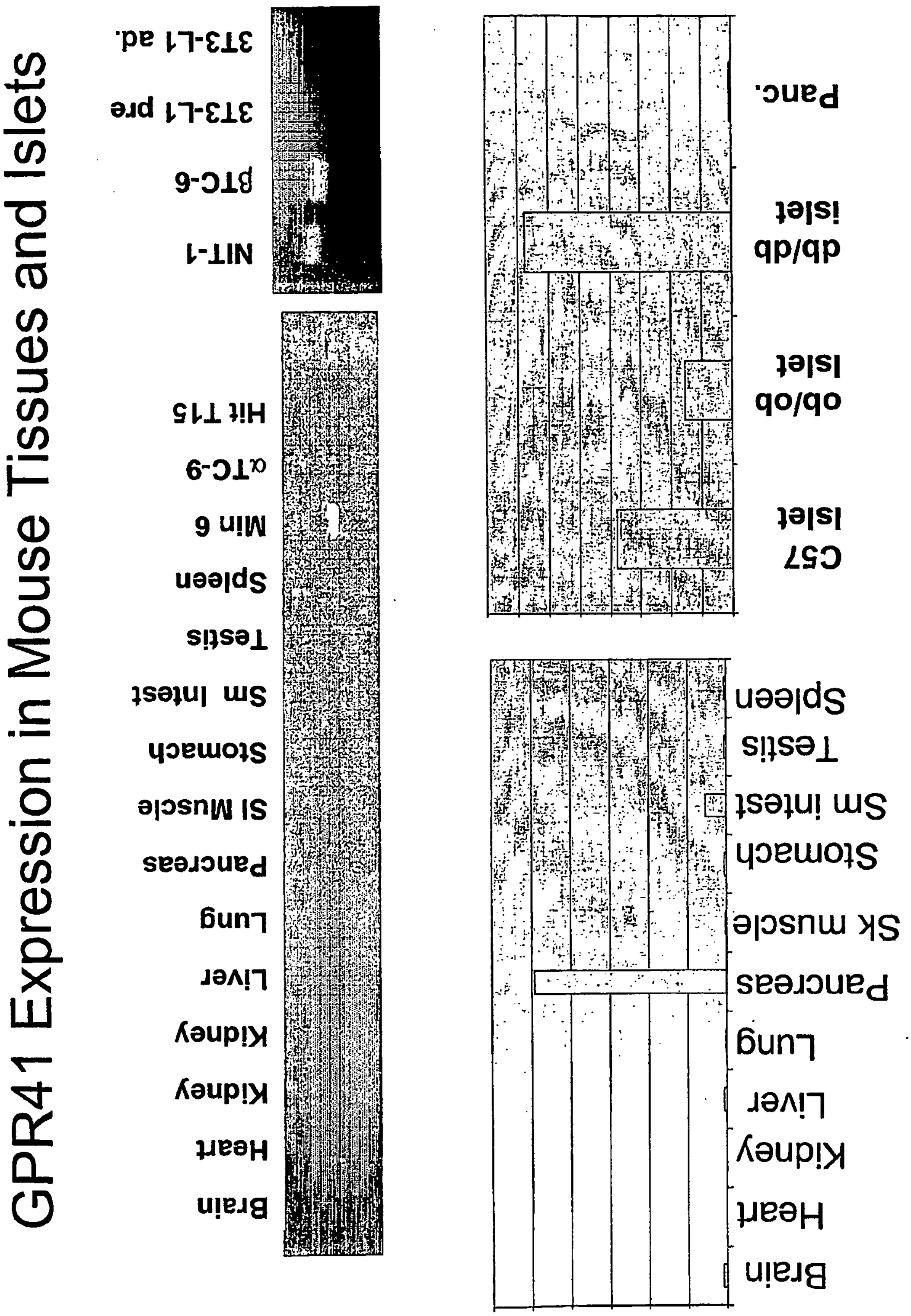
GPR41 Expression in Mouse Tissues and Islets
Brain
Heart
Kidney
Kidney
Liver
Lung
Pancreas
Sl Muscle
Stomach
Sm Intest
Testis
Spleen
Min 6
αTC-9
Hit T15
NIT-1
βTC-6
3T3-L1 pre
3T3-L1 ad.
Brain
Heart
Kidney
Liver
Lung
Pancreas
Sk muscle
Stomach
Sm intest
Testis
Spleen
C57 islet
ob/ob islet
db/db islet
Panc.

Figure 2

GRP41 RNase Protection Assay

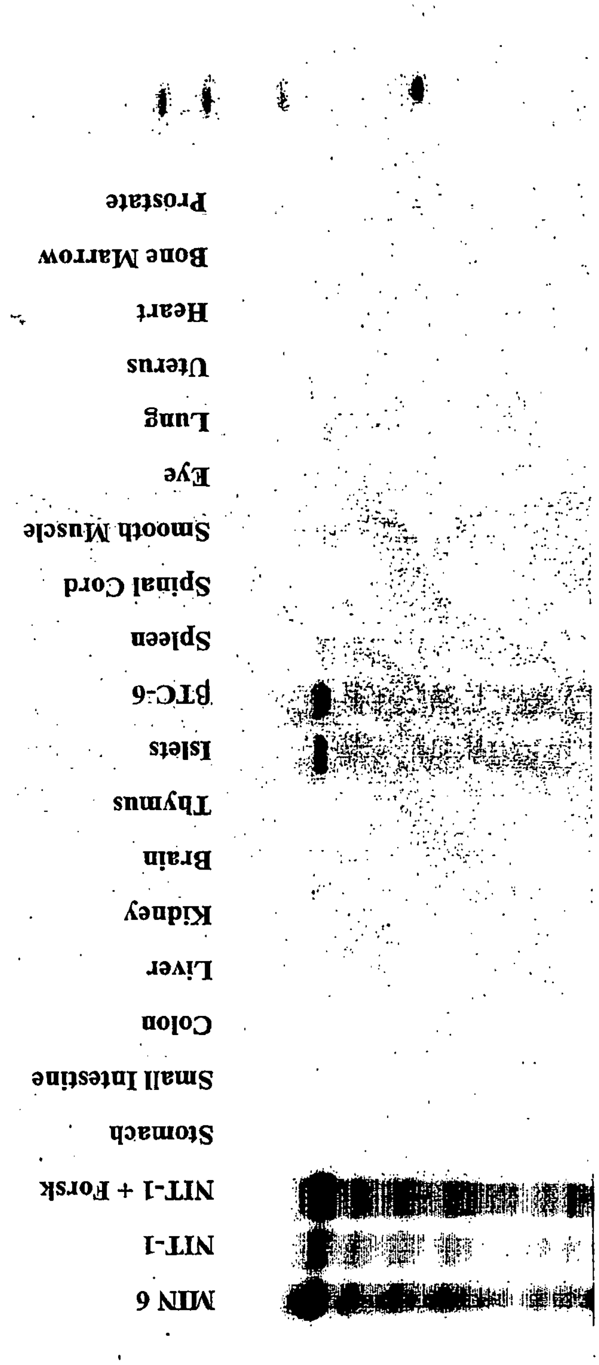


Figure 3

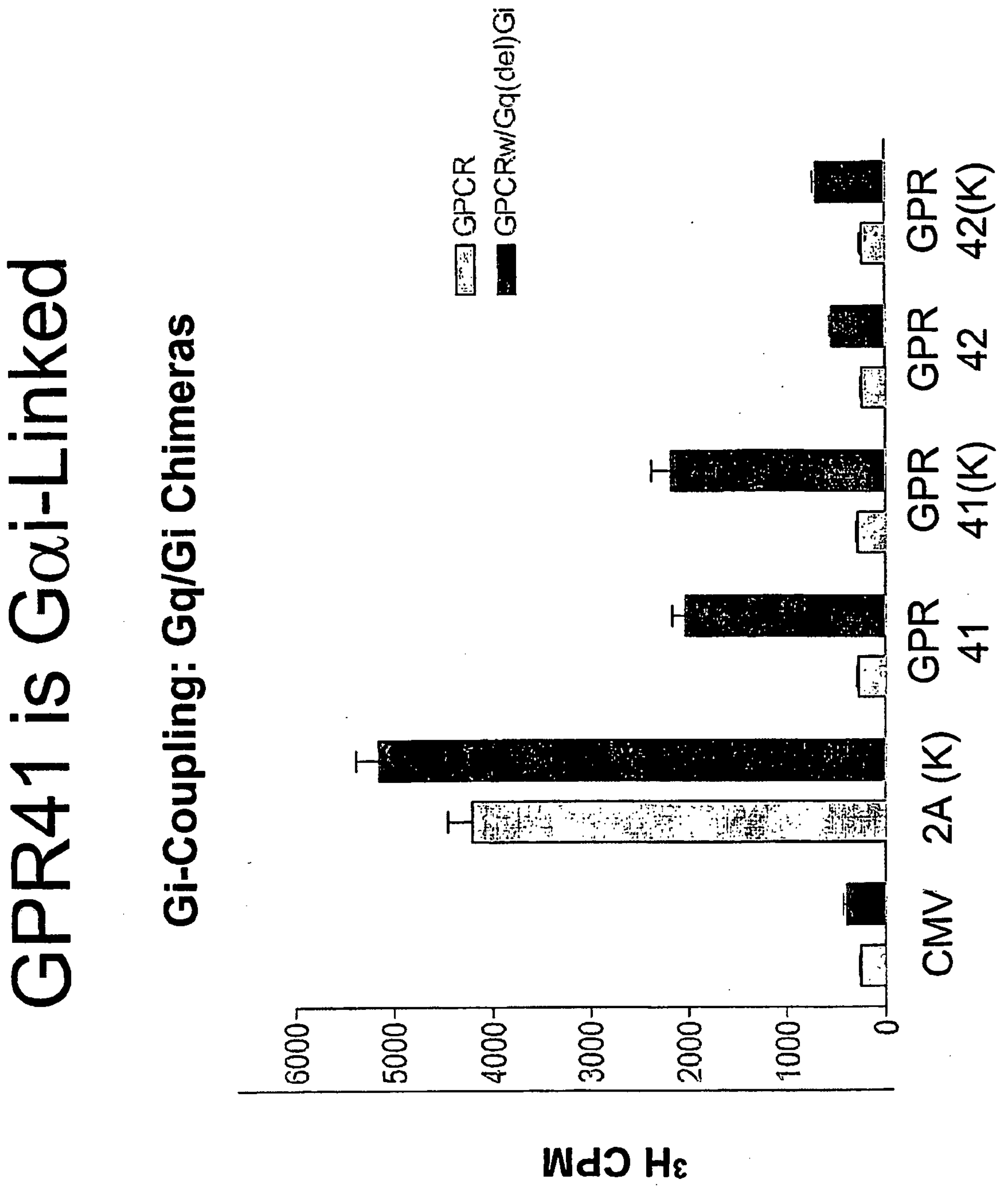
GPR41 is Gαi-Linked
Gi-Coupling: Gq/Gi Chimeras
GPCR
GPCRw/Gq(del)Gi
3H CPM
6000
5000
4000
3000
2000
1000
0
CMV
2A (K)
GPR 41
GPR 41(K)
GPR 42
GPR 42(K)

Figure 4

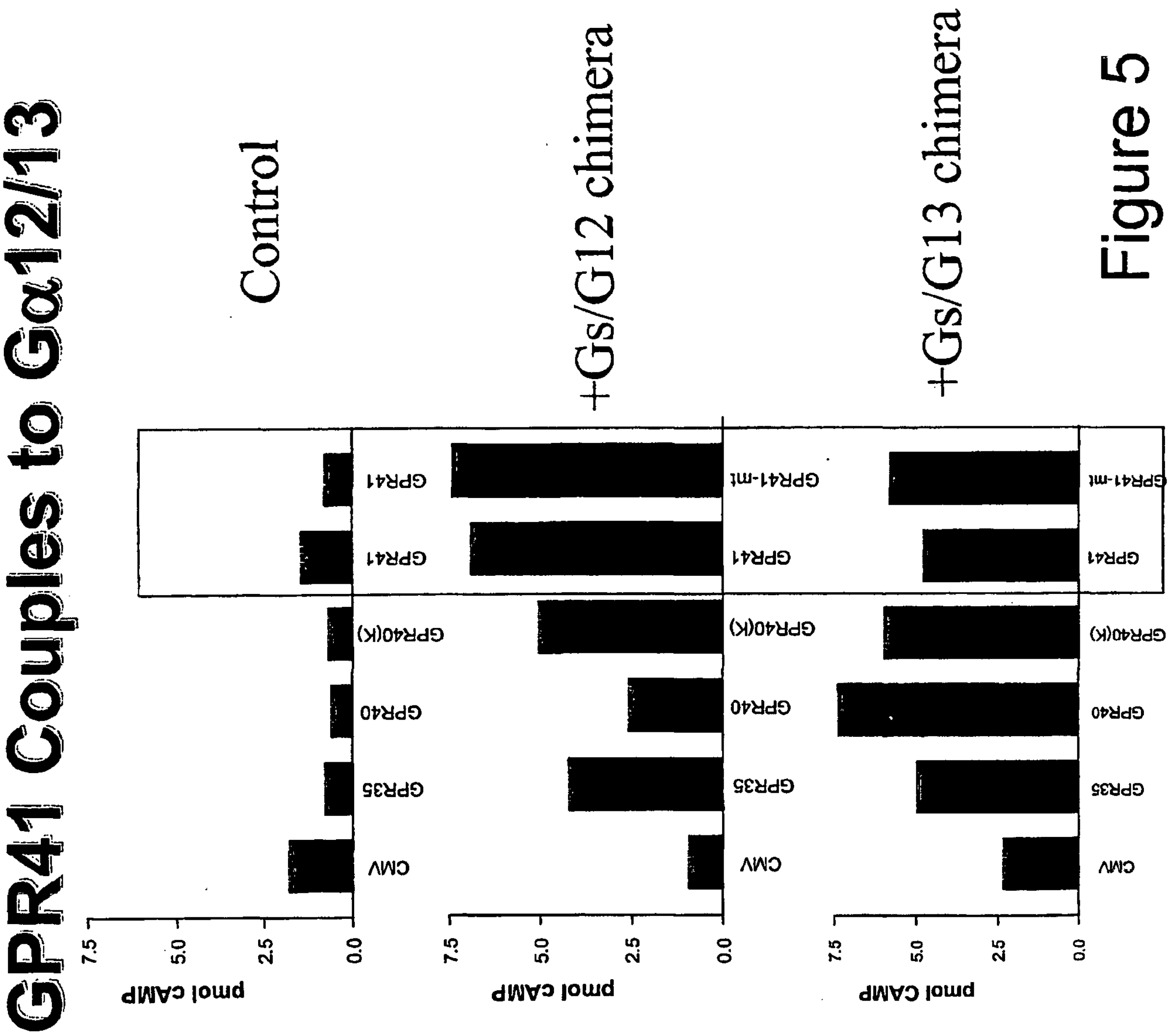
GPR41 Couples to Gα12/13
Control
+Gs/G12 chimera
+Gs/G13 chimera
pmol cAMP
7.5
5.0
2.5
0.0
CMV
GPR35
GPR40
GPR40(K)
GPR41
GPR41-mt

Figure 5

Efficacy of GPR41 Agonists
in Gq/Gi Cotransfected 293 Cells

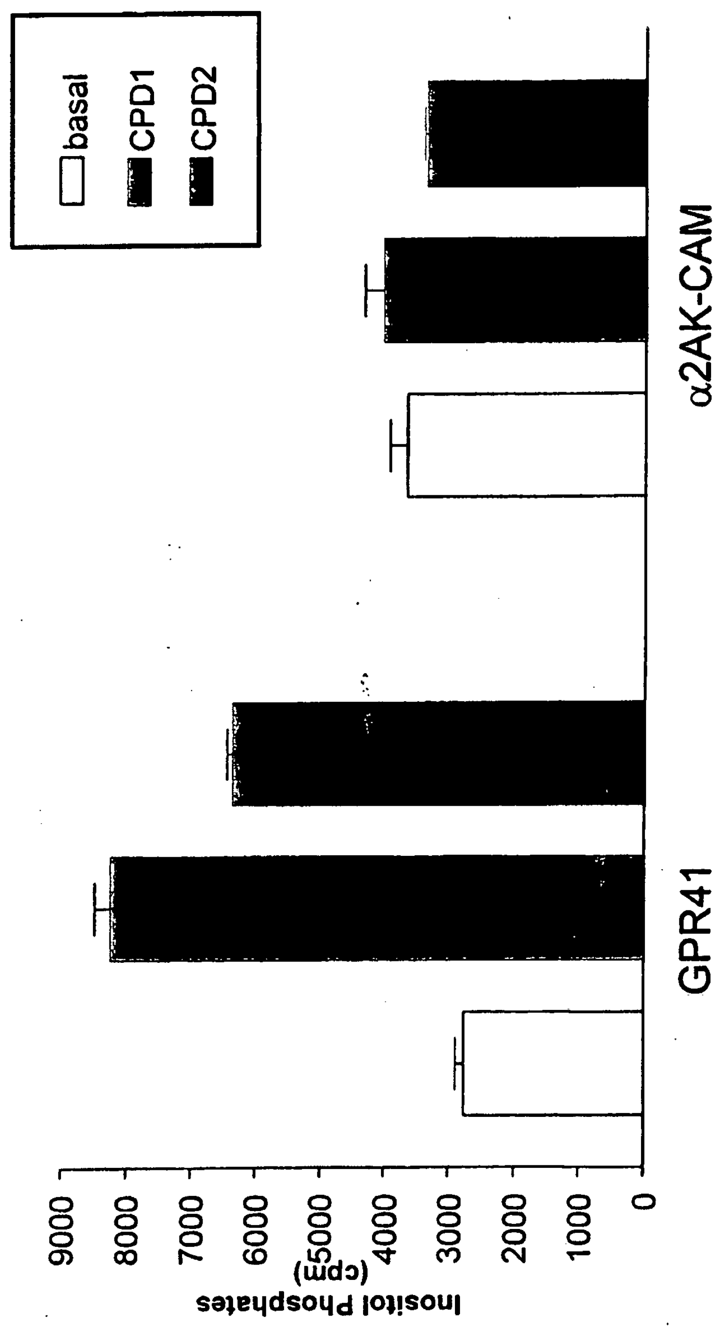


Figure 6

GPR41 Agonist Inhibits Insulin Release in MIN6 Insulinoma Cells

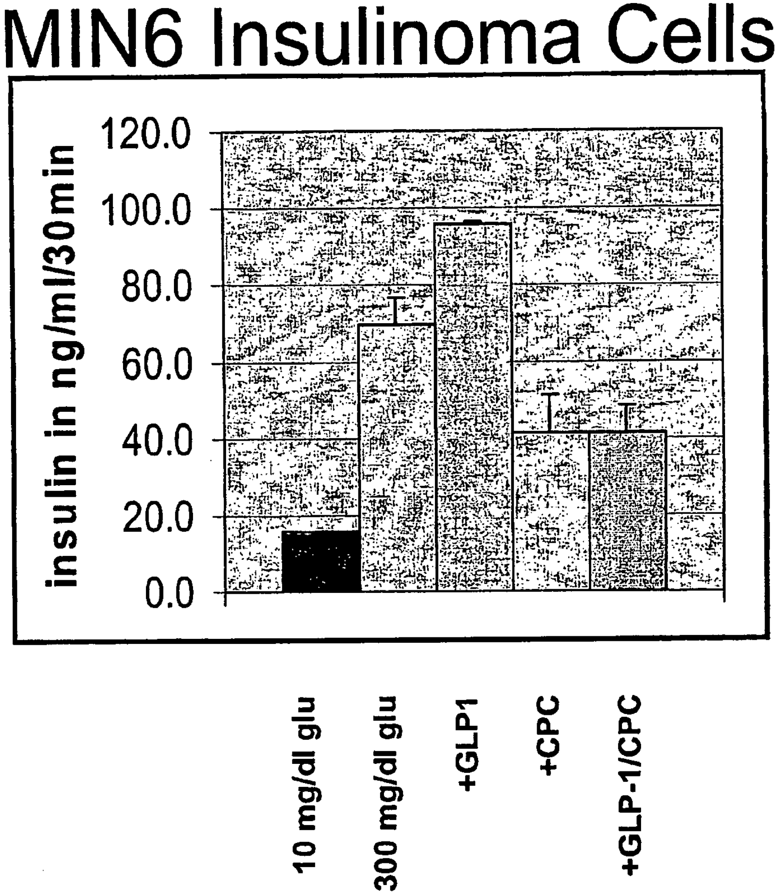


Figure 7

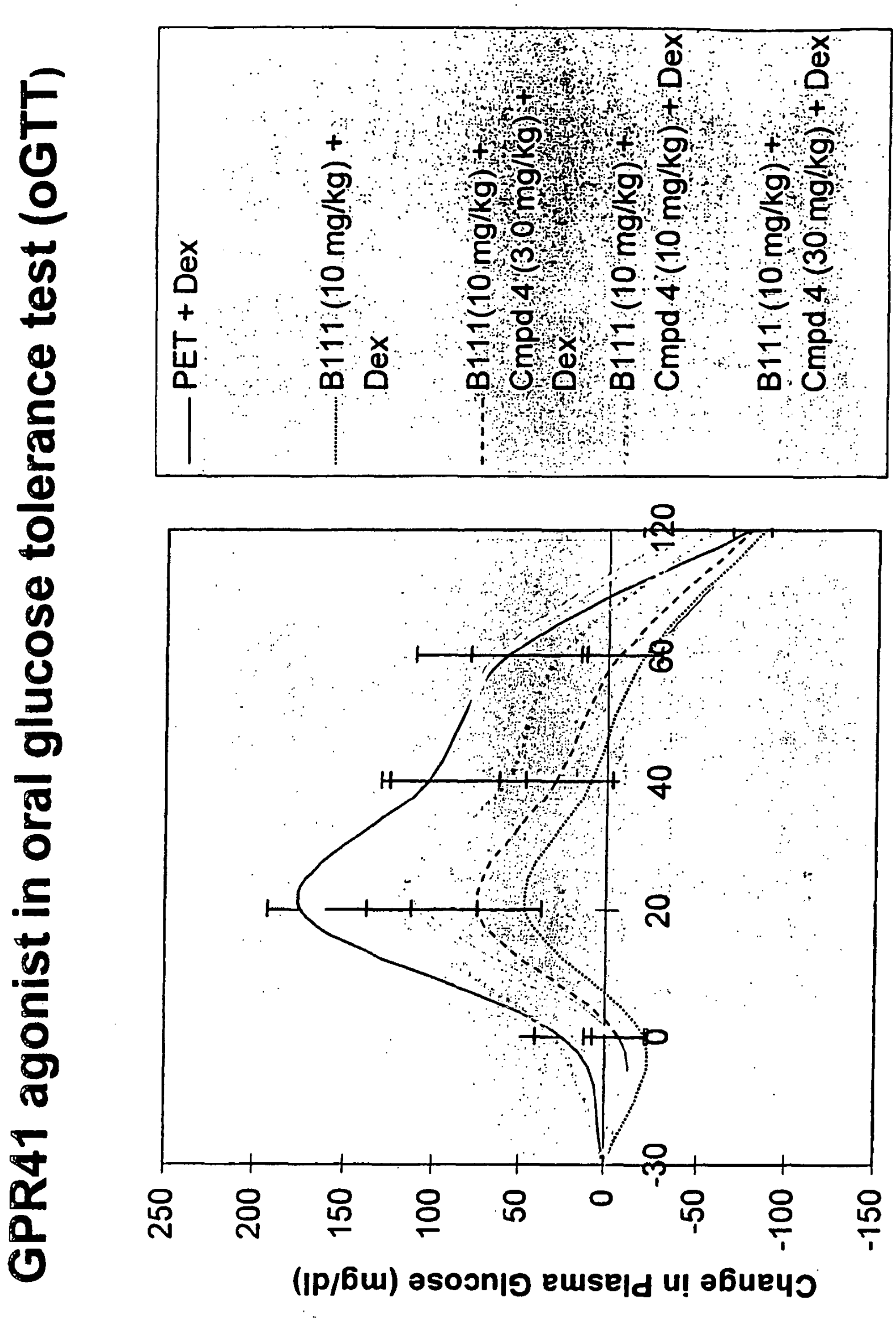
GPR41 agonist in oral glucose tolerance test (oGTT)
Change in Plasma Glucose (mg/dl)
250
200
150
100
50
0
-50
-100
-150
-30
0
20
40
60
120
PET + Dex
B111 (10 mg/kg) + Dex
B111 (10 mg/kg) + Cmpd 4 (3.0 mg/kg) + Dex
B111 (10 mg/kg) + Cmpd 4 (10 mg/kg) + Dex
B111 (10 mg/kg) + Cmpd 4 (30 mg/kg) + Dex

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0161359 A
- US 5462856 A
- US 6051386 A
- WO 2004065380 A1
- US 0205625 W
- WO 02068600 A


### Non-patent literature cited in the description


- **LE STUNFF et al.** *Diabetes,* 1989, vol. 43, 696-702
- **PEDERSON, P.** *Diab. Metab. Rev.,* 1989, vol. 5, 505-509
- **BRANCATI, F. L. et al.** *Arch. Intern. Med.,* 1999, vol. 159, 957-963
- **HILL, J. O. et al.** *Science,* 1998, vol. 280, 1371-1374
- **KENAKIN, T.** *Life Sciences,* 1988, vol. 43, 1095
- **OFFERMANNS ; SIMON.** *J Biol Chem,* 1995, vol. 270, 15175-80
- **MILLIGAN ; REES.** *Trends in Pharmaceutical Sciences,* 1999, vol. 20, 118-24
- **SAWZDARGO et al.** *Biochem. Biophys. Res. Commun.,* 1997, vol. 239, 543-547
- **BROWN et al.** *J. Biol. Chem.,* 2003, vol. 278, 11312-11319
- **KARLIN ; ALTSCHUL.** *Proc Natl Acad Sci USA,* 1990, vol. 87, 2264-8
- **ALTSCHUL et al.** *J Mol Biol,* 1990, vol. 215, 403-410
- **ALTSCHUL et al.** *Nature Genetics,* 1993, vol. 3, 266-72
- **ALTSCHUL et al.** *Nucleic Acids Res,* 1997, vol. 25, 3389-3402
- Remington's Pharmaceutical Sciences. Mack Publishing Co, 1980
- **REED ; SCRIBNER.** *Diabetes, Obesity and Metabolism,* 1999, vol. 1, 75-86
- **SEPILIAN ; NAGAMANI.** *J. Clin. Endocrinol Metab.,* 14 October 2003
- **BAILLARGEON et al.** *Fertil. Steril,* 2004, vol. 82, 893-902
- *Nature Medicine, Special Focus on Atherosclerosis,* 2002, vol. 8, 1209-1262
- **ZHU, D.-G.** *J. Org. Chem,* 2002, vol. 67, 943-948
- **COLLIER, T. L.** *J. Labelled Compd Radiopharm.,* 1999, vol. 42, S264-S266
- **BAS, M.-D.** *J. Labelled Compd Radiopharm.,* 2001, vol. 44, S280-S282
- **CARROLL et al.** *Journal of Medicinal Chemistry,* 2004, vol. 47, 3180-3192
- **SNIECKUS et al.** *Journal of Organic Chemistry,* 1991, vol. 56, 3763-3768
- **SUZUKI et al.** *Hum Gene Ther,* 1996, vol. 7, 1883-1893
- **ITOH, H. et al.** *Proc. Natl. Acad. Sci,* 1986, vol. 83, 3776
- *Diabetes,* 1982, vol. 31, 1-6
- *Diabetes,* 1983, vol. 32, 830-838
- *Annu Rep Sankyo Res Lab,* 1994, vol. 46, 1-57
- *J Clin Invest,* 1990, vol. 85, 962-967
- **COLEMAN.** *Diabetes,* 1982, vol. 31, 1
- **E SHAFRIR.** Diabetes Mellitus. Elsevier Science Publishing Co, 1990, 299-340
- **FRIEDMAN et al.** *Cell,* 1992, vol. 69, 217-220
- **TRUETT et al.** *roc Natl Acad Sci USA,* 1991, vol. 88, 7806
- **COLEMAN et al.** *Heredity,* 1990, vol. 81, 424
- **MOLLER DE.** *Nature,* 2001, vol. 414, 821-7
- **REED MJ et al.** *Diabetes, Obesity and Metabolism,* 1999, vol. 1, 75-86
- **MATSUDA, M et al.** *J Biol Chem,* July 2002
- **PLUMP AS et al.** *Cell,* 1992, vol. 71, 343-353
- **KAMADA N et al.** *J Atheroscler Thromb,* 2001, vol. 8, 1-6
- **GARBER DW et al.** *J Lipid Res,* 2001, vol. 42, 545-52
- **SMITH JD et al.** *J Intern Med,* 1997, vol. 242, 99-109
- **ROYO T et al.** *European Journal of Clinical Investigation,* 2000, vol. 30, 843-52
- **GERRITY RG et al.** *Diabetes,* 2001, vol. 50, 1654-65
- **COMHILL JF et al.** *Arteriosclerosis, Thrombosis, and Vascular Biology,* 1985, vol. 5, 415-26